# EUROPEAN PATENT SPECIFICATION

(11) **EP 2 625 277 B1**
(45) Date of publication and mention of the grant of the patent: **06.09.2017**
(21) Application number: 11805635.7
(22) Date of filing: 10.10.2011
(51) Int. Cl.: C12N 15/85

(54) **EXPRESSION VECTOR FOR HIGH LEVEL EXPRESSION OF RECOMBINANT PROTEINS**
EXPRESSIONSVEKTOR FÜR HOHE EXPRESSION REKOMBINANTER PROTEINE
VECTEUR D'EXPRESSION POUR L'EXPRESSION À HAUT NIVEAU DE PROTÉINES RECOMBINANTES

(30) Priority: 08.10.2010 IN 2806MU2010
(43) Date of publication of application: 14.08.2013
(73) Proprietor: Cadila Healthcare Limited, Ahmedabad 380 015, Gujarat (IN)
(72) Inventor: PARIKH, Aashini, Gujarat (IN); SINGH, Arun, Gujarat (IN); GUPTA, Ajit K, Gujarat (IN); JAKHADE, Mansi, Gujarat (IN); MENDIRATTA, Sanjeev Kumar, Gujarat (IN)
(74) Representative: Gill Jennings & Every LLP
(86) International application number: PCT/IN2011/000703
(87) International publication number: WO 2012/046255

(56) References cited:
- WO-A2-02/074969
- WO-A2-2007/017903
- WO-A2-2008/085962
- ZAHN-ZABAL M ET AL: "Development of stable cell lines for production or regulated expression using matrix attachment regions", JOURNAL OF BIOTECHNOLOGY, ELSEVIER SCIENCE PUBLISHERS, AMSTERDAM, NL, vol. 87, no. 1, 27 April 2001 (2001-04-27) , pages 29-42, XP004231294, ISSN: 0168-1656, DOI: 10.1016/S0168-1656(00)00423-5

## Description

### Field of the Invention

The present invention relates to a novel expression vector for high level expression of recombinant therapeutic proteins. In particular, the present invention discloses an expression vector having a gene sequence encoding a recombinant protein and at least one operably linked expression enhancing element such as, matrix attachment region. The said vector may further comprise of other regulatory elements. In another embodiment the invention comprises mammalian cells transfected with the said expression vector.

### Background of the Invention

The increased demand of therapeutic proteins is primarily due to their generally highly specific target of action which results in significantly reduced and well-defined risk of toxicity compared to small molecule based drugs. Despite having all these patient-friendly qualities, most therapeutic proteins remain inaccessible to most people in the world because they continue to remain prohibitively expensive. Therefore, life saving or other important drugs, like Erythropoietin, Darbepoietin, TNKase, Etanercept, Gonadotropins, that significantly improve quality of life, and many anticancer monoclonal drugs like Rituximab, Trastuzumab and other therapeutic monoclonal antibodies etc., are afforded only by a very small percentage of people while a vast majority of sick people around the world cannot afford them. There is therefore an urgent need to bring down the cost of these drugs.

A large component of this high cost is associated with manufacturing them which occurs because of their low production yields. The production yield of a clone depends upon selection of several factors such as the external factors like culture conditions (media components, temperature, pH etc.) and downstream purification process, and internal factors like selection of vector and its regulatory elements like promoter, transcription or translation enhancing elements, etc and their appropriate orientation and also choice of a suitable host cell. Mammalian cell is the most promising expression system to obtain high expression of recombinant therapeutic proteins as it has a natural capacity of glycosylation. Also, post-translational modifications in such expression systems are more likely to resemble those found in human cells expressing proteins, thus rendering physiological activity. However the expression levels in eukaryotic cells are also highly dependent on another internal factor, i.e. the integration site of the recombinant expression construct comprising the gene of interest in the genome of host cell.

Recombinant expression plasmids comprising a gene of interest encoding a desired protein are routinely used to generate stable CHO transfectants or other mammalian transfectants, expressing the desired recombinant protein. The ideal system for eukaryotic overexpression would have integration of the expression cassette in the genome of a target cell at a location that permits strong and stable or long term expression. However, most current methods achieve only random integration of plasmid DNA into the genome of the host cell. It is evident from the literature that the expression levels are highly variable in clonal populations arising out of such a transfection process (Brian K Lucas et al., Nucleic Acids Research, 1996, Vol. 24, No. 9 , R.T. Schimke et al., Br. J. Cancer (1985), 51, 459- 465, Würtele H, et al., Gene Ther. 2003 Oct;10(21):1791-9. Biotechnol Prog. 2000 Sep-Oct;16(5):710-5, Yoshikawa T et al., Biotechnol Prog. 2000 Sep-Oct;16(5):710-5., Kim NS, et al., Biotechnol Prog. 2001 Jan-Feb;17(1):69-75). Also, the frequency of transfectomas carrying the stably integrated recombinant gene that are capable of expressing a desired recombinant protein at high levels is quite low.

Usually a large number of stably transfected cells must be screened to identify clones which express the recombinant proteins at high levels. This is mainly believed to be due to the effects of the genomic environment of the integration site, as the mammalian genome is really large and only about 0.1% of it contains transcriptionally active sequences (Little (1993) Nature 366:204). This phenomenon of the site of integration influencing expression is called "position effect". The position effect regulates the expression levels of intergrated gene in a positive or negative manner due to any or all of the following mechanisms - 1) presence of regulatory elements near the site of integration, which may participate in regulating the expression of the integrated gene 2) the chromatin structure at the site of integration. 3) the DNA methylation activity at the site of integration. The negative impact of position effect can be as harsh as gene silencing via DNA methylation or histone deacetylation.
It is therefore highly unlikely that the technology of random plasmid integration into the genome of CHO cells will always result in the insertion of a recombinant gene into a transcriptionally active zone capable of high levels of gene expression. This means that the number of clones that need to be screened in order to find the high expressing clone would be very large.

To overcome the problem associated with random integration the discovery of systems to integrate the desired gene in site specific manner in the genome have been described in the literature and have been used with limited success. For example some groups have used certain enzymes for the site-specific recombination mediated introduction of genes of interest in transcriptionally active sites of the genome. Recombinases such as Cre and FLP perform both integration and excision with the same target sites (Sauer, B. (1994) Curr Opin. Biotechnol., 5, 521-527, Sternberg et al, J Mol Biol, 1981,150,467-486, Broach et al, Cell, 1980,21,501-508). However, although these recombinases efficiently perform integration in mammalian cell, the net integration frequency that they mediate is low because of the excessive back reaction. Therefore, the problem of stable and high expression still remains to be solved.

Traditionally, to achieve stable high producing cell lines, methods to increase the gene copy number such as methotrexate (MTX) mediated gene amplification process have been routinely done. Transfection is followed by extensive search of single cell clones having the desired phenotype. Also, levels of methotrexate are generally increased in small increments while giving sufficient time for cells to stabilize at each increment level. Thus, this process of increasing expression from transfected mammalian cells is time consuming and labor intensive.

Transcription of eukaryotic genes is regulated by a variety of cis- and trans-acting regulatory elements (Dillon et.al., (1993) Trends Genet. 9:134). Two of the best characterized cis- elements are promoters and enhancers. Promoters are DNA sequences immediately 5' to the coding sequence of the gene. They comprise multiple binding sites for trans-acting transcription factors, forming the basic transcription apparatus. Similarly enhancers are also composed of multiple binding sites for trans-acting transcription factors but can be found far upstream or downstream of coding sequences or even within introns. These elements can also act in an orientation independent manner. Activities of promoters and enhancers can be detected in transient expression systems and they contain elements which may or may not be tissue specific.

The inventors of the present invention have already disclosed in their application WO2007017903 the combined effect of regulatory elements such as a) a CMV promoter, b) an intron, c) TPL, d) VA genes and e) a bovine growth hormone polyadenylation sequence to achieve high expression levels of recombinant proteins.

Another category of cis-acting regulatory elements are ones which are believed to regulate the chromatin structure and includes locus control regions (LCRs) [Grosveld et.al., (1987) Cell 51:975], matrix attachment regions (MARs) [Phi-Van et.al., (1990) Mol. Cell. Biol. 10:2302], scaffold attachment regions (SARs) [Gasser and Laemmli (1987) Trens Genet. 3:16], insulator elements [Kellum and Schedl (1991) Cell 64:941] and Nuclear matrix-Associating DNAs [Bode J et.al., (1992) Science 255:195].

MARs and SARs are similar enhancers in that they are able to act over long distances, but are unique in that their effects are only detectable in stably transformed cell lines or transgenic animals. LCRs are also dissimilar to other types of enhancers in that they are position and orientation dependent, and are active in a tissue specific manner.

SARs/MARs elements have been used to remove the drawback of position effects and to provide highly active genes in the expression construct. They prevent the neighbouring host cell chromatin elements from affecting the transgene expression. MARs have been isolated from regions surrounding actively transcribed genes but also from centromere and telomeric regions. They increase the expression of desired gene by regulating the transcription activity.

Several different MARs/SARs such as Drosophila Scs boundary element, hspSAP MAR, Mouse T cell receptor TCRα, Rat locus control region, β-globin MAR, Apolipoprotein B SAR element etc. have been reported from different species and different highly expressed genes in the existing literature. Most of these elements showed low to moderate improvement in the expression levels of the desired gene in CHO cells (P.A. Girod and Nicolas Mermod, Gene Transfer and Expression in Mammalian Cell, 2003). In contrast, Chicken Lysozyme MAR (cLysMAR) was shown to have 5 to 6 fold higher expression levels as compared to controls where the MAR elements were absent (P.A. Girod and Nicolas Mermod, Gene Transfer and Expression in Mammalian Cell, 2003). Also, when the same MARs were used at both the sides flanking the expression cassette, a 4 to 5 fold further increase in expression levels is observed (P.A. Girod and Nicolas Mermod, Gene Transfer and Expression in Mammalian Cell, 2003). Thus, it is evident that cLysMAR was most promising element reported in prior art. cLysMAR is localized far upstream of the chicken lysozyme gene (Phi-Van and Stratling, EMBO, 7,3, pp 655-64,1988). In transformed animal cell lines, this MAR has been shown to increase the overall level of transgene expression and to decrease its position dependent variability when placed around a reporter gene (Stief et al., Nature, 341, pp 343-345, 1989). This effect has been found to extend to heterologous promoters and cells (Phi-Van et al., Molecular and Cellular Biology, 10,5 pp 2302-2307, 1990) as well as to the tissue specificity of transgene expression (McKnight et al., 1992).

US7422874 describes the use of β-globin MAR in combination with the regulatory elements - pSV-gal or pCMV-gal promoter, MCS site and a transcriptional termination site in the PMS vector construct to increase the expression of β galactosidase reporter gene, scu-PA gene and the TGF- β SRII genes. They were able to get moderate expression levels of 20 ug/million cells for β galactosidase in 88 % of the clones. They were also able to generate clones for scu-PA having 4 fold more expression levels as compared to control vector construct consisting of the same regulatory elements as the above vector except MARs. When MARs and DHFR system for gene amplification were used together in expression of TGF- β SRII, they were able to generate primary clones producing 100ng/million cells/day after transfections and 10 ug/million cells/day after several rounds of MTX mediated gene amplification upto 1uM MTX. However the expression levels obtained in this patent are not commercially viable today for biotherapeutics proteins such as TNKase, Darbepoietin, and monoclonal antibodies.

US7371542 describes the use of β IFN S/MAR in combination with the regulatory elements - CMV Promoter, Intron, Ori P and Poly A in the expression vector construct to increase the expression of a LTBR-Fc (Lymphotoxin beta receptor - IgG Fc Fusion protein) and achieved a 4.5 fold improvement in expression levels in CHO Cells as compared with control vector consisting of the same regulatory elements as the above vector except MARs. They also found that use of the β IFN S/MAR in expression vector increases the expression level 6.3 fold in 293 EBNA cells using the vector pCEP-LTBR-Fc. However the expression levels were still very low (20 mg/L in 5 days). The vector

pCB_SM1_LTBR-Fc was able to give clones in 293 EBNA cells having a productivity of 40 mg/L in 9 days. But the productivity levels obtained in this patent are far less than desirable for such biotherapeutic molecules.

US5731178 describes the enhanced expression of desired gene by using the cLysMARs in vector construct comprising promoter and enhancer. They showed that the use of the cLysMAR element in stable transfections was able to improve the reporter gene CAT activities by more than 10 fold when mar element was used in combination with enhancer and promoter element over the control construct consisting of just the enhancer and the promoter, however the MAR element was not able to show any major impact by itself.

Poljak et al., (1994) Nucleic Acid Res., 22(21):4386-94) reports the increase in expression of the CAT reporter gene by about 15 fold when cLysMAR was used in combination with an SV40 Promoter and an enhancer. The cLysMARs by itself was found to be very poor in increasing the expression of the desired gene, rather it showed a slight decrease.

Thus, the above examples demonstrate the fact that the control vector constructs comprising of the standard regulatory elements known to anyone skilled in the art were not in themselves sufficient to support high expression. And further even after combination with MARs/SARs, the expression levels did not increase to those required commercially for viable production of recombinant therapeutics. Thus, a unique combination of elements was still required to achieve desired expression levels.

Therefore it becomes clear that there exists an important need in the industry to further increase the expression of therapeutic proteins to make them more and more affordable. Therefore one feels the need for a combination of elements in the expression vector that can work along with MARs in a synergistica fashion to give increased expression with different kind of target genes. The inventors of the present invention have henceforth proved that a concerted action of a unique combination of regulatory elements is required for optimal expression of a recombinant protein in a time saving manner.

US7129062 describes the co-transfection of more than 2 unlinked vectors where one vector comprises gene of interest and second one comprises cLysMARs to increase the expression of two recombinant proteins - luciferase and anti Rhesus D IgG by about 20 folds and they were also able to produce human anti Rhesus D IgG at 200 mg/L using this cotransfection strategy. However it is well reported (DNA Cloning: Mammalian systems; By David M. Glover, B. D. Hames) in the literature and also in our experience that cotransfection increases heterogeneity and variability in the transfected population. Moreover, the present invention achieved the desired yields by using single transfection.

US20080102523 describes the use of β-globin MAR for increasing the expression of beta-galactosidase by 3 fold and immunoglobulin by 6 fold as compared to the control vector construct consisting of SV40 promoter-enhancer and/or CMV promoter, ori site, and a poly A region. The above patent application achieves only a moderate increase in expression via both the MTX mediated gene amplification pressure as well as with the help of the β-globin MAR regulatory element thus making the whole process tedious and time consuming. This is in contrast with the current invention where the inventors have achieved high expression with their unique combination of regulator elements and without using any long and tedious methods like the MTX-DHFR selection method.

US5888774 describes the high expression of erythropoietin by using human apolipoprotein B SAR element and reports an expression of 1500 to 1700 IU of EPO/ million cells/ 24 Hrs. WO2007017903 owned by the inventors describes a process to produce recombinant human erythropoietin at an expression level of 11,830 IU/ml (91 µg/ml) in a 168 hrs culture which is equivalent to 2366 to 3549 IU/10⁶ cells / 24 Hrs or 18.2 to 27.3 µg/10⁶ cells / 24 Hrs, which is remarkably higher than the reported values in US5888774.And the present invention further increases expression levels significantly over the vector of WO2007017903, for several therapeutic proteins.

WO2008/085956 discloses a method of producing a high titer of a recombinant protein without requiring gene amplification. The method involves introducing into cells, via high efficiency transfection, a nucleic acid encoding elements capable of opening chromatin and/or maintaining chromatin in an open state, operably linked to a sequence encoding the recombinant protein.

Thus, it is still desirable to develop novel expression vectors for further increasing the productivity of eukaryotic host cells. Surprisingly, in spite of the tremendous amount of knowledge generated in this field over the last decades even today a person skilled in the art cannot simply pick and choose a combination of internal factors or regulatory elements to design an expression vector that would give considerably high expression. Further, the combination of the suitable elements to create a high expressing vector cannot be routinely extrapolated by a skilled person since the high expression of the desired gene of interest using the vector cannot be just attributed to only one element but a combination of appropriate elements are desirable to get a stable high expressing cell lines. Hence, this invention provides a solution to this problem by providing novel expression vectors that comprise of expression enhancing elements like chicken lysozyme MAR element(s) in combination with other regulatory elements such as a CMV promoter, an intron, TPL, and VA genes which have multiple roles e.g., in increasing the mRNA levels by increased transcription, of extending the life of the mRNA molecule by increasing its stability, and by increasing the translation efficiency, thus working in a synergistic manner leading to a high and stable expression of the recombinant protein in transfected mammalian host cells. The combination of these elements in the expression vector of the present invention, consisting of the expression cassette flanked by cLysMAR in a cis or trans orientation results in a stable, high expression of therapeutic proteins in transfected cell lines.

### Objects of the Invention

The present invention provides an expression vector which increases the expression efficiency of the protein of interest in mammalian cells.
In one embodiment, the present invention provides a novel expression vector comprising a promoter operably linked to the gene of interest, expression enhancement element, TPL, VA I and II genes, a translation terminator and an antibiotic marker wherein the expression enhancement element is a matrix attachment region that is cLysMARs having the nucleotide sequence of SEQ ID NO: 5.

In one embodiment, the present invention provides the novel expression vector construct for the expression of therapeutic proteins and peptides where the expression vector construct comprises the promoter operably linked to cloning sites, gene of interest, translation terminator, TPL, VA I and II genes, suitable antibiotic marker in combination with an expression enhancing element selected from cLysMARs having the nucleotide sequence of SEQ ID NO: 5.
In an alternate embodiment, the present invention provides a novel expression vector construct for the expression of therapeutic proteins and peptides where the expression vector construct comprises the promoter operably linked to cloning sites, gene of interest, translation terminator such as BGH, intron, suitable marker and optionally internal ribosomal binding site in combination with an expression enhancing element selected from cLysMARs having the nucleotide sequence of SEQ ID NO: 5.

In one embodiment, the present invention provides the novel expression vector construct for the expression of therapeutic proteins and peptides where the expression vector construct comprises the promoter operably linked to cloning sites, gene of interest, translation terminator such as BGH, TPL, VA I and II genes, Intron, suitable antibiotic marker and optionally internal ribosomal binding site in combination with an expression enhancing element selected from cLysMARs having the nucleotide sequence of SEQ ID NO: 5.

Yet in another embodiment the present invention provides the process for expressing gene of interest in mammalian host cell which is transfected with the expression vector according to the embodiments of the invention.

### Brief description of Drawings

Figure 1 depicts the vector diagram of pZRCII
Figure 2 depicts the vector diagram of pZRC III
Figure 3 depicts the vector diagram of pZRC III- TNK- Hyg
Figure 4 depicts the vector diagram of pZRC III- Darbe- Hyg
Figure 5 depicts the vector diagram of pZRC III- Etanercept- Hyg
Figure 6 depicts the vector diagram of pZRC III- FSH α- IRES- FSH β - Hyg vector
Figure 7 depicts the vector diagram of pZRC III- FSH β - IRES- FSH α - Hyg vector
Figure 8 depicts the vector diagram of pZRC III - TNK- Puro
Figure 9 depicts the vector diagram of pZRC III - DARBE- Puro
Figure 10 depicts the vector diagram of pZRC III- FSH α- IRES- FSH **-** β Puro vector
Figure 11 depicts the vector diagram of pZRC III- FSH α- IRES- FSH β - Neo vector
Figure 12 depicts the vector diagram of pZRC III- FSH β - IRES- FSH α - Neo vector
Figure 13 depicts the vector diagram of pZRC III - Etanercept- Neo
Figure 14 depicts the vector diagram of pZRC III - TNK- Neo

### Description of the Invention

### Definition

Chromatin attachment regions are structural components of chromatin that form topologically constrained loops of DNA through their interaction with the proteinaceous nuclear matrix.

### Abbreviations used:

| | |
|---|---|
| CMV | Cytomegalovirus |
| TPL | Tripartite |
| Leader VA genes or VA I and II genes | Virus associated RNA genes I and II |
| BGH | Bovine growth hormone |

The present invention provides a novel expression vector which increases the efficiency of expression of therapeutic proteins and peptides significantly in mammalian host cell. The novel vector further removes the drawback associated with the position effect and adding the advantage of increased transcription and translation achieved with the unique combination of regulatory elements.

In one embodiment, the present invention provides a novel expression vector comprising a promoter operably linked to the gene of interest, expression enhancement element, TPL, VAI and II genes, translation terminator and an antibiotic marker wherein the expression enhancement element is a matrix attachment region that is cLysMARs having the nucleotide sequence of SEQ ID NO: 5. Chromatin attachment regions are selected from MARs and SARs.

In an embodiment the present invention provides an expression vector for the production of proteins and peptides which comprises promoter operably linked to gene of interest, TPL and VA genes I and II, a matrix attachment region cLysMARs having the nucleotide sequence of SEQ ID NO: 5, translation terminator antibiotic marker.

In an alternate embodiment, the present invention provides a novel expression vector construct for the expression of therapeutic proteins and peptides where the expression vector construct comprises the promoter operably linked to cloning sites, gene of interest, translation terminator such as BGH, intron, suitable antibiotic marker and optionally internal ribosomal binding site in combination with an expression enhancing element selected from cLysMARs having the nucleotide sequence of SEQ ID NO: 5.
In one embodiment, the present invention provides the novel expression vector construct for the expression of therapeutic proteins and peptides where the expression vector construct comprises the promoter operably linked to cloning sites, gene of interest, translation terminator such as BGH, TPL, VA I and II genes, Intron, suitable antibiotic marker and optionally internal ribosomal binding in combination with an expression enhancing element selected from cLysMARs having the nucleotide sequence of SEQ ID NO: 5.

According to an embodiment of the present invention, the promoter is selected from the group consisting of CMV promoter, SV40 promoter, adenovirus promoter, Beta actin promoter, metallothionin promoters or other prokaryotic or eukaryotic virus promoters. In preferred embodiment the CMV promoter is used.

The promoter is typically located near the gene it regulates, on the same strand and upstream i.e. towards the 5' region of the sense strand and it facilitates transcription.

According to an embodiment of the present invention, the cloning sites can be selected from but not limited to AatI, AatII, Acc113I, Acc16I, Acc65I, AccIII, AclNI, AseI, AsnI, Asp718I, BalI,

BamHI,BanI,BanII,BbeI,BbiII,BbsI,BbuI,Bbv16II,BclI,BcoI,BglI,BglII,BlnI,Bs h1365I,BsiEI,BsiHKAI,BsiI,BspEIBspHI,BstZI,CciNI,Cfr10I,Cfr42I,Cfr9I,CfrI,Csp45 I,DrdI,DsaIEaeI,EagI,Eam1104I,Eco47III,Eco52I,Eco57I,Eco88I,Eco91I,EcoICRI,Eco O109I,EcoRI,EcoT14I,FriOI,FseI,FspI,HaeII,Hin1I,HincII,HindII,KasI,Ksp632I,KspI, KpnI,LspI,MamI,MflI,MluNI,MroI,MroNI,MspA1I,NaeI,NarI,NcoI,NdeI,NgoAIV,Nh eI,NotI,NspBII,NspI,NspV,PacI,PaeI,PflMI,PinAI,Ple19I,Pme55I,PmeI,Ppu10I,PpuMI, PshBI,Psp124BI,Psp5II,PspAI,PspALI,PspEI,PstI,PstNHI,PvuI,PvuII,SacI,SacII,ScaI,S fcI,SfiI,SpeI,SphI,Van91I,VneI,XhoI, XhoII,XmaI,XmaIII,Zsp2I.

According to an embodiment of the present invention the translation terminator is selected from the group consisting of bovine growth hormone, adenovirus and Eukaryotic Virus translation terminator sequences. In a preferred embodiment, the translation terminator is BGH Poly A.

In a further embodiment, the internal ribosomal binding sites are selected from Picornavirus IRES, Aphthovirus IRES, Hepatitis A IRES, Hepatitis C IRES, Pestivirus IRES, Encephalomyocarditis virus IRES preferably Encephalomyocarditis virus IRES.

In preferred embodiment the Chicken Lysozyme MAR (Sequence id 5) is cloned at 5' flanking sequence or 3' flanking sequence. In most preferred embodiment the Chicken Lysozyme MAR is cloned at both 5' and 3' flanking sequence of the transcriptional assembly.

In the present invention the gene of interest is cloned in the expression vector according to method known in the art (SAMBROOK, J.; FRITSCH, E.F. and MANIATIS, T. Molecular Cloning: a laboratory manual. 2nd ed. N.Y., Cold Spring Harbor Laboratory, Cold Spring Harbor Laboratory Press, 1989. 1659). According to an embodiment of the present invention the gene of interest may encode suitable proteins and peptides and functional analogues thereof selected from tissue plasminogen activator, TNK-TPA, Darbepoietin, Erythropoietin, Insulin, GCSF, Interleukin, Tumor necrosis factor, Interferon, TNFR-IgGFc, Monoclonal antibodies such as Rituximab, Bevacizumab, Adalimumab, Trastuzumab (generic names) and their fragments like Fc region, Fab, GLP-I, GLP-II, IGF-I, IGF-II, Platelet derived growth factor, FVII, FVIII, FIV and FXIII, exendin-3, exendin 4, transcription factors like MYT-2, NF-κB repressing factor NRF, AML1/RUNX1, Gtx homeodomain protein, translation factors like Eukaryotic initiation factor 4G (elF4G)a, Eukaryotic initiation factor 4Gl (elF4Gl)a, Death associated protein 5 (DAP5), oncogene like c-myc, L-myc, Pim-1, Protein kinase p58PITSLRE, p53 hormones such as gonadotropic hormones selected from Follicle stimulating hormone, Human Chorionic Gonadotropin, Human Leutinizing Hormone, etc._and immunoglobulin heavy chain binding protein (BiP), Heat shock protein 70, β-subunit of mitochondrial H+-ATP synthase, Ornithine decarboxylase, connexins 32 and 43, HIF-1a, APC Accordingly, functional analogues means proteins or peptides having similar or identical functional to their native proteins and peptides.

According to the invention, expression vector construct comprises an expression assembly further comprising an operably linked promoter, cloning sites, gene of interest, transcription terminator, intron, suitable antibiotic marker, TPL, VA gene I and II. This expression vector is referred as pZRCII which is disclosed in sequence id no. 4(6). This new expression vector is referred as pZRCIII disclosed in sequence id no 6. A matrix attachment region is cloned at 5' or 3' flanking region of pZRCIII. In preferred embodiment matrix attachment regions is cloned at both 5' and 3' flanking region of pZRCIII. pZRCIII construct of the present invention is an advance over the vector known in prior art and enhance the expression of gene of interest significantly as well as improves transfection efficiencies. The present vector construct pZRCIII is suitable for expression of all proteins and peptides.

The suitable antibiotic marker in expression vector pZRCIII is selected from kanamycine, hygromycin puromycin and DHFR. In another embodiment the expression vector pZRCIII optionally carries the gene sequence of DHFR and/or internal ribosomal binding site (IRES).

In one embodiment, the present invention provides the novel expression vector construct for the expression of therapeutic proteins and peptides where the expression vector construct comprises the promoter operably linked to cloning sites, gene of interest, translation terminator, TPL (Seq ID no. 2), VA I and II genes (Seq ID no. 3), suitable antibiotic marker in combination with an expression enhancing element selected from cLysMARs having the nucleotide sequence of SEQ ID NO: 5

In another embodiment, the present invention provides the novel expression vector construct for the expression of therapeutic proteins and peptides where the expression vector construct comprises the promoter operably linked to cloning sites, gene of interest, transcription terminator, intron (Seq ID no. 1), suitable marker and optionally internal ribosomal binding site in combination with an expression enhancing element selected from cLysMARs having the nucleotide sequence of SEQ ID NO: 5.

The expression vector for the production of the desired expression of proteins and peptide comprises suitable elements but is not limited to the incorporation of 3 elements namely, *Adenoviral Tripartite leader* sequence at the 3' end of promoter, a hybrid (chimeric) intron comprising of 5' donor site of the adenovirus major late transcript and the 3' splice site of mouse immunoglobulin which is placed at the 3' end of the TPL (Seq ID no. 2), the adenoviral VA RNA I and II genes (Seq ID no. 3). The matrix attachment region is cloned at 5' flanking sequence at Mlu I or 3' flanking sequence at the end of BGH Poly A sequence. The orientation of matrix attachment region is optional. Moreover MARs element of the present invention not only enhance the expression of desired gene synergistically in combination with *Adenoviral Tripartite leader* sequence, hybrid (chimeric) intron, TPL (Seq ID no. 2) and the adenoviral VA RNA I and II genes (Seq ID no. 3) but also increase the transfection efficiency and numbers of desired clone.

In a preferred embodiment, the present invention provides the novel expression vector construct for the expression of therapeutic proteins and peptides where the expression vector construct comprises the promoter operably linked to cloning sites, gene of interest, transcription terminator, TPL (Seq ID no. 2), VA I and II genes (Seq ID no. 3), Intron (Seq ID no. 1), suitable marker and optionally internal ribosomal binding in combination with an expression enhancing element selected from cLysMARs having the nucleotide sequence of SEQ ID NO: 5. In embodiment, the expression vector comprises a Promoter or variant thereof, operably linked to gene of interest, VA I and II gene or variant thereof, TPL or variant thereof, Chimeric Intron or variant thereof, Antibiotic marker, Matrix attachment regions, Optionally internal ribosomal binding site, Bovine growth harmone polyadenylation

In one embodiment PZRCIII-gene of interest-Hygromycin vector contains cLysMARs setforth the in sequence id no 5 operably linked with gene of interest driven by a CMV promoter, TPL, a chimeric intron setforth in sequence id no 1, VA genes I and II setforth in sequence id no. 3, BGH polyadenylation and multiple cloning sites. Multiple cloning sites includes restriction sites like XhoI, NotI. Any gene of interest can be cloned at multiple cloning site like the chemically synthesised gene of the fusion protein Etanercept (TNFR-Fc) cloned into multiple cloning site of the vector having a chicken lysozyme MAR element both in the upstream and downstream of the expression cassette in combination with other regulatory elements such as a CMV promoter, TPL, a chimeric intron in the expression cassette and VA genes placed outside the expression cassette. The vector has a hygromycin resistance gene for selection of transfectants. As an example of a vector construct prepared according to this aspect of the present invention, the pZRCIII-etanerceptetanercept-Hygromycin vector is deposited under Budapest treaty and accession number is MTCC 5656.

In another embodiment PZRCIII-gene of interest-Neomycine vector contains C-Lys-MARs setforth the in sequence id no 5 operable linked with CMV promoter, TPL, A chimeric intron setforth in sequence id no 1, VA genes I and II setforth in sequence id no. 3, BGH polyadenylation and multiple cloning sites. Multiple cloning site includes restriction sites like XhoI, NotI. Any gene of interest can be cloned at multiple cloning site like the chemically synthesised gene of the fusion protein Etanercept (TNFR-Fc) cloned into multiple cloning site of the vector having a chicken lysozyme MAR element both in the upstream and downstream of the expression cassette in combination with other regulatory elements such as a CMV promoter, TPL, a chimeric intron in the expression cassette and VA genes placed outside the expression cassette. The vector has a hygromycin (neomycin) resistance gene for selection of transfectants.

As an example of a vector construct prepared according to this aspect of the present invention, the pZRCIII-etanercept-Neomycin vector is deposited under Budapest treaty and accession number is MTCC 5657.

In another embodiment PZRCIII-gene of interest-IRES-Hygromycin vector contains cLysMARs setforth the in sequence id no 5 operable linked with CMV promoter, TPL, a chimeric intron setforth in sequence id no 1, VA genes I and II setforth in sequence id no. 3, BGH polyadenylation and multiple cloning sites. Multiple cloning sites includes restriction sites like XhoI, NotI. Any gene of interest can be cloned at multiple cloning site like chemically synthesised genes of the FSH α and FSH β subunits cloned into multiple cloning site of the vector and both FSH α and FSH β subunits operably linked to each other by IRES, having a chicken lysozyme MAR element both in the upstream and downstream of the expression cassette in combination with other regulatory elements such as a CMV promoter, TPL, a chimeric intron in the expression casssete and VA genes placed outside the expression cassete. The vector has a hygromycin resistance gene for selection of transfectants. As an example of a vector construct prepared according to this aspect of the present invention, the pZRCIII FSH α -IRES-FSH β-hygromycin vector is deposited under Budapest treaty and accession number is MTCC 5655.

In an embodiment, the expression vector is transfected to mammalian host cell by processes known to a skilled person. The mammalian host cell may be selected from CHO (Chinese hamster ovary) cell line, BHK (Baby hamster kidney) cell line etc which are well known for commercial production of proteins. In another embodiment the transfected host cell is further transfected with a different vector containing suitable antibiotics selected from kanamycin, hygromycin, puromycin to increase further expression of gene of interest.

In one embodiment the transfected cell line is CHO K1 which is selected by using Hygromycin, puromycin, kanamycine, G418 or other antibiotics.
Furthermore the transfected cell lines are selected by using DHFR selection medium e.g. methotrexate, if the expression vector carries a genes of DHFR. This selection relies on a gradual increase in the selection pressure on the transfected cell-line. (Kaufman and Sharp, 1982; Schimke et al., 1982).

In yet another embodiment the transfected cell lines are selected in a Glutamine synthetase (GS) selection medium, e.g. methionine sulphoximine (MSX), as the expression vector carries a genes of Glutamine synthetase.

Hence the present invention provides a novel expression vector comprising a unique combination of regulatory elements which increase transcription and translation remarkably and also suppress the position effects of the gene integration, thus giving a synergistic effect to the stable, high expression of the recombinant protein. In addition it provides the production of therapeutic proteins and peptide, monoclonal antibodies at industrial scale in a time effective manner as the labour intensive screening of a huge number of clones is drastically reduced in presence of typical elements. In addition the present expression vector can be used for both transient as well as stable expression. The present invention is further illustrated with the help of examples. The examples are only for illustrative purpose and present invention is not limited to them only.

### Example 1. Construction of pZRC III vector

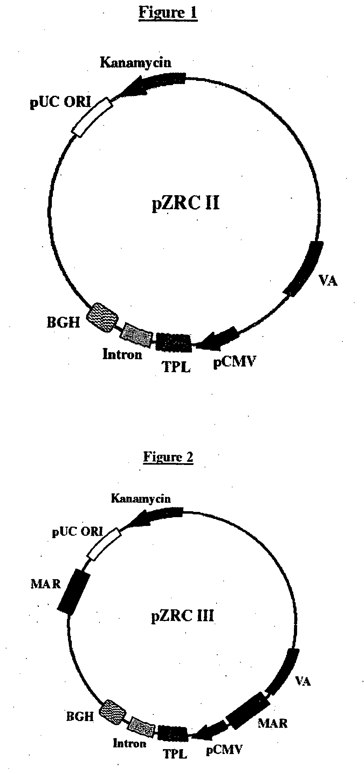

The whole transcription assembly with all the regulatory elements namely TPL, VA, CMV promoter, chimeric intron, and BGH polyadenylation and termination sequences, described in our earlier patent application WO2007017903, was chemically synthesized at GeneART, Germany. This whole assembly cloned in the cloning vector pMK (GeneART, Germany) was called pZRC II (Figure 1, Seq ID No. 4,). Chicken lysozyme MAR DNA fragment (Seq ID No 5), (Phi-Van, L. and Stratling, W.H, Biochemistry 35 (33), 10735-10742 (1996)) was chemically synthesized and cloned in a cloning vector. Two chicken lysozyme MAR fragments were inserted as flanks on either side of the expression cassette in the pZRC II vector using the SacI and MluI sites which were already pre-designed into the vector. SacI overhang was added to the Chicken lysozyme MAR fragment by PCR using primers having SacI site. Specifically 40 cycles of PCR amplification were carried out using 100 picomoles of gene specific oligonucleotide primers in a volume of 50 µl containing 50 mM Tris-Cl (pH8.3), 2.5 mM MgCl₂, 250 µM each of the 4 dNTPs and 5 units of Pfu Polymerase. Each PCR amplification cycle consisted of incubations at 95° C for 30 sec (denaturation), 62° C for 30 sec (annealing) and 72° C for 2 min (extension). Amplified product of the PCR reaction was resolved on a 1% Agarose gel. The desired fragment of approx 1664 base pairs size was excised out from the gel and purified using Qiagen Gel extraction kit. This purified DNA fragment was ligated into pZRC II vector after restriction digestion of both the vector and the purified PCR product with Sac I (MBI Fermentas, USA). The ligation product was transformed in *E. coli* Top 10F' and transformants obtained were scored on the basis of kanamycin resistance. Plasmid DNA isolated from few such colonies was analyzed for the presence of Chicken lysozyme MAR fragment by restriction digestion using various restriction enzymes. One such plasmid found to be having the correct integration in pZRC II vector was named pZRC II 1MAR (Sac) intermediate vector.

To add Mlu I overhang to another Chicken lysozyme MAR fragment, it was subjected to 40 cycles of PCR amplification using 100 picomoles of gene specific oligonucleotide primers in a volume of 50 µl containing 50 mM Tris-Cl (pH8.3), 2.5 mM MgCl₂, 250 µM each of the 4 dNTPs and 5 units of Pfu Polymerase. Each PCR amplification cycle consisted of incubations at 95° C for 30 sec (denaturation), 60° C for 30 sec (annealing) and 72° C for 2 min (extension). Amplified product of the PCR reaction was resolved on a 1% Agarose gel. The desired fragment of approx 1650 base pairs in size was excised out from the gel and purified using Qiagen Gel extraction kit. This purified DNA fragment was ligated into pZRC II-1MAR(Sac) vector after restriction digestion of both the vector and the purified PCR product with Mlu I (MBI Fermentas, USA). The ligation product was transformed in *E. coli* Top 10F' and transformants were scored on the basis of kanamycin resistance. Plasmid DNA isolated from about 10 such colonies was analyzed for the presence of Chicken lysozyme MAR fragment at the MluI position by restriction digestion using various restriction enzymes. One such plasmid found to be having the correct integration of the cLysMAR in MluI site in pZRC II-1MAR(Sac) vector was named **pZRC III** (Figure 2, Seq ID no 6).

### Example 2. Construction of pZRC III-TNK vector

Tenecteplase (TNKase or TNK-TPA) gene (Seq ID No7) was chemically synthesized and cloned into a cloning vector pMK (Geneart, Germany). To clone TNK gene in the pZRC II vector, first EcoR I and Not I overhangs were incorporated into the TNK gene using 40 cycles of PCR amplification using 100 picomoles of specific oligonucleotide primers containing the above restriction sites in a volume of 50 µl containing 50 mM Tris-Cl (pH8.3), 2.5 mM MgCl₂, 250 µM each of the 4 dNTPs and 5 units of Pfu Polymerase. Each PCR amplification cycle consisted of incubations at 95° C for 30 sec (denaturation), 60° C for 45 sec (annealing) and 72° C for 2 min (extension). Amplified product of the PCR reaction was resolved on a 1% Agarose gel. The desired fragment of approx 1710 base pairs in size was excised out from the gel and purified using Qiagen Gel extraction kit. This purified DNA fragment of TNK was digested with EcoR I and Not I and ligated into pZRC III vector (described in Example 1) digested with EcoR I and Not I (MBI Fermentas, USA). The ligation product was transformed in *E. coli* Top 10F' and transformants were scored on the basis of kanamycin resistance. Plasmid DNA isolated from about 10 such colonies was analyzed for the presence of TNK fragment by restriction digestion using various restriction enzymes. One such plasmid, having the TNK gene integrated in the pZRC III vector was named, pZRC III-TNK.

### Example 3. Construction of pZRC III-TNK-Hyg vector

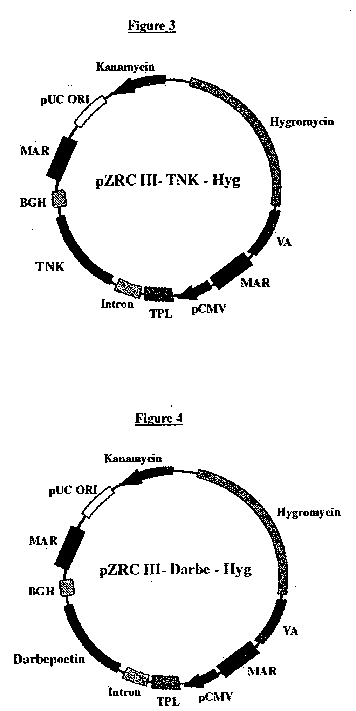

The Hygromycin transcription assembly of approx 1550 base pairs size and having the SV40 Promoter and terminator controlled Hygromycin resistance gene was blunt ended using Pfu polymerase (MBI Fermentas, USA) and then ligated into pZRC III-TNK vector, which was previously digested with Kpn I (MBI Fermentas, USA) and blunted using Pfu polymerase. The ligation product was transformed in *E. coli* Top 10F' and transformants were scored on the basis of kanamycin resistance. Plasmid DNA isolated from few such colonies was analyzed for the presence of Hygromycin resistance gene by restriction digestion using various restriction enzymes. One such plasmid having the Hygromycin transcription assembly integrated in pZRC III-TNK vector was named, pZRC III-TNK-Hyg vector (Figure 3, Seq ID No.8). This vector was then subjected to DNA sequencing using automated DNA sequencer (ABI) to verify the sequence of the cloned TNK gene.

### Example 4. Construction of pZRC III- Darbe-Hyg vector

pZRC - EPO (WO2007017903) was used as a template for carrying out site directed mutagenesis of the erythropoietin gene to obtain Darbepoetin gene fragment (Seq ID No.9 and the corresponding DNA sequence ID 21) of approx 600 by which was then cloned in TA vector pTZ57R (MBI Fermentas) and called, pTZ57R-Darbe.

pZRC III-TNK-Hyg was digested with Xho I and Not I to remove the TNK gene and the remaining high molecular weight DNA was used as the vector for ligation with Darbepoetin gene insert. pTZ57R-Darbe was digested with Xho I and Not I to gel isolate approx. 600 bp Darbepoetin gene fragment. Ligation of both the vector and insert was carried out and the ligation product was transformed in *E. coli* Top 10F' and transformants were scored on the basis of kanamycin resistance. Plasmid DNA isolated from few such colonies was analyzed for the presence of Darbepoetin gene by restriction digestion using various restriction enzymes. One such plasmid having the integrated Darbepoetin gene was named pZRC III-Darbe-Hyg vector (Figure 4, Seq ID No. 10). This vector was then subjected to DNA sequencing using automated DNA sequencer (ABI) to verify the sequence of the cloned Darbepoetin gene.

### Example 5. Construction of pZRC III- Etanercept -Hyg vector

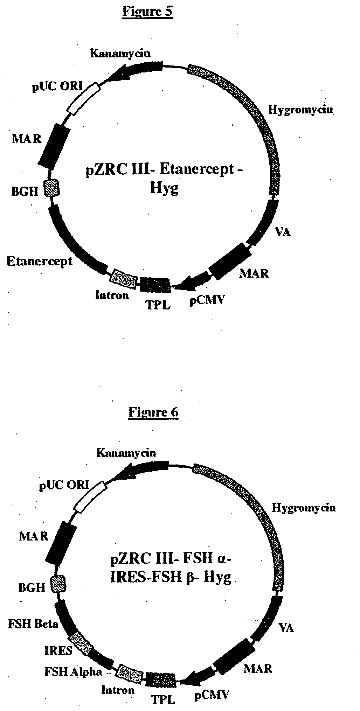

Vector pZRC III- Darbe-Hyg was digested with Xho I and Not I enzymes (MBI Fermentas) to remove the Darbepoetin gene of approx 600 bp and generate the vector backbone of approx 9430 bp. Chemically synthesized, CHO codon optimized, Etanercept gene (Seq ID No. 11 and corresponding DNA sequence ID 22) of approx. 1481 bp was isolated from the cloning vector using Xho I and Not I (MBI Fermentas) to obtain the insert. Ligation of both the vector and insert was carried out and the ligation product was transformed in *E. coli* Top 10F' and transformants were scored on the basis of kanamycin resistance. Plasmid DNA isolated from few such colonies was analyzed for the presence of Etanercept gene by restriction digestion using various restriction enzymes. One such plasmid having the integrated Etanercept gene was named pZRC III-Etanercept-Hyg vector (Figure 5, Seq ID No. 12). This vector was then subjected to DNA sequencing using automated DNA sequencer (ABI) to verify the sequence of the cloned Etanercept gene.

### Example 6. Construction of pZRC III- FSH α- IRES- FSH β - Hyg vector

### a) Construction of pZRC III- FSH a -Hyg vector

A vector backbone of approx. 9430 bp was generated by digesting pZRC III-Darbe-Hyg vector with Xho I and Not I (MBI Fermentas) to remove the approx. 600 bp of Darbepoetin gene. Chemically synthesized gene of FSH alpha subunit (Seq ID No. 13 the corresponding DNA sequence ID 23) of approx. 359 bp was isolated from the Geneart cloning vector pMA, using the enzymes Xho I and Not I (MBI Fermentas). Ligation of both the vector and insert was carried out and the ligation product was transformed in *E. coli* Top 10F' and transformants were scored on the basis of kanamycin resistance. Plasmid DNA isolated from few such colonies was analyzed for the presence of FSH alpha subunit gene by restriction digestion using various restriction enzymes. One such plasmid having the integrated FSH alpha subunit gene was named, PZRC III- **FSH α** -Hyg vector.

### b) Construction of pZRC III- FSH α- IRES- FSH β - Hyg vector

Vector pZRC III- **FSH a** -Hyg was digested with Not I (MBI Fermentas) to generate the vector backbone of approx. 9790 bp. An IRES gene fragment of approx. 591 bp (Seq ID No. 14) was isolated from the vector pIRES Hyg using the enzymes Not I and Xma I (MBI Fermentas), to obtain the first insert. Chemically synthesized gene of FSH beta subunit (Seq ID No. 15 the corresponding DNA sequence ID 24) of approx. 401 bp was isolated from the Geneart cloning vector pMA using the enzymes Xma I and Not I (MBI Fermentas), to obtain the second insert. The 2 inserts were fused and the fused gene product was ligated with the vector above. The ligation product was then transformed in *E. coli* Top 10F' and transformants were scored on the basis of kanamycin resistance. Plasmid DNA isolated from few such colonies was analyzed for the presence of IRES and FSH beta subunit genes by restriction digestion using various restriction enzymes. One such plasmid having the integrated IRES and FSH beta subunit genes was named pZRC **III- FSH α- IRES- FSH β - Hyg vector** (Figure 6, Seq ID No. 16). This vector was then subjected to DNA sequencing using automated DNA sequencer (ABI) to verify the sequence of the cloned FSH α and FSH β genes. The sequence of the cloned genes was confirmed by using automated DNA sequencer (ABI).

### Example 7. Construction of pZRC III- FSH β - IRES- FSH α - Hyg vector

### a) Construction of pZRC III- FSH β -Hyg vector

A vector pZRC III- FSH α - IRES- FSH β -Hyg was digested with Xho I and Not I (MBI Fermentas) to generate the vector backbone of approx. 9430 bp after removal of FSH α, IRES and FSH β genes. Chemically synthesized gene of FSH beta subunit of approx. 401 bp was isolated from the Geneart cloning vector pMA using the enzymes Xho I and Not I (MBI Fermentas). Ligation of both the vector and insert was carried out and the ligation product was transformed in *E. coli* Top 10F' and transformants were scored on the basis of kanamycin resistance. Plasmid DNA isolated from few such colonies was analyzed for the presence of FSH beta subunit gene by restriction digestion using various restriction enzymes. One such plasmid having the integrated FSH beta subunit gene was named pZRC III- **FSH β** -Hyg vector.

### b) Construction of pZRC III- FSH β - IRES- FSH α - Hyg vector

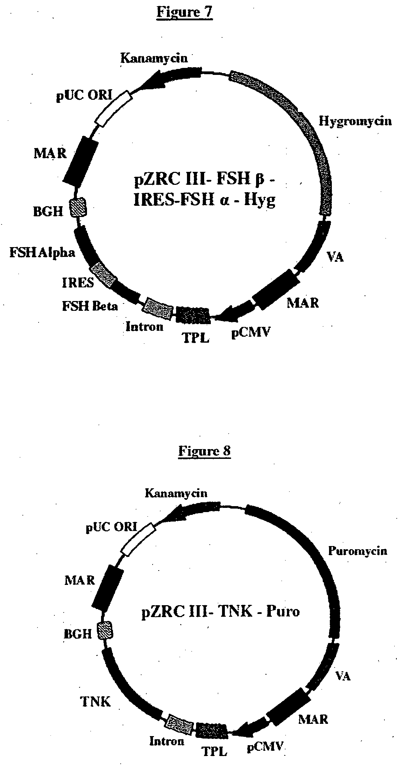

Vector pZRC III- **FSH β** -Hyg was digested with Not I (MBI Fermentas) to generate the vector backbone of approx. 9790 bp. An IRES gene fragment of approx. 591 bp was isolated from the vector pIRES Hyg using the enzymes Not I and Xho I (MBI Fermentas), to obtain the first insert (IRES). Chemically synthesized gene of FSH alpha subunit of approx 359 bp was isolated from the Geneart cloning vector pMA using the enzymes Xho I and Not I (MBI Fermentas), to obtain the second insert. This was followed by three piece ligation of the vector and the 2 inserts. The ligation product was then transformed in *E. coli* Top 10F' and transformants were scored on the basis of kanamycin resistance. Plasmid DNA isolated from few such colonies was analyzed for the presence of IRES and FSH alpha subunit genes by restriction digestion using various restriction enzymes. One such plasmid having the integrated IRES and FSH alpha subunit genes was named **pZRC III FSH β - IRES- FSH α - Hyg vector.** (Figure 7). The sequence of the cloned genes was confirmed by using automated DNA sequencer (ABI)

### Example 10. Construction of pZRC III- TNK-Puromycin vector

The Puromycin transcription assembly of approx. 1110 base pairs in size having the SV40 Promoter and terminator controlled Puromycin resistance gene and carrying BamH1 compatible ends ligated into pZRC III-TNK vector which was also digested with BamH I (MBI Fermentas, USA).The ligation product was transformed in *E. coli* Top 10F' and transformants were scored on the basis of kanamycin resistance. Plasmid DNA isolated from few such colonies was analyzed for the presence of Puromycin fragment by restriction digestion using various restriction enzymes. One such plasmid having the Puromycin transcription assembly integrated in pZRC III-TNK vector was named pZRC III- TNK-Puro vector (Figure 8, Seq ID No. 17). The sequence of the cloned TNK gene was confirmed by using automated DNA sequencer (ABI).

### Example 11. Construction of pZRC III- Darbepoetin-Puro vector

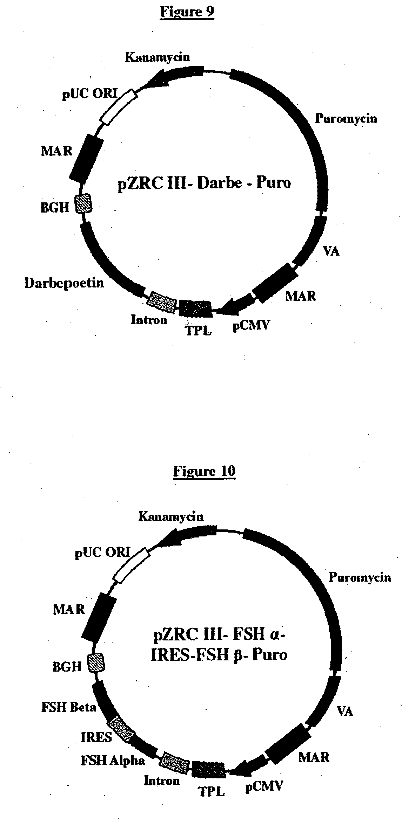

pZRC III-TNK-Puro was digested with Xho I and Not I (MBI Fermentas) to remove the TNK gene fragment. pTZ57R-Darbe was digested with Xho I and Not I to gel isolate approx. 600 bp Darbepoetin gene fragment. Ligation of both the vector and insert was done. The ligation product was transformed in *E. coli* Top 10F' and transformants were scored on the basis of kanamycin resistance. Plasmid DNA isolated from few such colonies was analyzed for the presence of Darbepoetin gene fragment by restriction digestion using various restriction enzymes. One such plasmid was having the integrated Darbepoetin gene named pZRC III-Darbe-Puro vector (Figure 9, Seq ID No. 18). The sequence of the cloned Darbepoietin gene was confirmed by using automated DNA sequencer (ABI).

### Example 12. Construction of pZRC III- FSH α- IRES- FSH β - Puro vector

Hygromycin cassette from pZRC III- FSH α - IRES- FSH β -Hyg vector was removed and replaced with the Puromycin transcription assembly of approx. 1110 base pairs in size having the SV40 Promoter and terminator controlled Puromycin resistance gene amplified from pZRC III - Darbe - Puro using a PCR reaction with specific oligonucleotide primers. The obtained PCR product was digested using specific endonucleases and used for further ligations with the vector backbone. The ligation product was transformed in *E. coli* Top 10F' and transformants were scored on the basis of kanamycin resistance. Plasmid DNA isolated from few such colonies was analyzed for the presence of Puromycin resistance gene by restriction digestion using various restriction enzymes. One such plasmid having the integrated Puromycin resistant gene was named **pZRC III- FSH α- IRES- FSH β - Puro vector** (Figure 10)

### Example 13. Construction of pZRC III- FSH α- IRES- FSH β - Neo vector

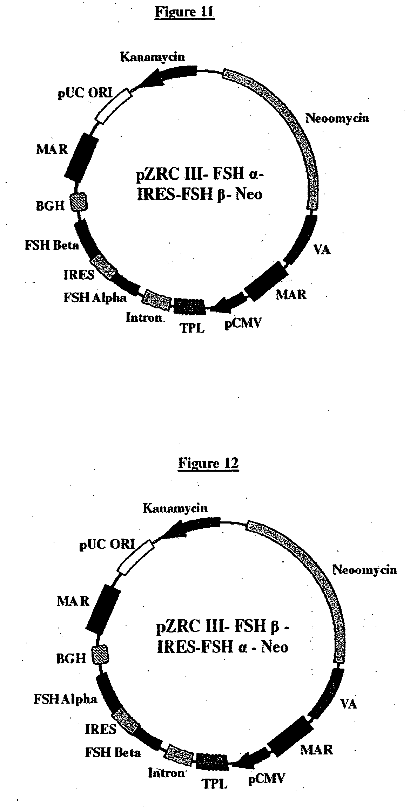

Vector pZRC III- FSH α - IRES- FSH β -Puro was digested with Pac I and Bam HI (MBI Fermentas) to generate the vector backbone of approx. 9980 bp after removal of Puromycin resistant gene. Neomycin resistant gene of approx. 1518 bp was isolated from pcDNA 3.1 (Invitrogen) plasmid. Ligation of both the vector and insert was carried out and the ligation product was transformed in *E. coli* Top 10F' and transformants were scored on the basis of kanamycin resistance. Plasmid DNA isolated from few such colonies was analyzed for the presence of Neomycin resistant gene by restriction digestion using various restriction enzymes. One such plasmid having the integrated Neomycin resistant subunit gene was named **pZRC III- FSH α- IRES- FSH β - Neo vector** (Figure 11). The sequence of FSH α, IRES and FSH β genes was confirmed by using automated DNA sequencer (ABI).

### Example 14. Construction of pZRC III- FSH P- IRES- FSH α - Neo vector

### a) Construction of pZRC III- FSH β -Neo vector

Vector pZRC III- FSH α - IRES- FSH β -Neo was digested with Xho I and Not I (MBI Fermentas) to generate the vector backbone of approx. 9400 bp after removal of FSH α, IRES and FSH β genes. Chemically synthesized gene of FSH beta subunit of approx. 401 bp was isolated from the Geneart cloning vector pMA using the enzymes Xho I and Not I (MBI Fermentas). Ligation of both the vector and insert was carried out and the ligation product was transformed in *E. coli* Top 10F' and transformants were scored on the basis of kanamycin resistance. Plasmid DNA isolated from few such colonies was analyzed for the presence of FSH beta subunit gene by restriction digestion using various restriction enzymes. One such plasmid having the integrated FSH beta subunit gene was named pZRC III- **FSH P** -Neo vector.

### b) Construction of pZRC III FSH β - IRES- FSH α - Neo vector

Vector pZRC III- **FSH β** -Neo was digested with Not I (MBI Fermentas) to generate the vector backbone of approx. 9800 bp. The IRES DNAfragment of approx. 591 bp was isolated from the vector pIRES Hyg using the enzymes Not I and Xho I (MBI Fermentas), to obtain the first insert. Chemically synthesized gene of FSH alpha subunit of approx. 359 bp was isolated from the Geneart vector pMA using the enzymes Xho I and Not I (MBI Fermentas), to obtain the second insert. This was followed by three piece ligation of the vector and the 2 inserts. The ligation product was then transformed in *E. coli* Top 10F' and transformants were scored on the basis of kanamycin resistance. Plasmid DNA isolated from few such colonies was analyzed for the presence of IRES and FSH alpha subunit genes by restriction digestion using various restriction enzymes. One such plasmid having the integrated IRES and FSH alpha subunit genes was named obtain **pZRC III FSH β - IRES- FSH α - Neo** vector (Figure 12). The sequence of the cloned genes was confirmed by using automated DNA sequencer (ABI).

### Example 17. Construction of pZRC III- Etanercept -Neo vector

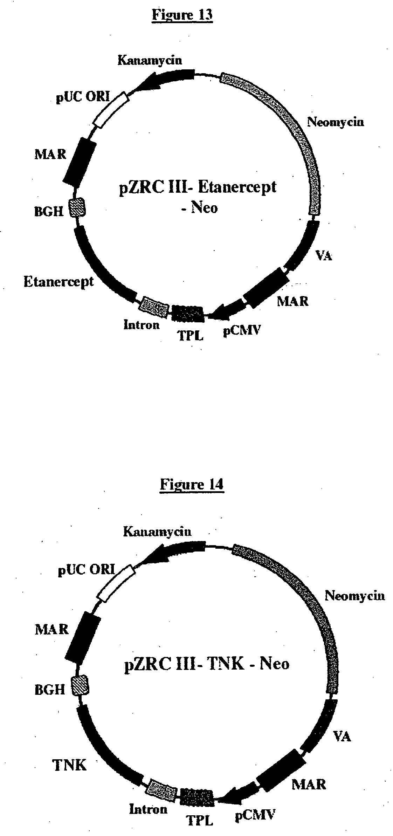

Vector pZRC III- FSH α- IRES- FSH β - Neo was digested with Xho I and Not I (MBI Fermentas) enzymes to remove the FSH α, IRES and FSH β genes, and obtain the vector backbone of approx.9400 bp to be used for cloning the Etanercept gene. The approx.1481 bp gene of Etanercept was isolated from the vector pZRC III-Etanercept-Hyg using Xho I and Not I (MBI Fermentas) enzymes to obtain the insert. Ligation of both the vector and insert was carried out and the ligation product was transformed in *E. coli* Top 10F' and transformants were scored on the basis of kanamycin resistance. Plasmid DNA isolated from about few colonies was analyzed for the presence of Etanercept gene by restriction digestion using various restriction enzymes. One such plasmid having the integrated Etanercept gene was named pZRC III-Etanercept-Neo vector (Figure 13, Seq ID No. 19). The sequence of the cloned Etanercept gene was confirmed by using automated DNA sequencer (ABI).

### Example 18. Construction of pZRC III- TNK- Neo vector

Vector pZRC III-Etanercept-Neo was digested with the enzymes Xho I and Not I (MBI Fermentas) to remove the approx.1481 Etanercept gene and obtain the vector construct of approx. 9400 bp. The insert of TNK gene of approx.1692 bp was obtained after digesting the vector **pZRC III- TNK- Hyg** with Xho I and Not I enzymes (MBI Fermentas). Ligation of both the vector and insert was carried out and the ligation product was transformed in *E. coli* Top 10F' and transformants were scored on the basis of kanamycin resistance. Plasmid DNA isolated from few such colonies was analyzed for the presence of TNK gene by restriction digestion using various restriction enzymes. One such plasmid having the integrated TNK gene was named pZRC III-TNK-Neo vector (Figure 14,). The sequence of the cloned TNK gene was confirmed by using automated DNA sequencer (ABI).

### Example 19. Expression of Etanercept

### Set I - Stable transfections in CHO-K1-S cell line using pZRC III-Etanercept-Hyg vector

Freestyle^{™} CHO-K1-S cell were cultivated routinely in PowerCHO2 CD medium (chemically defined medium, Lonza) supplemented with 4 mM Glutamine. Cells were maintained under agitation (120 rpm) at 37°C, and 5% CO2 in a humidified incubator. Cells were counted every 3rd/4th day and given a complete medium exchange. Transfections were carried out using Neon Transfection system (Invitrogen). One day prior to transfection, CHO-KI-S cells were passaged into fresh medium and allowed at least one doubling before use for transfection. Transfections were carried out using Sgs I (Asc I) linearised pZRC III-Etanercept-Hyg plasmid as per standard protocols described by the manufacturer (Invitrogen). After Transfection, the cells were transferred into one well of a 24 well plate, containing 1 mL of pre-warmed culture medium. Cells were maintained at 37°C, 5% CO2 in a humidified incubator. On the next day, for minipool generation, transfected population was plated in 96 well plates in Pro CHO 5 medium (Lonza) supplemented with 4 mM Glutamine and 600 µg/ml of Hygromycin. After 15-30 days, supernatants from 96 well plates were removed for product formation analysis by ELISA. The selected high expressing minipools were then transferred to 24 well plate and subsequently to 6 well plate in PowerCHO2 CD medium (chemically defined medium, Lonza) supplemented with 4 mM Glutamine and 600 µg/ml of Hygromycin and expression levels were analyzed at each level by ELISA. High expressing minipools were chosen to carry out single cell dimiting dilution in 96 well plates in Pro CHO 5 medium (Lonza) supplemented with 4 mM Glutamine and 600 µg/ml of Hygromycin. After around 15-30 days, supernatants from 96 well plates were removed for product formation analysis by ELISA. The selected high expressing clones were then transferred to 24 well plate and then to 6 well plate in PowerCHO2 CD medium (chemically defined medium, Lonza) supplemented with 4 mM Glutamine and 600 µg/ml of Hygromycin and expression levels were analyzed at each level by ELISA. High producing clones were selected for re transfections.

### Set II - Stable retransfections of clones obtained from Set I using pZRC III-Etanercept-Neo vector

High expressing clones were chosen to carry out re-transfections using pZRC III-Etanercept-Neo plasmid linearized by Sgs I (Acs I) by the same procedure as in Set I transfections. On the next day, for minipool generation, transfected population was plated in 96 well plates in Pro CHO 5 medium (Lonza) supplemented with 4 mM Glutamine, 600 µg/ml of Hygromycin, and 500 µg/ml of Neomycin. After 15-30 days, supernatants from 96 well plates were removed for product formation analysis by ELISA. The selected high expressing minipools were then transferred to 24 well plate and then to 6 well plate in PowerCHO2 CD medium (chemically defined medium, Lonza) supplemented with 4 mM Glutamine and said antibiotic pressures and expression levels were analyzed at each level by ELISA. High producing minipools were chosen to carry out single cell limiting dilution in 96 well plates in Pro CHO 5 medium (Lonza) supplemented with 4 mM Glutamine. Again after 15-30 days, supernatants from 96 well plates were removed for product formation analysis by ELISA. The selected high expressing clones were then transferred to 24 well plate and then to 6 well plate in PowerCHO2 CD medium (chemically defined medium, Lonza) supplemented with 4 mM Glutamine and expression levels were analyzed at each level by ELISA. High expressing retransfected clones were selected to analyse the product formation in shake tubes in fed batch mode. These experiments were carried out using these selected clones in spin tubes on shaker (Kuhner-Germany) at 120 rpm, 37°C, 5% CO₂. Clones yielded production levels in the range of approx. 700 mg/l to 1000 mg/l in 12 days.

### Example 20. Expression of TNK

### Set I - Stable transfections in CHO-K1-S cell line using pZRC III-TNK-Hyg vector

Freestyle^{™} CHO-K1-S cell were cultivated routinely in PowerCHO2 CD medium (chemically defined medium, Lonza) supplemented with 4 mM Glutamine. Cells were maintained under agitation (120 rpm) at 37°C, and 5% CO2 in a humidified incubator. Cells were counted every 3rd/4th day and given a complete medium exchange. Transfections were carried out using Neon Transfection system (Invitrogen). One day prior to transfection, CHO-KI-S cells were passaged into fresh medium and allowed at least one doubling before use for transfection. Transfections were carried out using Sgs I (Asc I) linearised pZRC III-TNK-Hyg plasmid as per standard protocols described by the manufacturer (Invitrogen). After Transfection, the cells were transferred into one well of a 24 well plate, containing 1 mL of pre-warmed culture medium. Cells were maintained at 37°C, 5% CO2 in a humidified incubator. On the next day, for minipool generation, transfected population was plated in 96 well plates in PowerCHO2 CD medium (chemically defined medium, Lonza) supplemented with 4 mM Glutamine and 500 µg/ml of Hygromycin. After 15-30 days, supernatants from 96 well plates were removed for product formation analysis by ELISA. The selected high expressing minipools were then transferred to 24 well plate and subsequently to 6 well plate and expression levels were analyzed at each level by ELISA. High expressing minipools were chosen to carry out single cell dimiting dilution in 96 well plates in PowerCHO2 CD medium (chemically defined medium, Lonza) supplemented with 4 mM Glutamine and 500 µg/ml of Hygromycin. After around 15-30 days, supernatants from 96 well plates were removed for product formation analysis by ELISA. The selected high expressing clones were then transferred to 24 well plate and then to 6 well plate and expression levels were analyzed at each level by ELISA. High producing clones were selected for re transfections. High expressing clones were selected to analyse the product formation in shake tubes in fed batch mode. These experiments were carried out using these selected clones in 10 ml media in spin tubes on shaker (Kuhner-Germany) at 230 rpm, 37°C, 5% CO₂. Clone yielded productions levels of 150 mg/l in 9 days.

### Set IIa - Stable re-transfections of clones obtained from Set I using pZRC III-TNK-Puro vector

High expressing clones were chosen to carry out re-transfections using pZRC III-TNK-Puro plasmid linerised by Sgs I (Acs I) by the same procedure as in Set I transfections. On the next day, for minipool generation, transfected population was plated in 96 well plates in PowerCHO2 CD medium (chemically defined medium, Lonza) supplemented with 4 mM Glutamine, 500 µg/ml of Hygromycin, and 3 ug/ml of Puromycin. After 15-30 days, supernatants from 96 well plates were removed for product formation analysis by ELISA. The selected high expressing minipools were then transferred to 24 well plate and then to 6 well plate and expression levels were analysed at each level by ELISA. High producing minipools were chosen to carry out single cell limiting dilution in 96 well plates in PowerCHO2 CD medium (chemically defined medium, Lonza) supplemented with 4 mM Glutamine. Again after 15-20 days, supernatants from 96 well plates were removed for product formation analysis by ELISA. The selected high expressing clones were then transferred to 24 well plate and then to 6 well plate and expression levels were analysed at each level by ELISA. High expressing clones were selected to analyse the product formation in shake tubes in fed batch mode. These experiments were carried out using these selected clones in spin tubes on shaker (Kuhner-Germany) at 120 rpm, 37°C, 5% CO₂. Clones yielded productions levels of 290 mg/l in 11 days.

### Set IIb - Stable re-transfections of clones obtained from Set I using pZRC III-TNK-Neo vector

High producing clones were chosen to carry out re-transfections using pZRC III-TNK-Neo plasmid linearized by Sgs I (Acs I) by the same procedure as in Set I transfections. On the next day, for minipool generation, transfected population was plated in 96 well plates in Pro CHO 5 medium (Lonza) supplemented with 4 mM Glutamine, 600 µg/ml of Hygromycin, and 500 µg/ml of Neomycin. After 15-30 days, supernatants from 96 well plates were removed for product formation analysis by ELISA. The selected high expressing minipools were then transferred to 24 well plate and then to 6 well plate in PowerCHO2 CD medium (chemically defined medium, Lonza) supplemented with 4 mM Glutamine and the mentioned antibiotic pressures and expression levels were analyzed at each level by ELISA. High producing minipools were chosen to carry out single cell limiting dilution in 96 well plates in Pro CHO 5 medium (Lonza) supplemented with 4 mM Glutamine. Again after 15-30 days, supernatants from 96 well plates were removed for product formation analysis by ELISA. The selected high expressing clones were then transferred to 24 well plate and then to 6 well plate in PowerCHO2 CD medium (chemically defined medium, Lonza) supplemented with 4 mM Glutamine and expression levels were analyzed at each level by ELISA. High expressing clones were selected to analyze the product formation in shake tubes in fed batch mode. These experiments were carried out using these selected clones in spin tubes on shaker (Kuhner-Germany) at 120 rpm, 37°C, 5% CO₂. Clone yielded productions levels of 390 mg/l in 10 days.

### Example 21. Expression of Darbepoetin using pZRC III-Darbe-Hyg vector

### Set I - Stable transfections in CHO-K1-S cell line using pZRC III Darbe-Hyg vector

Freestyle^{™} CHO-K1-S cell were cultivated routinely in PowerCHO2 CD medium (chemically defined medium, Lonza) supplemented with 4 mM Glutamine. Cells were maintained under agitation (120 rpm) at 37°C, and 5% CO2 in a humidified incubator. Cells were counted every 3rd/4th day and given a complete medium exchange. Transfections were carried out using Neon Transfection system (Invitrogen). One day prior to transfection, CHO-KI-S cells were passaged into fresh medium and allowed at least one doubling before use for transfection. Transfections were carried out using Sgs I (Asc I) linearised pZRC III-Darbe-Hyg plasmid as per standard protocols described by the manufacturer (Invitrogen). After transfection of DNA, the cells were transferred into one well of a 24 well plate, containing 1 mL of pre-warmed culture medium. Cells were maintained at 37°C, 5% CO2 in a humidified incubator. On the next day, for minipool generation, transfected population was plated in 96 well plates in PowerCHO2 CD medium (chemically defined medium, Lonza) supplemented with 4 mM Glutamine and 600 µg/ml of Hygromycin. After 15-20 days, supernatants from 96 well plates were removed for product formation analysis by ELISA. The selected high expressing minipools were then transferred to 24 well plate and then to 6 well plate and expression levels were analyzed at each level by ELISA. Two high expressing minipools were chosen to carry out single cell limiting dilution in 96 well plates in ProCHO5 medium (Lonza) supplemented with 4 mM Glutamine and 600 µg/ml of Hygromycin. Again after 15-30 days, supernatants from 96 well plates were removed for product formation analysis by ELISA. The selected high expressing clones were then transferred to 24 well plate and then to 6 well plate and expression levels were analyzed at each level by ELISA. High expressing clones were selected to analyse the product formation in shake tubes in fed batch mode. These experiments were carried out using these selected clones in 10 ml media in spin tubes on shaker (Kuhner-Germany) at 230 rpm, 37°C, 5% CO2. Clone yielded productions levels of 340 mg/l in 11 days.

### Example 22. Expression of FSH

### Set I - Stable transfections in CHO-K1-S cell line using pZRC III- FSH α- IRES-FSH β- Hyg vector

Freestyle^{™} CHO-K1-S cell were cultivated routinely in PowerCHO2 CD medium (chemically defined medium, Lonza) supplemented with 4 mM Glutamine. Cells were maintained under agitation (120 rpm) at 37°C, and 5% CO2 in a humidified incubator. Cells were counted every 3rd/4th day and given a complete medium exchange. Transfections were carried out using Neon Transfection system (Invitrogen). One day prior to transfection, CHO-KI-S cells were passaged into fresh medium and allowed at least one doubling before use for transfection. Transfections were carried out using Sgs I (Asc I) linearised **pZRC III- FSH α- IRES- FSH β - Hyg**_plasmid as per standard protocols described by the manufacturer (Invitrogen). After Transfection, the cells were transferred into one well of a 24 well plate, containing 1 mL of pre-warmed culture medium. Cells were maintained at 37°C, 5% CO2 in a humidified incubator. On the next day, for minipool generation, transfected population was plated in 96 well plates in Pro CHO 5 medium (Lonza) supplemented with 4 mM Glutamine and 600 µg/ml of Hygromycin. After 15-30 days, supernatants from 96 well plates were removed for product formation analysis by ELISA. The selected high expressing minipools were then transferred to 24 well plate and subsequently to 6 well plate in PowerCHO2 CD medium (chemically defined medium, Lonza) supplemented with 4 mM Glutamine and said antibiotic pressure and expression levels were analyzed at each level by ELISA. High expressing minipools were chosen to carry out single cell limiting dilution in 96 well plates in Pro CHO 5 medium (Lonza) supplemented with 4 mM Glutamine and 600 µg/ml of Hygromycin. After around 15-30 days, supernatants from 96 well plates were removed for product formation analysis by ELISA. The selected high expressing clones were then transferred to 24 well plate and then to 6 well plate in PowerCHO2 CD medium (chemically defined medium, Lonza) supplemented with 4 mM Glutamine and said antibiotic pressure and expression levels were analyzed at each level by ELISA. High producing clones were selected for retransfections.

### Set II - Stable re-transfections of clones obtained from Set I using pZRC III- FSH α- IRES FSH β- Neo vector

Clones were chosen to carry out re-transfections using **pZRC III- FSH α-IRES- FSH β- Neo**_plasmid linearized by Sgs I (Acs I) by the same procedure as Set I transfections. On the next day, for minipool generation, transfected population was plated in 96 well plates in Pro CHO 5 medium (Lonza) supplemented with 4 mM Glutamine, 600 µg/ml of Hygromycin, and 500 µg/ml of Neomycin. After 15-30 days, supernatants from 96 well plates were removed for product formation analysis by ELISA. The selected high expressing minipools were then transferred to 24 well plate and then to 6 well plate in PowerCHO2 CD medium (chemically defined medium, Lonza) supplemented with 4 mM Glutamine and said antibiotic pressures and expression levels were analyzed at each level by ELISA High producing retransfected minipools were chosen to carry out single cell limiting dilution in 96 well plates in Pro CHO 5 medium (Lonza) supplemented with 4 mM Glutamine and mentioned antibiotic pressure. Again after 15-30 days, supernatants from 96 well plates were removed for product formation analysis by ELISA. The selected high expressing clones were then transferred to 24 well plate and then to 6 well plate in PowerCHO2 CD medium (chemically defined medium, Lonza) supplemented with 4 mM Glutamine and mentioned antibiotic pressure and expression levels were analyzed at each level by ELISA. High expressing clones namely were selected to analyse the product formation in shake tubes in fed batch mode. These experiments were carried out using these selected clones in spin tubes on shaker (Kuhner-Germany) at 120 rpm, 37°C, 5% CO₂. Production levels were obtained in range of approx. 20 mg/l to 50 mg/l in 10 days with different clones.

## Claims

1. An expression vector comprising a promoter operably linked to the gene of interest, expression enhancement element, TPL, VA I and II genes, translation terminator and an antibiotic marker wherein the expression enhancement element is a matrix attachment region that is cLysMARs having the nucleotide sequence of SEQ ID NO:5.

2. The expression vector as claimed in claim 1 which further comprises
a) Intron or variants thereof; and
b) optionally an Internal ribosomal binding site or variants thereof.

3. The expression vector as claimed in claim 1 wherein the promoter is selected from the group consisting of CMV promoter, SV40 promoter, adenovirus promoter, Beta actin promoter, metallothionin Promoters or other prokaryotic or eukaryotic virus promoters.

4. The expression vector as claimed in claim 2 wherein the internal ribosomal binding site is Encephalomyocarditis virus IRES.

5. The expression vector as claimed in claim 2 and 4 wherein the internal ribosomal binding site has nucleotide sequence set forth in sequence id no 14.

6. The expression vector as claimed in claim 1 wherein the VA I and II genes have nucleotide sequence set forth in sequence id no 3.

7. The expression vector as claimed in claim 1 wherein the TPL has nucleotide sequence set forth in sequence id no. 2.

8. The expression vector as claimed in claim 2 wherein the chimeric Intron has nucleotide sequence set forth in sequence id no. 1.

9. The expression vector as claimed in claim 1 wherein the gene of interest encodes proteins and peptides and analogues thereof selected from tissue plasminogen activator, TNK-TPA, Darbepoietin, Erythropoietin, Insulin, GCSF, Interleukin, Tumor necrosis factor, Interferon, TNFR-IgGFc, monoclonal antibodies selected from rituximab, bevacizumab, adalimumab, trastuzumab and their fragments like Fc region, Fab, GLP-I, GLP-II, IGF-I, IGF-II, Platelet derived growth factor, FVII, FVIII, FIV and FXIII, exendin-3, exendin 4, transcription factors like MYT-2, NF-κB repressing factor NRF, AML1/RUNX1, Gtx homeodomain protein, translation factors selected from Eukaryotic initiation factor 4G (eIF4G)a, Eukaryotic initiation factor 4G1 (eIF4GI)a, Death associated protein 5 (DAP5), oncogene like c-myc, L-myc, Pim-1, Protein kinase p58PITSLRE, p53 hormones selected from gonadotropic hormones selected from Follicle stimulating hormone, Human Chorionic Gonadotropin, Human Luteinizing Hormone, and immunoglobulin heavy chain binding protein (BiP), Heat shock protein 70, β-subunit of mitochondrial H+- ATP synthase, Ornithine decarboxylase, connexins 32 and 43, HIF-Ia, APC.

10. The expression vector as claimed in any of the preceding claims wherein the cLysMARs is cloned at either flank of the expression cassette.

11. The expression vector as claimed in claims 1 and 2 which further comprises a chimeric intron and a Bovine growth hormone polyadenylation sequence.

12. The expression vector as claimed in any of preceding claims having accession number MTCC 5655, MTCC 5656, or MTCC 5657.

13. A host cell transformed with a vector as claimed in any of preceding claims.

14. A process for production of proteins and peptides and variants thereof comprising
a) constructing an expression vector as claimed in any of the preceding claims; and
b) transformation of said expression vector in suitable host cell which expresses the protein or peptide of interest.

15. A process for production of proteins and peptides and variants thereof comprising
a) constructing an expression vector as claimed in any of the preceding claims;
b) transfection of said expression vector in a suitable host cell;
c) selecting suitable transfected host cell expressing a protein or peptide of interest;
d) suitable host cell selected in step (c) further retransfected with expression vector as claimed in any of preceding claims;
e) suitable retransfected host cell expressing a protein or peptide of interest.

16. The process as claimed in claim 14 and 15 wherein the expression vector has accession number MTCC 5655, MTCC 5656, or MTCC 5657.

17. The process as claimed in claim 14 and 15 wherein the proteins and peptides are selected from tissue plasminogen activator,TNK-TPA, Darbepoietin, Erythropoietin, Insulin, GCSF, Interleukin, Tumor necrosis factor, Interferon, TNFR-IgGFc, Monoclonal antibodies such as rituximab, bevacizumab, adalimumab, trastuzumab and their fragments like Fc region, Fab, GLP-I, GLP-II, IGF-I, IGF-II, Platelet derived growth factor, FVII, FVIII, FIV and FXIII, exendin-3, exendin 4, hormones such as gonadotropic hormones selected from Follicle stimulating hormone, Human Chorionic Gonadotropin, Human Luteinizing Hormone.

## Patentansprüche

1. Expressionsvektor, umfassend einen Promotor, der mit dem Gen von Interesse, dem Expressionsverstärkungselement, TPL, VA-I- und -II-Genen, dem Translationsterminator und einem antibiotischen Marker wirkverbunden ist, wobei das Expressionsverstärkungselement eine Matrixanheftungsregion ist, die cLysMARs mit der Nukleotidsequenz SEQ ID NR: 5 ist.

2. Expressionsvektor nach Anspruch 1, der ferner Folgendes umfasst:
a) ein Intron oder Varianten davon; und
b) optional eine interne ribosomale Bindungsstelle oder Varianten davon.

3. Expressionsvektor nach Anspruch 1, wobei der Promotor ausgewählt ist aus der Gruppe bestehend aus CMV-Promotor, SV40-Promotor, Adenovirus-Promotor, Beta-Actin-Promotor, Metallothionein-Promotoren oder anderen prokaryotischen oder eukaryotischen Virus-Promotoren.

4. Expressionsvektor nach Anspruch 2, wobei die interne ribosomale Bindungsstelle Enzephalomyokarditisvirus IRES ist.

5. Expressionsvektor nach Anspruch 2 und 4, wobei die interne ribosomale Bindungsstelle die nach Sequenz-ID NR: 14 festgelegte Nukleotidsequenz aufweist.

6. Expressionsvektor nach Anspruch 1, wobei die VA-I- und -II-Gene die nach Sequenz-ID NR: 3 festgelegte Nukleotidsequenz aufweisen.

7. Expressionsvektor nach Anspruch 1, wobei der TPL die nach Sequenz-ID NR: 2 festgelegte Nukleotidsequenz aufweist.

8. Expressionsvektor nach Anspruch 2, wobei das chimäre Intron die nach Sequenz-ID NR: 1 festgelegte Nukleotidsequenz aufweist.

9. Expressionsvektor nach Anspruch 1, wobei das Gen von Interesse für Proteine und Peptide und Analoga davon codiert, ausgewählt aus Gewebsplasminogenaktivator, TNK-TPA, Darbepoetin, Erythropoetin, Insulin, GCSF, Interleukin, Tumor-Nekrose-Faktor, Interferon, TNFR-IgGFc, monoklonalen Antikörpern, ausgewählt aus Rituximab, Bevacizumab, Adalimumab, Trastuzumab und deren Fragmenten wie Fc-Region, Fab, GLP-I, GLP-II, IGF-I, IGF-II, Platelet-derived Growth Factor, FVII, FVIII, FIV und FXIII, Exendin-3, Exendin-4, Transkriptionsfaktoren wie MYT-2, NF-κB-Repressionsfaktor NRF, AML1/RUNX1, Gtx-Homeodomain-Protein, Translationsfaktoren, ausgewählt aus eukaryotischem Initiationsfaktor 4G (eIF4G)a, eukaryotischem Initiationsfaktor 4G1 (eIF4GI)a, Death-associated Protein 5 (DAP5), onkogenähnlichem c-Myc, L-Myc, Pim-1, Proteinkinase p58PITSLRE, p53-Hormonen, ausgewählt aus Gonadotropinhormonen, ausgewählt aus follikelstimulierendem Hormon, humanem Choriongonadotropin, humanem luteinisierendem Hormon, und schwerkettigem Immunglobulinbindungsprotein (BiP), Hitzeschockprotein 70, β-Untereinheit von mitochondrialer H+-ATP-Synthase, Ornithindecarboxylase, Connexinen 32 und 43, HIF-la, APC.

10. Expressionsvektor nach einem der vorhergehenden Ansprüche, wobei das cLysMARs an beiden Flanken der Expressionskassette geklont ist.

11. Expressionsvektor nach Anspruch 1 und 2, der ferner ein chimäres Intron und eine Rinderwachstumshormon-Polyadenylierungssequenz umfasst.

12. Expressionsvektor nach einem der vorhergehenden Ansprüche mit der Zugangsnummer MTCC 5655, MTCC 5656 oder MTCC 5657.

13. Mit einem Vektor nach einem der vorhergehenden Ansprüche transformierte Wirtszelle.

14. Vorgang zum Herstellen von Proteinen und Peptiden und Varianten davon, Folgendes umfassend:
a) Gestalten eines Expressionsvektors nach einem der vorhergehenden Ansprüche; und
b) Transformieren des Expressionsvektors in eine geeignete Wirtszelle, die das Protein oder das Peptid von Interesse exprimiert.

15. Vorgang zum Herstellen von Proteinen und Peptiden und Varianten davon, Folgendes umfassend:
a) Gestalten eines Expressionsvektors nach einem der vorhergehenden Ansprüche;
b) Transfizieren des Expressionsvektors in eine geeignete Wirtszelle;
c) Auswählen einer geeigneten transfizierten Wirtszelle, die ein Protein oder ein Peptid von Interesse exprimiert;
d) geeignete Wirtszelle, ausgewählt in Schritt (c), ferner transfiziert mit einem Expressionsvektor nach einem der vorhergehenden Ansprüche;
e) geeignete retransfizierte Wirtszelle, die ein Protein oder ein Peptid von Interesse exprimiert.

16. Vorgang nach Anspruch 14 und 15, wobei der Expressionsvektor die Zugangsnummer MTCC 5655, MTCC 5656 oder MTCC 5657 aufweist.

17. Vorgang nach Anspruch 14 und 15, wobei die Proteine und Peptide ausgewählt sind aus Gewebsplasminogenaktivator, TNK-TPA, Darbepoetin, Erythropoetin, Insulin, GCSF, Interleukin, Tumor-Nekrose-Faktor, Interferon, TNFR-IgGFc, monoklonalen Antikörpern wie etwa Rituximab, Bevacizumab, Adalimumab, Trastuzumab und deren Fragmenten wie Fc-Region, Fab, GLP-I, GLP-II, IGF-I, IGF-II, Platelet-derived Growth Factor, FVII, FVIII, FIV und FXIII, Exendin-3, Exendin-4, Hormonen wie etwa Gonadotropinhormonen, ausgewählt aus follikelstimulierendem Hormon, humanem Choriongonadotropin, humanem luteinisierendem Hormon.

## Revendications

1. Vecteur d'expression comprenant un promoteur opérationnellement lié au gène d'intérêt, à l'élément de stimulation de l'expression, à TPL, aux gènes VA I et II, un terminateur de la traduction et un marqueur de résistance aux antibiotiques, dans lequel l'élément de stimulation de l'expression est une région de fixation de matrice qui est cLysMAR ayant la séquence nucléotidique de SÉQ ID n° : 5.

2. Vecteur d'expression selon la revendication 1, qui comprend en outre :
a) un intron ou des variants de celui-ci ; et
b) facultativement un site interne d'attachement du ribosome ou des variants de celui-ci.

3. Vecteur d'expression selon la revendication 1, dans lequel le promoteur est choisi dans le groupe constitué par le promoteur CMV, le promoteur SV40, le promoteur de l'adénovirus, le promoteur de l'actine bêta, les promoteurs de la métallothionéine ou d'autres promoteurs des virus de procaryotes ou d'eucaryotes.

4. Vecteur d'expression selon la revendication 2, dans lequel le site interne d'attachement du ribosome est l'IRES du virus de l'encéphalomyocardite.

5. Vecteur d'expression selon les revendications 2 et 4, dans lequel le site interne d'attachement du ribosome a la séquence nucléotidique indiquée dans la séquence SÉQ ID n° : 14.

6. Vecteur d'expression selon la revendication 1, dans lequel les gènes VA I et II ont la séquence nucléotidique indiquée dans la séquence SÉQ ID n° : 3.

7. Vecteur d'expression selon la revendication 1, dans lequel la TPL a la séquence nucléotidique indiquée dans la séquence SÉQ ID n° : 2.

8. Vecteur d'expression selon la revendication 2, dans lequel l'intron chimérique a la séquence nucléotidique indiquée dans la séquence SÉQ ID n° : 1.

9. Vecteur d'expression selon la revendication 1, dans lequel le gène d'intérêt code pour les protéines et les peptides et les analogues de ceux-ci choisis parmi l'activateur tissulaire du plasminogène, TNK-TPA, la darbepoïétine, l'érythropoïétine, l'insuline, GCSF, l'interleukine, le facteur de nécrose tumorale, l'interféron, TNFR-IgGFc, les anticorps monoclonaux choisis parmi le rituximab, le bévacizumab, l'adalimumab, le trastuzumab et leurs fragments tels que la région Fc, Fab, GLP-I, GLP-II, IGF-I, IGF-II, le facteur de croissance dérivé des plaquettes, FVII, FVIII, FIV et FXIII, l'exendine-3, l'exendine-4, les facteurs de transcription tels que MYT-2, le facteur de répression du NF-κB NRF, AML1/RUNX1, la protéine de l'homéodomaine Gtx, les facteurs de traduction choisis parmi le facteur d'initiation eucaryote 4G (eIF4G)a, le facteur d'initiation eucaryote 4G1 (eIF4GI)a, la protéine associée à la mort 5 (DAP5), l'oncogène tel que c-myc, L-myc, Pim-1, la protéine kinase p58PITSLRE, les hormones p53 choisies parmi les hormones gonadotropes choisies parmi la folliculostimuline, la gonadotrophine chorionique humaine, la lutéostimuline humaine et la protéine de liaison de la chaîne lourde des immunoglobulines (BiP), la protéine de choc thermique 70, la sous-unité β de la ATP synthase H+- mitochondriale, l'ornithine décarboxylase, les connexines 32 et 43, HIF-la, APC.

10. Vecteur d'expression selon l'une quelconque des revendications précédentes, dans lequel la cLysMAR est clonée au niveau de l'un des flancs de la cassette d'expression.

11. Vecteur d'expression selon les revendications 1 et 2, qui comprend en outre un intron chimérique et une séquence de polyadénylation de l'hormone de croissance bovine.

12. Vecteur d'expression selon l'une quelconque des revendications précédentes, ayant le numéro d'accès MTCC 5655, MTCC 5656 ou MTCC 5657.

13. Cellule hôte transformée avec un vecteur selon l'une quelconque des revendications précédentes.

14. Procédé de production de protéines et de peptides et de variants de ceux-ci comprenant :
a) la construction d'un vecteur d'expression selon l'une quelconque des revendications précédentes ; et
b) la transformation dudit vecteur d'expression dans la cellule hôte appropriée qui exprime la protéine ou le peptide d'intérêt.

15. Procédé de production de protéines et de peptides et de variants de ceux-ci comprenant :
a) la construction d'un vecteur d'expression selon l'une quelconque des revendications précédentes ;
b) la transfection dudit vecteur d'expression dans une cellule hôte appropriée ;
c) la sélection de la cellule hôte transfectée appropriée exprimant une protéine ou un peptide d'intérêt ;
d) le nouvelle transfection de la cellule hôte appropriée sélectionnée dans l'étape c) avec le vecteur d'expression selon l'une quelconque des revendications précédentes ;
e) l'expression d'une protéine ou d'un peptide d'intérêt par la cellule hôte nouvellement transfectée appropriée.

16. Procédé selon les revendications 14 et 15, dans lequel le vecteur d'expression a le numéro d'accès MTCC 5655, MTCC 5656 ou MTCC 5657.

17. Procédé selon les revendications 14 et 15, dans lequel les protéines et les peptides sont choisis parmi l'activateur tissulaire du plasminogène, TNK-TPA, la darbepoïétine, l'érythropoïétine, l'insuline, GCSF, l'interleukine, le facteur de nécrose tumorale, l'interféron, TNFR-IgGFc, les anticorps monoclonaux tels que le rituximab, le bévacizumab, l'adalimumab, le trastuzumab et leurs fragments tels que la région Fc, Fab, GLP-I, GLP-II, IGF-I, IGF-II, le facteur de croissance dérivé des plaquettes, FVII, FVIII, FIV et FXIII, l'exendine-3, l'exendine-4, les hormones telles que les hormones gonadotropes choisies parmi la folliculostimuline, la gonadotrophine chorionique humaine, la lutéostimuline humaine.


### SEQUENCE LISTING

<110> CADILA HEALTHCARE LIMITED
<120> EXPRESSION VECTOR FOR HIGH LEVEL EXPRESSION OF RECOMBINANT PROTEINS
<130> 2806-MUM-10
<150> IN: 2806/MUM/2010
   <151> 2010-10-08
<160> 24
<170> PatentIn version 3.5
<210> 1
   <211> 324
   <212> DNA
   <213> Artificial
<220>
   <223> Chimeric INTRON
<400> 1
   aattaattcg ctgtctgcga gggccagctg ttggggtgag tactccctct caaaagcggg      60
   catgacttct gcgctaagat tgtcagtttc caaaaacgag gaggatttga tattcacctg     120
   gcccgcggtg atgcctttga gggtggccgc gtccatctgg tcagaaaaga caatcttttt     180
   gttgtcaagc ttgaggtgtg gcaggcttga gatctggcca tacacttgag tgacaatgac     240
   atccactttg cctttctctc cacaggtgtc cactcccagg tccaactgca ggtcgagcat     300
   gcatctaggg cgcccgcact agag                                            324
<210> 2
   <211> 395
   <212> DNA
   <213> Artificial
<220>
   <223> TPL
<400> 2
   gtttagtgaa ccgtcagatc ctcttccgca tcgctgtctg cgagggccag ctgttggggt      60
   gagtactccc tctcaaaagc gggcatgact tctgcgctaa gaatgtcagt ttccaaaaac     120
   gaggaggatt tgatattcac tggcccgcgg tgatgccttt gagggtggcc gcgtccatct     180
   ggtcagaaaa gacaatcttt ttgttgtcaa gcttccttga tgatgtcata cttatcctgt     240
   cccttttttt tccacagctc gcggttgagg acaaactctt cgcggtcttt ccagtactct     300
   tggatcggaa acccgtcggc ctccgaacgg tactccgcca ccgagggacc tgagcgagtc     360
   cgcatcgacc ggatcggaaa acctctcgac gtacc                                395
<210> 3
   <211> 627
   <212> DNA
   <213> Artificial
<220>
   <223> VA I & II Genes
<400> 3
   ggtacctcgg gacgctctgg ccggtgaggc gtgcgcagtc gttgacgctc tagaccgtgc      60
   aaaaggagag cctgtaagcg ggcactcttc cgtggtctgg tggataaatt cgcaagggta     120
   tcatggcgga cgaccggggt tcgaaccccg gatccggccg tccgccgtga tccatgcggt     180
   taccgcccgc gtgtcgaacc caggtgtgcg acgtcagaca acgggggagc gctccttttg     240
   gcttccttcc aggcgcggcg gctgctgcgc tagctttttt ggccactggc cgcgcgcggc     300
   gtaagcggtt aggctggaaa gcgaaagcat taagtggctc gctccctgta gccggagggt     360
   tattttccaa gggttgagtc gcaggacccc cggttcgagt ctcgggccgg ccggactgcg     420
   gcgaacgggg gtttgcctcc ccgtcatgca agaccccgct tgcaaattcc tccggaaaca     480
   gggacgagcc ccttttttgc ttttcccaga tgcatccggt gctgcggcag atgcgccccc      540
   ctcctcagca gcggcaagag caagagcagc ggcagacatg cagggcaccc tccccttctc     600
   ctaccgcgtc aggaggggca acatccg                                         627
<210> 4
   <211> 4584
   <212> DNA
   <213> Artificial
<220>
   <223> pZRC II
<400> 4
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gattgaacag gatggcctgc atgcgggtag cccggcagcg tgggtggaac     180
   gtctgtttgg ctatgattgg gcgcagcaga ccattggctg ctctgatgcg gcggtgtttc     240
   gtctgagcgc gcagggtcgt ccggtgctgt ttgtgaaaac cgatctgagc ggtgcgctga     300
   acgagctgca ggatgaagcg gcgcgtctga gctggctggc caccaccggt gttccgtgtg     360
   cggcggtgct ggatgtggtg accgaagcgg gccgtgattg gctgctgctg ggcgaagtgc     420
   cgggtcagga tctgctgtct agccatctgg cgccggcaga aaaagtgagc attatggcgg     480
   atgccatgcg tcgtctgcat accctggacc cggcgacctg tccgtttgat catcaggcga     540
   aacatcgtat tgaacgtgcg cgtacccgta tggaagcggg cctggtggat caggatgatc     600
   tggatgaaga acatcagggc ctggcaccgg cagagctgtt tgcgcgtctg aaagcgagca     660
   tgccggatgg cgaagatctg gtggtgaccc atggtgatgc gtgcctgccg aacattatgg     720
   tggaaaatgg ccgttttagc ggctttattg attgcggccg tctgggcgtg gcggatcgtt     780
   atcaggatat tgcgctggcc acccgtgata ttgcggaaga actgggcggc gaatgggcgg     840
   atcgttttct ggtgctgtat ggcattgcgg caccggatag ccagcgtatt gcgttttatc     900
   gtctgctgga tgaatttttc taataactgt cagaccaagt ttactcatat atactttaga     960
   ttgatttaaa acttcatttt taatttaaaa ggatctaggt gaagatcctt tttgataatc    1020
   tcatgaccaa aatcccttaa cgtgagtttt cgttccactg agcgtcagac cccgtagaaa    1080
   agatcaaagg atcttcttga gatccttttt ttctgcgcgt aatctgctgc ttgcaaacaa    1140
   aaaaaccacc gctaccagcg gtggtttgtt tgccggatca agagctacca actctttttc 1200
   cgaaggtaac tggcttcagc agagcgcaga taccaaatac tgttcttcta gtgtagccgt 1260
   agttaggcca ccacttcaag aactctgtag caccgcctac atacctcgct ctgctaatcc 1320
   tgttaccagt ggctgctgcc agtggcgata agtcgtgtct taccgggttg gactcaagac 1380
   gatagttacc ggataaggcg cagcggtcgg gctgaacggg gggttcgtgc acacagccca 1440
   gcttggagcg aacgacctac accgaactga gatacctaca gcgtgagcta tgagaaagcg 1500
   ccacgcttcc cgaagggaga aaggcggaca ggtatccggt aagcggcagg gtcggaacag 1560
   gagagcgcac gagggagctt ccagggggaa acgcctggta tctttatagt cctgtcgggt 1620
   ttcgccacct ctgacttgag cgtcgatttt tgtgatgctc gtcagggggg cggagcctat 1680
   ggaaaaacgc cagcaacgcg gcctttttac ggttcctggc cttttgctgg ccttttgctc 1740
   attaggcacc ccaggcttta cccgaacgac cgagcgcagc gagtcagtga gcgaggaagc 1800
   ggagagcgcc caatacgcaa ggaaacagct atgaccatgt taatgcagct ggcacgacag 1860
   gtttcccgac tggaaagcgg gcagtgaaag gaaggcccat gaggccagtt aattaagagg 1920
   tacctcggga cgctctggcc ggtgaggcgt gcgcagtcgt tgacgctcta gaccgtgcaa 1980
   aaggagagcc tgtaagcggg cactcttccg tggtctggtg gataaattcg caagggtatc 2040
   atggcggacg accggggttc gaaccccgga tccggccgtc cgccgtgatc catgcggtta 2100
   ccgcccgcgt gtcgaaccca ggtgtgcgac gtcagacaac gggggagcgc tccttttggc 2160
   ttccttccag gcgcggcggc tgctgcgcta gcttttttgg ccactggccg cgcgcggcgt 2220
   aagcggttag gctggaaagc gaaagcatta agtggctcgc tccctgtagc cggagggtta 2280
   ttttccaagg gttgagtcgc aggacccccg gttcgagtct cgggccggcc ggactgcggc 2340
   gaacgggggt ttgcctcccc gtcatgcaag accccgcttg caaattcctc cggaaacagg 2400
   gacgagcccc ttttttgctt ttcccagatg catccggtgc tgcggcagat gcgcccccct 2460
   cctcagcagc ggcaagagca agagcagcgg cagacatgca gggcaccctc cccttctcct 2520
   accgcgtcag gaggggcaac atccgacgcg ttgacattga ttattgacta gttattaata 2580
   gtaatcaatt acggggtcat tagttcatag cccatatatg gagttccgcg ttacataact 2640
   tacggtaaat ggcccgcctg gctgaccgcc caacgacccc cgcccattga cgtcaataat 2700
   gacgtatgtt cccatagtaa cgccaatagg gactttccat tgacgtcaat gggtggagta 2760
   tttacggtaa actgcccact tggcagtaca tcaagtgtat catatgccaa gtacgccccc 2820
   tattgacgtc aatgacggta aatggcccgc ctggcattat gcccagtaca tgaccttatg 2880
   ggactttcct acttggcagt acatctacgt attagtcatc gctattacca tggtgatgcg 2940
   gttttggcag tacatcaatg ggcgtggata gcggtttgac tcacggggat ttccaagtct 3000
   ccaccccatt gacgtcaatg ggagtttgtt ttggcaccaa aatcaacggg actttccaaa 3060
   atgtcgtaac aactccgccc cattgacgca aatgggcggt aggcgtgtac ggtgggaggt 3120
   ctatataagc agagctggtt tagtgaaccg tcagatcctc ttccgcatcg ctgtctgcga 3180
   gggccagctg ttggggtgag tactccctct caaaagcggg catgacttct gcgctaagaa 3240
   tgtcagtttc caaaaacgag gaggatttga tattcactgg cccgcggtga tgcctttgag    3300
   ggtggccgcg tccatctggt cagaaaagac aatctttttg ttgtcaagct tccttgatga    3360
   tgtcatactt atcctgtccc ttttttttcc acagctcgcg gttgaggaca aactcttcgc    3420
   ggtctttcca gtactcttgg atcggaaacc cgtcggcctc cgaacggtac tccgccaccg    3480
   agggacctga gcgagtccgc atcgaccgga tcggaaaacc tctcgacgta ccaattaatt    3540
   cgctgtctgc gagggccagc tgttggggtg agtactccct ctcaaaagcg ggcatgactt    3600
   ctgcgctaag attgtcagtt tccaaaaacg aggaggattt gatattcacc tggcccgcgg    3660
   tgatgccttt gagggtggcc gcgtccatct ggtcagaaaa gacaatcttt ttgttgtcaa    3720
   gcttgaggtg tggcaggctt gagatctggc catacacttg agtgacaatg acatccactt    3780
   tgcctttctc tccacaggtg tccactccca ggtccaactg caggtcgagc atgcatctag    3840
   ggcgcccgca ctagaggctc gaggaattcc ccggggatat cgtttaaacc cacgatcgtg    3900
   gaagcggccg cccgctgatc agcctcgact gtgccttcta gttgccagcc atctgttgtt    3960
   tgcccctccc ccgtgccttc cttgaccctg gaaggtgcca ctcccactgt cctttcctaa    4020
   taaaatgagg aaattgcatc gcattgtctg agtaggtgtc attctattct ggggggtggg    4080
   gtggggcagg acagcaaggg ggaggattgg gaagacaata gcaggcatgc tggggatgcg    4140
   gtgggctcta tggcttctga ggcggaaaga accagctggg gctctagggg gtatccccac    4200
   ggagctcatg gcgcgcctag gccttgacgg ccttccttca attcgcccta tagtgagtcg    4260
   tattacgtcg cgctcactgg ccgtcgtttt acaacgtcgt gactgggaaa accctggcgt    4320
   tacccaactt aatcgccttg cagcacatcc ccctttcgcc agctggcgta atagcgaaga    4380
   ggcccgcacc gaaacgccct tcccaacagt tgcgcagcct gaatggcgaa tgggagcgcc    4440
   ctgtagcggc cactcaaccc tatctcggtc tattcttttg atttataagg gattttgccg    4500
   atttcggcct attggttaaa aaatgagctg atttaacaaa aatttaacgc gaattttaac    4560
   aaaatattaa cgcttacaat ttag                                            4584
<210> 5
   <211> 1668
   <212> DNA
   <213> Artificial
<220>
   <223> CLysMAR
<400> 5
   gcatccataa tataactgta ccaggttttg gtttattaca tgtgactgac ggcttcctat    60
   gcgtgctcag aaaacggcag ttgggcactg cactgcccgg tgatggtgcc acggtggctc    120
   ctgccgcctt ctttgatatt cactctgttg tatttcatct cttgttgccg atgaaaggat    180
   ataacagtct ctgaggaaat acttggtatt tcttctgatc agcgttttta taagtaatgt    240
   tgaatattgg ataaggctgt gtgtcctttg tcttgggaga caaagcccac agcaggtggt    300
   ggttgggtgg tggcagctca gtgacaggag aggttttttt gcctgttttt tttgttgttt    360
   ttttttttta agtaaggtgt tcttttttct tagtaaaatt tctactggac tgtatgtttt    420
   gacaggtcag aaacatttct tcaaaagaag aaccttttgg aaactgtaca gcccttttct    480
   ttcattccct ttttgctttc tgtgccaatg cctttggttc tgattgcatt atggaaaacg     540
   ttgatcggaa cttgaggttt ttatttatag tgtggcttga aagcttggat agctgttgtt     600
   acatgagata ccttattaag tttaggccag cttgatgctt tatttttttt cctttgaagt     660
   agtgagcgtt ctctggtttt tttcctttga aactggcgag gcttagattt ttctaatggg     720
   attttttacc tgatgatcta gttgcatacc caaatgcttg taaatgtttt cctagttaac     780
   atgttgataa cttcggattt acatgttgta tatacttgtc atctgtgttt ctagtaaaaa     840
   tatatggcat ttatagaaat acgtaattcc tgatttcctt ttttttttat ctctatgctc      900
   tgtgtgtaca ggtcaaacag acttcactcc tatttttatt tatagaattt tatatgcagt     960
   ctgtcgttgg ttcttgtgtt gtaaggatac agccttaaat ttcctagagc gatgctcagt    1020
   aaggcgggtt gtcacatggg ttcaaatgta aaacgggcac gtttgctgct gccttcccag    1080
   atccaggaca ctaaactgct tctgcaactg aggtataaat cgcttcagat cccaggaagt    1140
   gtagatccac gtgcatattc ttaaagaaga atgaatactt tctaaaatat gttggcatag    1200
   gaagcaagct gcatggattt atttgggact taaattattt tggtaacgga gtgcataggt    1260
   tttaaacaca gttgcagcat gctaacgagt cacagcattt atgcagaagt gatgcctgtt    1320
   gcagctgttt acggcactgc cttgcagtga gcattgcaga taggggtggg gtgctttgtg    1380
   tcgtgttggg acacgctgcc acacagccac ctcccgaaca tatctcacct gctgggtact    1440
   tttcaaacca tcttagcagt agtagatgag ttactatgaa acagagaagt tcctcagttg    1500
   gatattctca tgggatgtct tttttcccat gttgggcaaa gtatgataaa gcatctctat    1560
   ttgtaaatta tgcacttgtt agttcctgaa tcctttctat agcaccactt attgcagcag    1620
   gtgtaggctc tggtgtggcc tgtgtctgtg cttcaatctt ttaagctt                 1668
<210> 6
   <211> 7932
   <212> DNA
   <213> Artificial
<220>
   <223> pZRC III
<400> 6
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gattgaacag gatggcctgc atgcgggtag cccggcagcg tgggtggaac     180
   gtctgtttgg ctatgattgg gcgcagcaga ccattggctg ctctgatgcg gcggtgtttc     240
   gtctgagcgc gcagggtcgt ccggtgctgt ttgtgaaaac cgatctgagc ggtgcgctga     300
   acgagctgca ggatgaagcg gcgcgtctga gctggctggc caccaccggt gttccgtgtg     360
   cggcggtgct ggatgtggtg accgaagcgg gccgtgattg gctgctgctg ggcgaagtgc     420
   cgggtcagga tctgctgtct agccatctgg cgccggcaga aaaagtgagc attatggcgg     480
   atgccatgcg tcgtctgcat accctggacc cggcgacctg tccgtttgat catcaggcga     540
   aacatcgtat tgaacgtgcg cgtacccgta tggaagcggg cctggtggat caggatgatc 600
   tggatgaaga acatcagggc ctggcaccgg cagagctgtt tgcgcgtctg aaagcgagca 660
   tgccggatgg cgaagatctg gtggtgaccc atggtgatgc gtgcctgccg aacattatgg 720
   tggaaaatgg ccgttttagc ggctttattg attgcggccg tctgggcgtg gcggatcgtt 780
   atcaggatat tgcgctggcc acccgtgata ttgcggaaga actgggcggc gaatgggcgg 840
   atcgttttct ggtgctgtat ggcattgcgg caccggatag ccagcgtatt gcgttttatc 900
   gtctgctgga tgaatttttc taataactgt cagaccaagt ttactcatat atactttaga 960
   ttgatttaaa acttcatttt taatttaaaa ggatctaggt gaagatcctt tttgataatc 1020
   tcatgaccaa aatcccttaa cgtgagtttt cgttccactg agcgtcagac cccgtagaaa 1080
   agatcaaagg atcttcttga gatccttttt ttctgcgcgt aatctgctgc ttgcaaacaa 1140
   aaaaaccacc gctaccagcg gtggtttgtt tgccggatca agagctacca actctttttc 1200
   cgaaggtaac tggcttcagc agagcgcaga taccaaatac tgttcttcta gtgtagccgt 1260
   agttaggcca ccacttcaag aactctgtag caccgcctac atacctcgct ctgctaatcc 1320
   tgttaccagt ggctgctgcc agtggcgata agtcgtgtct taccgggttg gactcaagac 1380
   gatagttacc ggataaggcg cagcggtcgg gctgaacggg gggttcgtgc acacagccca 1440
   gcttggagcg aacgacctac accgaactga gatacctaca gcgtgagcta tgagaaagcg 1500
   ccacgcttcc cgaagggaga aaggcggaca ggtatccggt aagcggcagg gtcggaacag 1560
   gagagcgcac gagggagctt ccagggggaa acgcctggta tctttatagt cctgtcgggt 1620
   ttcgccacct ctgacttgag cgtcgatttt tgtgatgctc gtcagggggg cggagcctat 1680
   ggaaaaacgc cagcaacgcg gcctttttac ggttcctggc cttttgctgg ccttttgctc 1740
   attaggcacc ccaggcttta cccgaacgac cgagcgcagc gagtcagtga gcgaggaagc 1800
   ggagagcgcc caatacgcaa ggaaacagct atgaccatgt taatgcagct ggcacgacag 1860
   gtttcccgac tggaaagcgg gcagtgaaag gaaggcccat gaggccagtt aattaagagg 1920
   tacctcggga cgctctggcc ggtgaggcgt gcgcagtcgt tgacgctcta gaccgtgcaa 1980
   aaggagagcc tgtaagcggg cactcttccg tggtctggtg gataaattcg caagggtatc 2040
   atggcggacg accggggttc gaaccccgga tccggccgtc cgccgtgatc catgcggtta 2100
   ccgcccgcgt gtcgaaccca ggtgtgcgac gtcagacaac gggggagcgc tccttttggc 2160
   ttccttccag gcgcggcggc tgctgcgcta gcttttttgg ccactggccg cgcgcggcgt 2220
   aagcggttag gctggaaagc gaaagcatta agtggctcgc tccctgtagc cggagggtta 2280
   ttttccaagg gttgagtcgc aggacccccg gttcgagtct cgggccggcc ggactgcggc 2340
   gaacgggggt ttgcctcccc gtcatgcaag accccgcttg caaattcctc cggaaacagg 2400
   gacgagcccc ttttttgctt ttcccagatg catccggtgc tgcggcagat gcgcccccct 2460
   cctcagcagc ggcaagagca agagcagcgg cagacatgca gggcaccctc cccttctcct 2520
   accgcgtcag gaggggcaac atccgacgcg tgcatccata atataactgt accaggtttt 2580
   ggtttattac atgtgactga cggcttccta tgcgtgctca gaaaacggca gttgggcact 2640
   gcactgcccg gtgatggtgc cacggtggct cctgccgcct tctttgatat tcactctgtt 2700
   gtatttcatc tcttgttgcc gatgaaagga tataacagtc tctgaggaaa tacttggtat 2760
   ttcttctgat cagcgttttt ataagtaatg ttgaatattg gataaggctg tgtgtccttt 2820
   gtcttgggag acaaagccca cagcaggtgg tggttgggtg gtggcagctc agtgacagga 2880
   gaggtttttt tgcctgtttt ttttgttgtt tttttttttt aagtaaggtg ttcttttttc 2940
   ttagtaaaat ttctactgga ctgtatgttt tgacaggtca gaaacatttc ttcaaaagaa 3000
   gaaccttttg gaaactgtac agcccttttc tttcattccc tttttgcttt ctgtgccaat 3060
   gcctttggtt ctgattgcat tatggaaaac gttgatcgga acttgaggtt tttatttata 3120
   gtgtggcttg aaagcttgga tagctgttgt tacatgagat accttattaa gtttaggcca 3180
   gcttgatgct ttattttttt tcctttgaag tagtgagcgt tctctggttt ttttcctttg 3240
   aaactggcga ggcttagatt tttctaatgg gattttttac ctgatgatct agttgcatac 3300
   ccaaatgctt gtaaatgttt tcctagttaa catgttgata acttcggatt tacatgttgt 3360
   atatacttgt catctgtgtt tctagtaaaa atatatggca tttatagaaa tacgtaattc 3420
   ctgatttcct ttttttttta tctctatgct ctgtgtgtac aggtcaaaca gacttcactc 3480
   ctatttttat ttatagaatt ttatatgcag tctgtcgttg gttcttgtgt tgtaaggata 3540
   cagccttaaa tttcctagag cgatgctcag taaggcgggt tgtcacatgg gttcaaatgt 3600
   aaaacgggca cgtttgctgc tgccttccca gatccaggac actaaactgc ttctgcaact 3660
   gaggtataaa tcgcttcaga tcccaggaag tgtagatcca cgtgcatatt cttaaagaag 3720
   aatgaatact ttctaaaata tgttggcata ggaagcaagc tgcatggatt tatttgggac 3780
   ttaaattatt ttggtaacgg agtgcatagg ttttaaacac agttgcagca tgctaacgag 3840
   tcacagcatt tatgcagaag tgatgcctgt tgcagctgtt tacggcactg ccttgcagtg 3900
   agcattgcag ataggggtgg ggtgctttgt gtcgtgttgg gacacgctgc cacacagcca 3960
   cctcccgaac atatctcacc tgctgggtac ttttcaaacc atcttagcag tagtagatga 4020
   gttactatga aacagagaag ttcctcagtt ggatattctc atgggatgtc tttttcccca 4080
   tgttgggcaa agtatgataa agcatctcta tttgtaaatt atgcacttgt tagttcctga 4140
   atcctttcta tagcaccact tattgcagca ggtgtaggct ctggtgtggc ctgtgtctgt 4200
   gcttcaatct tttaagctta cgcgttgaca ttgattattg actagttatt aatagtaatc 4260
   aattacgggg tcattagttc atagcccata tatggagttc cgcgttacat aacttacggt 4320
   aaatggcccg cctggctgac cgcccaacga cccccgccca ttgacgtcaa taatgacgta 4380
   tgttcccata gtaacgccaa tagggacttt ccattgacgt caatgggtgg agtatttacg 4440
   gtaaactgcc cacttggcag tacatcaagt gtatcatatg ccaagtacgc cccctattga 4500
   cgtcaatgac ggtaaatggc ccgcctggca ttatgcccag tacatgacct tatgggactt 4560
   tcctacttgg cagtacatct acgtattagt catcgctatt accatggtga tgcggttttg 4620
   gcagtacatc aatgggcgtg gatagcggtt tgactcacgg ggatttccaa gtctccaccc 4680
   cattgacgtc aatgggagtt tgttttggca ccaaaatcaa cgggactttc caaaatgtcg 4740
   taacaactcc gccccattga cgcaaatggg cggtaggcgt gtacggtggg aggtctatat 4800
   aagcagagct ggtttagtga accgtcagat cctcttccgc atcgctgtct gcgagggcca 4860
   gctgttgggg tgagtactcc ctctcaaaag cgggcatgac ttctgcgcta agaatgtcag 4920
   tttccaaaaa cgaggaggat ttgatattca ctggcccgcg gtgatgcctt tgagggtggc 4980
   cgcgtccatc tggtcagaaa agacaatctt tttgttgtca agcttccttg atgatgtcat 5040
   acttatcctg tccctttttt ttccacagct cgcggttgag gacaaactct tcgcggtctt 5100
   tccagtactc ttggatcgga aacccgtcgg cctccgaacg gtactccgcc accgagggac 5160
   ctgagcgagt ccgcatcgac cggatcggaa aacctctcga cgtaccaatt aattcgctgt 5220
   ctgcgagggc cagctgttgg ggtgagtact ccctctcaaa agcgggcatg acttctgcgc 5280
   taagattgtc agtttccaaa aacgaggagg atttgatatt cacctggccc gcggtgatgc 5340
   ctttgagggt ggccgcgtcc atctggtcag aaaagacaat ctttttgttg tcaagcttga 5400
   ggtgtggcag gcttgagatc tggccataca cttgagtgac aatgacatcc actttgcctt 5460
   tctctccaca ggtgtccact cccaggtcca actgcaggtc gagcatgcat ctagggcgcc 5520
   cgcactagag gctcgaggaa ttccccgggg atatcgttta aacccacgat cgtggaagcg 5580
   gccgcccgct gatcagcctc gactgtgcct tctagttgcc agccatctgt tgtttgcccc 5640
   tcccccgtgc cttccttgac cctggaaggt gccactccca ctgtcctttc ctaataaaat 5700
   gaggaaattg catcgcattg tctgagtagg tgtcattcta ttctgggggg tggggtgggg 5760
   caggacagca agggggagga ttgggaagac aatagcaggc atgctgggga tgcggtgggc 5820
   tctatggctt ctgaggcgga aagaaccagc tggggctcta gggggtatcc ccacggagct 5880
   cgcatccata atataactgt accaggtttt ggtttattac atgtgactga cggcttccta 5940
   tgcgtgctca gaaaacggca gttgggcact gcactgcccg gtgatggtgc cacggtggct 6000
   cctgccgcct tctttgatat tcactctgtt gtatttcatc tcttgttgcc gatgaaagga 6060
   tataacagtc tctgaggaaa tacttggtat ttcttctgat cagcgttttt ataagtaatg 6120
   ttgaatattg gataaggctg tgtgtccttt gtcttgggag acaaagccca cagcaggtgg 6180
   tggttgggtg gtggcagctc agtgacagga gaggtttttt tgcctgtttt ttttgttgtt 6240
   tttttttttt aagtaaggtg ttcttttttc ttagtaaaat ttctactgga ctgtatgttt 6300
   tgacaggtca gaaacatttc ttcaaaagaa gaaccttttg gaaactgtac agcccttttc 6360
   tttcattccc tttttgcttt ctgtgccaat gcctttggtt ctgattgcat tatggaaaac 6420
   gttgatcgga acttgaggtt tttatttata gtgtggcttg aaagcttgga tagctgttgt 6480
   tacatgagat accttattaa gtttaggcca gcttgatgct ttattttttt tcctttgaag 6540
   tagtgagcgt tctctggttt ttttcctttg aaactggcga ggcttagatt tttctaatgg 6600
   gattttttac ctgatgatct agttgcatac ccaaatgctt gtaaatgttt tcctagttaa 6660
   catgttgata acttcggatt tacatgttgt atatacttgt catctgtgtt tctagtaaaa 6720
   atatatggca tttatagaaa tacgtaattc ctgatttcct ttttttttta tctctatgct 6780
   ctgtgtgtac aggtcaaaca gacttcactc ctatttttat ttatagaatt ttatatgcag 6840
   tctgtcgttg gttcttgtgt tgtaaggata cagccttaaa tttcctagag cgatgctcag    6900
   taaggcgggt tgtcacatgg gttcaaatgt aaaacgggca cgtttgctgc tgccttccca    6960
   gatccaggac actaaactgc ttctgcaact gaggtataaa tcgcttcaga tcccaggaag    7020
   tgtagatcca cgtgcatatt cttaaagaag aatgaatact ttctaaaata tgttggcata    7080
   ggaagcaagc tgcatggatt tatttgggac ttaaattatt ttggtaacgg agtgcatagg    7140
   ttttaaacac agttgcagca tgctaacgag tcacagcatt tatgcagaag tgatgcctgt    7200
   tgcagctgtt tacggcactg ccttgcagtg agcattgcag ataggggtgg ggtgctttgt    7260
   gtcgtgttgg gacacgctgc cacacagcca cctcccgaac atatctcacc tgctgggtac    7320
   ttttcaaacc atcttagcag tagtagatga gttactatga aacagagaag ttcctcagtt    7380
   ggatattctc atgggatgtc tttttcccca tgttgggcaa agtatgataa agcatctcta    7440
   tttgtaaatt atgcacttgt tagttcctga atcctttcta tagcaccact tattgcagca    7500
   ggtgtaggct ctggtgtggc ctgtgtctgt gcttcaatct tttaagcttg agctcatggc    7560
   gcgcctaggc cttgacggcc ttccttcaat tcgccctata gtgagtcgta ttacgtcgcg    7620
   ctcactggcc gtcgttttac aacgtcgtga ctgggaaaac cctggcgtta cccaacttaa    7680
   tcgccttgca gcacatcccc ctttcgccag ctggcgtaat agcgaagagg cccgcaccga    7740
   aacgcccttc ccaacagttg cgcagcctga atggcgaatg ggagcgccct gtagcggcca    7800
   ctcaacccta tctcggtcta ttcttttgat ttataaggga ttttgccgat ttcggcctat    7860
   tggttaaaaa atgagctgat ttaacaaaaa tttaacgcga attttaacaa aatattaacg    7920
   cttacaattt ag                                                        7932
<210> 7
   <211> 562
   <212> PRT
   <213> Artificial
<220>
   <223> TNK
<400> 7
   Met Asp Ala Met Lys Arg Gly Leu Cys Cys Val Leu Leu Leu Cys Gly
   1               5                   10                  15
   Ala Val Phe Val Ser Pro Ser Gln Glu Ile His Ala Arg Phe Arg Arg
               20                  25                  30
   Gly Ala Arg Ser Tyr Gln Val Ile Cys Arg Asp Glu Lys Thr Gln Met
           35                  40                  45
   Ile Tyr Gln Gln His Gln Ser Trp Leu Arg Pro Val Leu Arg Ser Asn
       50                  55                  60
   Arg Val Glu Tyr Cys Trp Cys Asn Ser Gly Arg Ala Gln Cys His Ser
   65                  70                  75                  80
   Val Pro Val Lys Ser Cys Ser Glu Pro Arg Cys Phe Asn Gly Gly Thr
   85 90 95
   Cys Gln Gln Ala Leu Tyr Phe Ser Asp Phe Val Cys Gln Cys Pro Glu
   100 105 110
   Gly Phe Ala Gly Lys Cys Cys Glu Ile Asp Thr Arg Ala Thr Cys Tyr
   115 120 125
   Glu Asp Gln Gly Ile Ser Tyr Arg Gly Asn Trp Ser Thr Ala Glu Ser
   130 135 140
   Gly Ala Glu Cys Thr Asn Trp Gln Ser Ser Ala Leu Ala Gln Lys Pro
   145 150 155 160
   Tyr Ser Gly Arg Arg Pro Asp Ala Ile Arg Leu Gly Leu Gly Asn His
   165 170 175
   Asn Tyr Cys Arg Asn Pro Asp Arg Asp Ser Lys Pro Trp Cys Tyr Val
   180 185 190
   Phe Lys Ala Gly Lys Tyr Ser Ser Glu Phe Cys Ser Thr Pro Ala Cys
   195 200 205
   Ser Glu Gly Asn Ser Asp Cys Tyr Phe Gly Asn Gly Ser Ala Tyr Arg
   210 215 220
   Gly Thr His Ser Leu Thr Glu Ser Gly Ala Ser Cys Leu Pro Trp Asn
   225 230 235 240
   Ser Met Ile Leu Ile Gly Lys Val Tyr Thr Ala Gln Asn Pro Ser Ala
   245 250 255
   Gln Ala Leu Gly Leu Gly Lys His Asn Tyr Cys Arg Asn Pro Asp Gly
   260 265 270
   Asp Ala Lys Pro Trp Cys His Val Leu Lys Asn Arg Arg Leu Thr Trp
   275 280 285
   Glu Tyr Cys Asp Val Pro Ser Cys Ser Thr Cys Gly Leu Arg Gln Tyr
   290 295 300
   Ser Gln Pro Gln Phe Arg Ile Lys Gly Gly Leu Phe Ala Asp Ile Ala
   305 310 315 320
   Ser His Pro Trp Gln Ala Ala Ile Phe Ala Ala Ala Ala Ala Ser Pro
   325 330 335
   Gly Glu Arg Phe Leu Cys Gly Gly Ile Leu Ile Ser Ser Cys Trp Ile
   340 345 350
   Leu Ser Ala Ala His Cys Phe Gln Glu Arg Phe Pro Pro His His Leu
   355 360 365
   Thr Val Ile Leu Gly Arg Thr Tyr Arg Val Val Pro Gly Glu Glu Glu
       370                     375                     380
   Gln Lys Phe Glu Val Glu Lys Tyr Ile Val His Lys Glu Phe Asp Asp
   385                 390                     395                 400
   Asp Thr Tyr Asp Asn Asp Ile Ala Leu Leu Gln Leu Lys Ser Asp Ser
                   405                     410                     415
   Ser Arg Cys Ala Gln Glu Ser Ser Val Val Arg Thr Val Cys Leu Pro
               420                     425                     430
   Pro Ala Asp Leu Gln Leu Pro Asp Trp Thr Glu Cys Glu Leu Ser Gly
           435                     440                     445
   Tyr Gly Lys His Glu Ala Leu Ser Pro Phe Tyr Ser Glu Arg Leu Lys
       450                     455                     460
   Glu Ala His Val Arg Leu Tyr Pro Ser Ser Arg Cys Thr Ser Gln His
   465                 470                     475                 480
   Leu Leu Asn Arg Thr Val Thr Asp Asn Met Leu Cys Ala Gly Asp Thr
                   485                     490                     495
   Arg Ser Gly Gly Pro Gln Ala Asn Leu His Asp Ala Cys Gln Gly Asp
               500                     505                     510
   Ser Gly Gly Pro Leu Val Cys Leu Asn Asp Gly Arg Met Thr Leu Val
           515                     520                     525
   Gly Ile Ile Ser Trp Gly Leu Gly Cys Gly Gln Lys Asp Val Pro Gly
       530                     535                     540
   Val Tyr Thr Lys Val Thr Asn Tyr Leu Asp Trp Ile Arg Asp Asn Met
   545                 550                     555                 560
   Arg Pro
<210> 8
   <211> 11138
   <212> DNA
   <213> Artificial
<220>
   <223> pZRC III- TNK- Hyg
<400> 8
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gattgaacag gatggcctgc atgcgggtag cccggcagcg tgggtggaac     180
   gtctgtttgg ctatgattgg gcgcagcaga ccattggctg ctctgatgcg gcggtgtttc     240
   gtctgagcgc gcagggtcgt ccggtgctgt ttgtgaaaac cgatctgagc ggtgcgctga 300
   acgagctgca ggatgaagcg gcgcgtctga gctggctggc caccaccggt gttccgtgtg 360
   cggcggtgct ggatgtggtg accgaagcgg gccgtgattg gctgctgctg ggcgaagtgc 420
   cgggtcagga tctgctgtct agccatctgg cgccggcaga aaaagtgagc attatggcgg 480
   atgccatgcg tcgtctgcat accctggacc cggcgacctg tccgtttgat catcaggcga 540
   aacatcgtat tgaacgtgcg cgtacccgta tggaagcggg cctggtggat caggatgatc 600
   tggatgaaga acatcagggc ctggcaccgg cagagctgtt tgcgcgtctg aaagcgagca 660
   tgccggatgg cgaagatctg gtggtgaccc atggtgatgc gtgcctgccg aacattatgg 720
   tggaaaatgg ccgttttagc ggctttattg attgcggccg tctgggcgtg gcggatcgtt 780
   atcaggatat tgcgctggcc acccgtgata ttgcggaaga actgggcggc gaatgggcgg 840
   atcgttttct ggtgctgtat ggcattgcgg caccggatag ccagcgtatt gcgttttatc 900
   gtctgctgga tgaatttttc taataactgt cagaccaagt ttactcatat atactttaga 960
   ttgatttaaa acttcatttt taatttaaaa ggatctaggt gaagatcctt tttgataatc 1020
   tcatgaccaa aatcccttaa cgtgagtttt cgttccactg agcgtcagac cccgtagaaa 1080
   agatcaaagg atcttcttga gatccttttt ttctgcgcgt aatctgctgc ttgcaaacaa 1140
   aaaaaccacc gctaccagcg gtggtttgtt tgccggatca agagctacca actctttttc 1200
   cgaaggtaac tggcttcagc agagcgcaga taccaaatac tgttcttcta gtgtagccgt 1260
   agttaggcca ccacttcaag aactctgtag caccgcctac atacctcgct ctgctaatcc 1320
   tgttaccagt ggctgctgcc agtggcgata agtcgtgtct taccgggttg gactcaagac 1380
   gatagttacc ggataaggcg cagcggtcgg gctgaacggg gggttcgtgc acacagccca 1440
   gcttggagcg aacgacctac accgaactga gatacctaca gcgtgagcta tgagaaagcg 1500
   ccacgcttcc cgaagggaga aaggcggaca ggtatccggt aagcggcagg gtcggaacag 1560
   gagagcgcac gagggagctt ccagggggaa acgcctggta tctttatagt cctgtcgggt 1620
   ttcgccacct ctgacttgag cgtcgatttt tgtgatgctc gtcagggggg cggagcctat 1680
   ggaaaaacgc cagcaacgcg gcctttttac ggttcctggc cttttgctgg ccttttgctc 1740
   attaggcacc ccaggcttta cccgaacgac cgagcgcagc gagtcagtga gcgaggaagc 1800
   ggagagcgcc caatacgcaa ggaaacagct atgaccatgt taatgcagct ggcacgacag 1860
   gtttcccgac tggaaagcgg gcagtgaaag gaaggcccat gaggccagtt aattaagagg 1920
   atccttatcg attttaccac atttgtagag gttttacttg ctttaaaaaa cctcccacac 1980
   ctccccctga acctgaaaca taaaatgaat gcaattgttg ttgttaactt gtttattgca 2040
   gcttataatg gttacaaata aagcaatagc atcacaaatt tcacaaataa agcatttttt 2100
   tcactgcatt ctagttgtgg tttgtccaaa ctcatcaatg tatcttatca tgtctgctcg 2160
   aagcggccgg ccgccccgac tctagactat tcctttgccc tcggacgagt gctggggcgt 2220
   cggtttccac tatcggcgag tacttctaca cagccatcgg tccagacggc cgcgcttctg 2280
   cgggcgattt gtgtacgccc gacagtcccg gctccggatc ggacgattgc gtcgcatcga 2340
   ccctgcgccc aagctgcatc atcgaaattg ccgtcaacca agctctgata gagttggtca 2400
   agaccaatgc ggagcatata cgcccggagc cgcggcgatc ctgcaagctc cggatgcctc 2460
   cgctcgaagt agcgcgtctg ctgctccata caagccaacc acggcctcca gaagaagatg 2520
   ttggcgacct cgtattggga atccccgaac atcgcctcgc tccagtcaat gaccgctgtt 2580
   atgcggccat tgtccgtcag gacattgttg gagccgaaat ccgcgtgcac gaggtgccgg 2640
   acttcggggc agtcctcggc ccaaagcatc agctcatcga gagcctgcgc gacggacgca 2700
   ctgacggtgt cgtccatcac agtttgccag tgatacacat ggggatcagc aatcgcgcat 2760
   atgaaatcac gccatgtagt gtattgaccg attccttgcg gtccgaatgg gccgaacccg 2820
   ctcgtctggc taagatcggc cgcagcgatc gcatccatgg cctccgcgac cggctgcaga 2880
   acagcgggca gttcggtttc aggcaggtct tgcaacgtga caccctgtgc acggcgggag 2940
   atgcaatagg tcaggctctc gctgaattcc ccaatgtcaa gcacttccgg aatcgggagc 3000
   gcggccgatg caaagtgccg ataaacataa cgatctttgt agaaaccatc ggcgcagcta 3060
   tttacccgca ggacatatcc acgccctcct acatcgaagc tgaaagcacg agattcttcg 3120
   ccctccgaga gctgcatcag gtcggagacg ctgtcgaact tttcgatcag aaacttctcg 3180
   acagacgtcg cggtgagttc aggctttccg gatctatcca tcatggtggc tttaccaaca 3240
   gtaccggaat gccaagcttt ttgcaaaagc ctaggcctcc aaaaaagcct cctcactact 3300
   tctggaatag ctcagaggcc gaggcggcct cggcctctgc ataaataaaa aaaattagtc 3360
   agcgatgggg cggagaatgg gcggaactgg gcggagttag gggcgggatg ggcggagtta 3420
   ggggcgggac tatggttgct gactaattga gatgcagatc gcagatcctc gggacgctct 3480
   ggccggtgag gcgtgcgcag tcgttgacgc tctagaccgt gcaaaaggag agcctgtaag 3540
   cgggcactct tccgtggtct ggtggataaa ttcgcaaggg tatcatggcg gacgaccggg 3600
   gttcgaaccc cggatccggc cgtccgccgt gatccatgcg gttaccgccc gcgtgtcgaa 3660
   cccaggtgtg cgacgtcaga caacggggga gcgctccttt tggcttcctt ccaggcgcgg 3720
   cggctgctgc gctagctttt ttggccactg gccgcgcgcg gcgtaagcgg ttaggctgga 3780
   aagcgaaagc attaagtggc tcgctccctg tagccggagg gttattttcc aagggttgag 3840
   tcgcaggacc cccggttcga gtctcgggcc ggccggactg cggcgaacgg gggtttgcct 3900
   ccccgtcatg caagaccccg cttgcaaatt cctccggaaa cagggacgag cccctttttt 3960
   gcttttccca gatgcatccg gtgctgcggc agatgcgccc ccctcctcag cagcggcaag 4020
   agcaagagca gcggcagaca tgcagggcac cctccccttc tcctaccgcg tcaggagggg 4080
   caacatccga cgcgtgcatc cataatataa ctgtaccagg ttttggttta ttacatgtga 4140
   ctgacggctt cctatgcgtg ctcagaaaac ggcagttggg cactgcactg cccggtgatg 4200
   gtgccacggt ggctcctgcc gccttctttg atattcactc tgttgtattt catctcttgt 4260
   tgccgatgaa aggatataac agtctctgag gaaatacttg gtatttcttc tgatcagcgt 4320
   ttttataagt aatgttgaat attggataag gctgtgtgtc ctttgtcttg ggagacaaag 4380
   cccacagcag gtggtggttg ggtggtggca gctcagtgac aggagaggtt tttttgcctg 4440
   ttttttttgt tgtttttttt ttttaagtaa ggtgttcttt tttcttagta aaatttctac 4500
   tggactgtat gttttgacag gtcagaaaca tttcttcaaa agaagaacct tttggaaact 4560
   gtacagccct tttctttcat tccctttttg ctttctgtgc caatgccttt ggttctgatt 4620
   gcattatgga aaacgttgat cggaacttga ggtttttatt tatagtgtgg cttgaaagct 4680
   tggatagctg ttgttacatg agatacctta ttaagtttag gccagcttga tgctttattt 4740
   tttttccttt gaagtagtga gcgttctctg gtttttttcc tttgaaactg gcgaggctta 4800
   gatttttcta atgggatttt ttacctgatg atctagttgc atacccaaat gcttgtaaat 4860
   gttttcctag ttaacatgtt gataacttcg gatttacatg ttgtatatac ttgtcatctg 4920
   tgtttctagt aaaaatatat ggcatttata gaaatacgta attcctgatt tccttttttt 4980
   tttatctcta tgctctgtgt gtacaggtca aacagacttc actcctattt ttatttatag 5040
   aattttatat gcagtctgtc gttggttctt gtgttgtaag gatacagcct taaatttcct 5100
   agagcgatgc tcagtaaggc gggttgtcac atgggttcaa atgtaaaacg ggcacgtttg 5160
   ctgctgcctt cccagatcca ggacactaaa ctgcttctgc aactgaggta taaatcgctt 5220
   cagatcccag gaagtgtaga tccacgtgca tattcttaaa gaagaatgaa tactttctaa 5280
   aatatgttgg cataggaagc aagctgcatg gatttatttg ggacttaaat tattttggta 5340
   acggagtgca taggttttaa acacagttgc agcatgctaa cgagtcacag catttatgca 5400
   gaagtgatgc ctgttgcagc tgtttacggc actgccttgc agtgagcatt gcagataggg 5460
   gtggggtgct ttgtgtcgtg ttgggacacg ctgccacaca gccacctccc gaacatatct 5520
   cacctgctgg gtacttttca aaccatctta gcagtagtag atgagttact atgaaacaga 5580
   gaagttcctc agttggatat tctcatggga tgtctttttt cccatgttgg gcaaagtatg 5640
   ataaagcatc tctatttgta aattatgcac ttgttagttc ctgaatcctt tctatagcac 5700
   cacttattgc agcaggtgta ggctctggtg tggcctgtgt ctgtgcttca atcttttaag 5760
   cttacgcgtt gacattgatt attgactagt tattaatagt aatcaattac ggggtcatta 5820
   gttcatagcc catatatgga gttccgcgtt acataactta cggtaaatgg cccgcctggc 5880
   tgaccgccca acgacccccg cccattgacg tcaataatga cgtatgttcc catagtaacg 5940
   ccaataggga ctttccattg acgtcaatgg gtggagtatt tacggtaaac tgcccacttg 6000
   gcagtacatc aagtgtatca tatgccaagt acgcccccta ttgacgtcaa tgacggtaaa 6060
   tggcccgcct ggcattatgc ccagtacatg accttatggg actttcctac ttggcagtac 6120
   atctacgtat tagtcatcgc tattaccatg gtgatgcggt tttggcagta catcaatggg 6180
   cgtggatagc ggtttgactc acggggattt ccaagtctcc accccattga cgtcaatggg 6240
   agtttgtttt ggcaccaaaa tcaacgggac tttccaaaat gtcgtaacaa ctccgcccca 6300
   ttgacgcaaa tgggcggtag gcgtgtacgg tgggaggtct atataagcag agctggttta 6360
   gtgaaccgtc agatcctctt ccgcatcgct gtctgcgagg gccagctgtt ggggtgagta 6420
   ctccctctca aaagcgggca tgacttctgc gctaagaatg tcagtttcca aaaacgagga 6480
   ggatttgata ttcactggcc cgcggtgatg cctttgaggg tggccgcgtc catctggtca 6540
   gaaaagacaa tctttttgtt gtcaagcttc cttgatgatg tcatacttat cctgtccctt 6600
   ttttttccac agctcgcggt tgaggacaaa ctcttcgcgg tctttccagt actcttggat 6660
   cggaaacccg tcggcctccg aacggtactc cgccaccgag ggacctgagc gagtccgcat 6720
   cgaccggatc ggaaaacctc tcgacgtacc aattaattcg ctgtctgcga gggccagctg 6780
   ttggggtgag tactccctct caaaagcggg catgacttct gcgctaagat tgtcagtttc 6840
   caaaaacgag gaggatttga tattcacctg gcccgcggtg atgcctttga gggtggccgc 6900
   gtccatctgg tcagaaaaga caatcttttt gttgtcaagc ttgaggtgtg gcaggcttga 6960
   gatctggcca tacacttgag tgacaatgac atccactttg cctttctctc cacaggtgtc 7020
   cactcccagg tccaactgca ggtcgagcat gcatctaggg cgcccgcact agaggctcga 7080
   ggaattccac catggacgcc atgaagcggg gactgtgctg tgtgctgctg ctgtgtggcg 7140
   ccgtgttcgt gtcccctagc caggaaatcc acgcccggtt cagaaggggc gccaggtcct 7200
   accaagttat ctgccgggac gaaaagaccc agatgatcta ccagcagcac cagtcctggc 7260
   tgcggcccgt gctgcggtcc aaccgggtgg agtactgctg gtgcaactcc ggcagagccc 7320
   agtgccactc cgtgcctgtg aagtcctgct ccgagcctag atgcttcaac ggcggcacct 7380
   gtcagcaggc cctgtacttc tccgacttcg tgtgccagtg ccctgagggc ttcgccggca 7440
   agtgctgcga gatcgacacc cgggccacct gttacgagga ccagggcatc tcctaccggg 7500
   gcaactggtc taccgccgag tctggcgccg agtgcaccaa ctggcagtcc tccgccctgg 7560
   cccagaagcc ttactctggc agacggcctg acgccatcag actgggcctg ggcaaccaca 7620
   actactgccg gaaccctgac cgggactcca agccttggtg ctacgtgttc aaggccggca 7680
   agtactcctc cgagttctgc tccacccctg cctgctctga gggcaactcc gactgctact 7740
   tcggcaacgg ctccgcctac agaggcaccc actccctgac cgagtccggc gcctcttgcc 7800
   tgccttggaa ctccatgatc ctgatcggca aggtgtacac cgcccagaac ccttccgctc 7860
   aggccctggg actcggaaag cacaattatt gtcgcaatcc cgacggcgac gccaaacctt 7920
   ggtgtcacgt gctgaagaac cggcggctga catgggaata ctgcgacgtg ccttcctgct 7980
   ctacctgcgg cctgcggcag tactcccagc ctcagttccg gatcaagggc ggcctgttcg 8040
   ccgatatcgc ctcccaccct tggcaggccg ccatcttcgc cgctgctgcc gcttctcctg 8100
   gcgagagatt cctgtgcggc ggcatcctga tctccagctg ctggattctg tctgccgccc 8160
   actgcttcca ggaacggttc cctcctcacc acctgaccgt gatcctgggc cggacctaca 8220
   gagtcgtgcc cggcgaggaa gaacagaaat tcgaggtgga gaagtatatc gtgcacaaag 8280
   agttcgacga cgacacctac gacaacgata tcgccctgct gcagctgaag tccgactcct 8340
   ccagatgcgc ccaggaatcc tccgtcgttc ggaccgtgtg tctgccacct gccgacctgc 8400
   agctgcctga ctggaccgag tgcgagctgt ccggctacgg caagcacgag gccctgtccc 8460
   ccttctactc cgagcggctg aaagaagctc atgtacggct gtacccctct agccggtgca 8520
   cctcccagca tctgctgaac cggaccgtga ccgacaacat gctgtgtgcc ggcgacacca 8580
   gatctggcgg ccctcaggcc aacctgcacg acgcctgcca gggcgatagt ggcggacctc 8640
   tcgtgtgcct caacgacggc aggatgaccc tcgtgggcat catctcttgg ggcctgggct 8700
   gtggccagaa agacgtgcct ggcgtgtaca ccaaagtgac caactacctg gactggatca 8760
   gggacaacat gcggccttga tgagcggccg cccgctgatc agcctcgact gtgccttcta 8820
   gttgccagcc atctgttgtt tgcccctccc ccgtgccttc cttgaccctg gaaggtgcca 8880
   ctcccactgt cctttcctaa taaaatgagg aaattgcatc gcattgtctg agtaggtgtc 8940
   attctattct ggggggtggg gtggggcagg acagcaaggg ggaggattgg gaagacaata 9000
   gcaggcatgc tggggatgcg gtgggctcta tggcttctga ggcggaaaga accagctggg 9060
   gctctagggg gtatccccac ggagctcgca tccataatat aactgtacca ggttttggtt 9120
   tattacatgt gactgacggc ttcctatgcg tgctcagaaa acggcagttg ggcactgcac 9180
   tgcccggtga tggtgccacg gtggctcctg ccgccttctt tgatattcac tctgttgtat 9240
   ttcatctctt gttgccgatg aaaggatata acagtctctg aggaaatact tggtatttct 9300
   tctgatcagc gtttttataa gtaatgttga atattggata aggctgtgtg tcctttgtct 9360
   tgggagacaa agcccacagc aggtggtggt tgggtggtgg cagctcagtg acaggagagg 9420
   tttttttgcc tgtttttttt gttgtttttt ttttttaagt aaggtgttct tttttcttag 9480
   taaaatttct actggactgt atgttttgac aggtcagaaa catttcttca aaagaagaac 9540
   cttttggaaa ctgtacagcc cttttctttc attccctttt tgctttctgt gccaatgcct 9600
   ttggttctga ttgcattatg gaaaacgttg atcggaactt gaggttttta tttatagtgt 9660
   ggcttgaaag cttggatagc tgttgttaca tgagatacct tattaagttt aggccagctt 9720
   gatgctttat tttttttcct ttgaagtagt gagcgttctc tggttttttt cctttgaaac 9780
   tggcgaggct tagatttttc taatgggatt ttttacctga tgatctagtt gcatacccaa 9840
   atgcttgtaa atgttttcct agttaacatg ttgataactt cggatttaca tgttgtatat 9900
   acttgtcatc tgtgtttcta gtaaaaatat atggcattta tagaaatacg taattcctga 9960
   tttccttttt tttttatctc tatgctctgt gtgtacaggt caaacagact tcactcctat 10020
   ttttatttat agaattttat atgcagtctg tcgttggttc ttgtgttgta aggatacagc 10080
   cttaaatttc ctagagcgat gctcagtaag gcgggttgtc acatgggttc aaatgtaaaa 10140
   cgggcacgtt tgctgctgcc ttcccagatc caggacacta aactgcttct gcaactgagg 10200
   tataaatcgc ttcagatccc aggaagtgta gatccacgtg catattctta aagaagaatg 10260
   aatactttct aaaatatgtt ggcataggaa gcaagctgca tggatttatt tgggacttaa 10320
   attattttgg taacggagtg cataggtttt aaacacagtt gcagcatgct aacgagtcac 10380
   agcatttatg cagaagtgat gcctgttgca gctgtttacg gcactgcctt gcagtgagca 10440
   ttgcagatag gggtggggtg ctttgtgtcg tgttgggaca cgctgccaca cagccacctc 10500
   ccgaacatat ctcacctgct gggtactttt caaaccatct tagcagtagt agatgagtta 10560
   ctatgaaaca gagaagttcc tcagttggat attctcatgg gatgtctttt ttcccatgtt 10620
   gggcaaagta tgataaagca tctctatttg taaattatgc acttgttagt tcctgaatcc 10680
   tttctatagc accacttatt gcagcaggtg taggctctgg tgtggcctgt gtctgtgctt 10740
   caatctttta agcttgagct catggcgcgc ctaggccttg acggccttcc ttcaattcgc     10800
   cctatagtga gtcgtattac gtcgcgctca ctggccgtcg ttttacaacg tcgtgactgg     10860
   gaaaaccctg gcgttaccca acttaatcgc cttgcagcac atcccccttt cgccagctgg     10920
   cgtaatagcg aagaggcccg caccgaaacg cccttcccaa cagttgcgca gcctgaatgg     10980
   cgaatgggag cgccctgtag cggccactca accctatctc ggtctattct tttgatttat     11040
   aagggatttt gccgatttcg gcctattggt taaaaaatga gctgatttaa caaaaattta     11100
   acgcgaattt taacaaaata ttaacgctta caatttag                             11138
<210> 9
   <211> 193
   <212> PRT
   <213> Artificial
<220>
   <223> Darbepoetin
<400> 9
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ala Pro Pro Arg Leu
               20                  25                  30
   Ile Cys Asp Ser Arg Val Leu Glu Arg Tyr Leu Leu Glu Ala Lys Glu
           35                  40                  45
   Ala Glu Asn Ile Thr Thr Gly Cys Asn Glu Thr Cys Ser Leu Asn Glu
       50                  55                  60
   Asn Ile Thr Val Pro Asp Thr Lys Val Asn Phe Tyr Ala Trp Lys Arg
   65                  70                  75                  80
   Met Glu Val Gly Gln Gln Ala Val Glu Val Trp Gln Gly Leu Ala Leu
                   85                  90                  95
   Leu Ser Glu Ala Val Leu Arg Gly Gln Ala Leu Leu Val Asn Ser Ser
               100                 105                 110
   Gln Val Asn Glu Thr Leu Gln Leu His Val Asp Lys Ala Val Ser Gly
           115                 120                 125
   Leu Arg Ser Leu Thr Thr Leu Leu Arg Ala Leu Gly Ala Gln Lys Glu
       130                 135                 140
   Ala Ile Ser Pro Pro Asp Ala Ala Ser Ala Ala Pro Leu Arg Thr Ile
   145                 150                 155                 160
   Thr Ala Asp Thr Phe Arg Lys Leu Phe Arg Val Tyr Ser Asn Phe Leu
                   165                 170                 175
   Arg Gly Lys Leu Lys Leu Tyr Thr Gly Glu Ala Cys Arg Thr Gly Asp
   180 185 190
   Arg
<210> 10
   <211> 10023
   <212> DNA
   <213> Artificial
<220>
   <223> pZRC III- DARBE- Hyg
<400> 10
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gattgaacag gatggcctgc atgcgggtag cccggcagcg tgggtggaac     180
   gtctgtttgg ctatgattgg gcgcagcaga ccattggctg ctctgatgcg gcggtgtttc     240
   gtctgagcgc gcagggtcgt ccggtgctgt ttgtgaaaac cgatctgagc ggtgcgctga     300
   acgagctgca ggatgaagcg gcgcgtctga gctggctggc caccaccggt gttccgtgtg     360
   cggcggtgct ggatgtggtg accgaagcgg gccgtgattg gctgctgctg ggcgaagtgc     420
   cgggtcagga tctgctgtct agccatctgg cgccggcaga aaaagtgagc attatggcgg     480
   atgccatgcg tcgtctgcat accctggacc cggcgacctg tccgtttgat catcaggcga     540
   aacatcgtat tgaacgtgcg cgtacccgta tggaagcggg cctggtggat caggatgatc     600
   tggatgaaga acatcagggc ctggcaccgg cagagctgtt tgcgcgtctg aaagcgagca     660
   tgccggatgg cgaagatctg gtggtgaccc atggtgatgc gtgcctgccg aacattatgg     720
   tggaaaatgg ccgttttagc ggctttattg attgcggccg tctgggcgtg gcggatcgtt     780
   atcaggatat tgcgctggcc acccgtgata ttgcggaaga actgggcggc gaatgggcgg     840
   atcgttttct ggtgctgtat ggcattgcgg caccggatag ccagcgtatt gcgttttatc     900
   gtctgctgga tgaatttttc taataactgt cagaccaagt ttactcatat atactttaga     960
   ttgatttaaa acttcatttt taatttaaaa ggatctaggt gaagatcctt tttgataatc    1020
   tcatgaccaa aatcccttaa cgtgagtttt cgttccactg agcgtcagac cccgtagaaa    1080
   agatcaaagg atcttcttga gatccttttt ttctgcgcgt aatctgctgc ttgcaaacaa    1140
   aaaaaccacc gctaccagcg gtggtttgtt tgccggatca agagctacca actctttttc    1200
   cgaaggtaac tggcttcagc agagcgcaga taccaaatac tgttcttcta gtgtagccgt    1260
   agttaggcca ccacttcaag aactctgtag caccgcctac atacctcgct ctgctaatcc    1320
   tgttaccagt ggctgctgcc agtggcgata agtcgtgtct taccgggttg gactcaagac    1380
   gatagttacc ggataaggcg cagcggtcgg gctgaacggg gggttcgtgc acacagccca    1440
   gcttggagcg aacgacctac accgaactga gatacctaca gcgtgagcta tgagaaagcg    1500
   ccacgcttcc cgaagggaga aaggcggaca ggtatccggt aagcggcagg gtcggaacag    1560
   gagagcgcac gagggagctt ccagggggaa acgcctggta tctttatagt cctgtcgggt    1620
   ttcgccacct ctgacttgag cgtcgatttt tgtgatgctc gtcagggggg cggagcctat     1680
   ggaaaaacgc cagcaacgcg gcctttttac ggttcctggc cttttgctgg ccttttgctc     1740
   attaggcacc ccaggcttta cccgaacgac cgagcgcagc gagtcagtga gcgaggaagc     1800
   ggagagcgcc caatacgcaa ggaaacagct atgaccatgt taatgcagct ggcacgacag     1860
   gtttcccgac tggaaagcgg gcagtgaaag gaaggcccat gaggccagtt aattaagagg     1920
   atccttatcg attttaccac atttgtagag gttttacttg ctttaaaaaa cctcccacac     1980
   ctccccctga acctgaaaca taaaatgaat gcaattgttg ttgttaactt gtttattgca     2040
   gcttataatg gttacaaata aagcaatagc atcacaaatt tcacaaataa agcatttttt     2100
   tcactgcatt ctagttgtgg tttgtccaaa ctcatcaatg tatcttatca tgtctgctcg     2160
   aagcggccgg ccgccccgac tctagactat tcctttgccc tcggacgagt gctggggcgt     2220
   cggtttccac tatcggcgag tacttctaca cagccatcgg tccagacggc cgcgcttctg     2280
   cgggcgattt gtgtacgccc gacagtcccg gctccggatc ggacgattgc gtcgcatcga     2340
   ccctgcgccc aagctgcatc atcgaaattg ccgtcaacca agctctgata gagttggtca     2400
   agaccaatgc ggagcatata cgcccggagc cgcggcgatc ctgcaagctc cggatgcctc     2460
   cgctcgaagt agcgcgtctg ctgctccata caagccaacc acggcctcca gaagaagatg     2520
   ttggcgacct cgtattggga atccccgaac atcgcctcgc tccagtcaat gaccgctgtt     2580
   atgcggccat tgtccgtcag gacattgttg gagccgaaat ccgcgtgcac gaggtgccgg     2640
   acttcggggc agtcctcggc ccaaagcatc agctcatcga gagcctgcgc gacggacgca     2700
   ctgacggtgt cgtccatcac agtttgccag tgatacacat ggggatcagc aatcgcgcat     2760
   atgaaatcac gccatgtagt gtattgaccg attccttgcg gtccgaatgg gccgaacccg     2820
   ctcgtctggc taagatcggc cgcagcgatc gcatccatgg cctccgcgac cggctgcaga     2880
   acagcgggca gttcggtttc aggcaggtct tgcaacgtga caccctgtgc acggcgggag     2940
   atgcaatagg tcaggctctc gctgaattcc ccaatgtcaa gcacttccgg aatcgggagc     3000
   gcggccgatg caaagtgccg ataaacataa cgatctttgt agaaaccatc ggcgcagcta     3060
   tttacccgca ggacatatcc acgccctcct acatcgaagc tgaaagcacg agattcttcg     3120
   ccctccgaga gctgcatcag gtcggagacg ctgtcgaact tttcgatcag aaacttctcg     3180
   acagacgtcg cggtgagttc aggctttccg gatctatcca tcatggtggc tttaccaaca     3240
   gtaccggaat gccaagcttt ttgcaaaagc ctaggcctcc aaaaaagcct cctcactact     3300
   tctggaatag ctcagaggcc gaggcggcct cggcctctgc ataaataaaa aaaattagtc     3360
   agcgatgggg cggagaatgg gcggaactgg gcggagttag gggcgggatg ggcggagtta     3420
   ggggcgggac tatggttgct gactaattga gatgcagatc gcagatcctc gggacgctct     3480
   ggccggtgag gcgtgcgcag tcgttgacgc tctagaccgt gcaaaaggag agcctgtaag     3540
   cgggcactct tccgtggtct ggtggataaa ttcgcaaggg tatcatggcg gacgaccggg     3600
   gttcgaaccc cggatccggc cgtccgccgt gatccatgcg gttaccgccc gcgtgtcgaa     3660
   cccaggtgtg cgacgtcaga caacggggga gcgctccttt tggcttcctt ccaggcgcgg     3720
   cggctgctgc gctagctttt ttggccactg gccgcgcgcg gcgtaagcgg ttaggctgga 3780
   aagcgaaagc attaagtggc tcgctccctg tagccggagg gttattttcc aagggttgag 3840
   tcgcaggacc cccggttcga gtctcgggcc ggccggactg cggcgaacgg gggtttgcct 3900
   ccccgtcatg caagaccccg cttgcaaatt cctccggaaa cagggacgag cccctttttt 3960
   gcttttccca gatgcatccg gtgctgcggc agatgcgccc ccctcctcag cagcggcaag 4020
   agcaagagca gcggcagaca tgcagggcac cctccccttc tcctaccgcg tcaggagggg 4080
   caacatccga cgcgtgcatc cataatataa ctgtaccagg ttttggttta ttacatgtga 4140
   ctgacggctt cctatgcgtg ctcagaaaac ggcagttggg cactgcactg cccggtgatg 4200
   gtgccacggt ggctcctgcc gccttctttg atattcactc tgttgtattt catctcttgt 4260
   tgccgatgaa aggatataac agtctctgag gaaatacttg gtatttcttc tgatcagcgt 4320
   ttttataagt aatgttgaat attggataag gctgtgtgtc ctttgtcttg ggagacaaag 4380
   cccacagcag gtggtggttg ggtggtggca gctcagtgac aggagaggtt tttttgcctg 4440
   ttttttttgt tgtttttttt ttttaagtaa ggtgttcttt tttcttagta aaatttctac 4500
   tggactgtat gttttgacag gtcagaaaca tttcttcaaa agaagaacct tttggaaact 4560
   gtacagccct tttctttcat tccctttttg ctttctgtgc caatgccttt ggttctgatt 4620
   gcattatgga aaacgttgat cggaacttga ggtttttatt tatagtgtgg cttgaaagct 4680
   tggatagctg ttgttacatg agatacctta ttaagtttag gccagcttga tgctttattt 4740
   tttttccttt gaagtagtga gcgttctctg gtttttttcc tttgaaactg gcgaggctta 4800
   gatttttcta atgggatttt ttacctgatg atctagttgc atacccaaat gcttgtaaat 4860
   gttttcctag ttaacatgtt gataacttcg gatttacatg ttgtatatac ttgtcatctg 4920
   tgtttctagt aaaaatatat ggcatttata gaaatacgta attcctgatt tccttttttt 4980
   tttatctcta tgctctgtgt gtacaggtca aacagacttc actcctattt ttatttatag 5040
   aattttatat gcagtctgtc gttggttctt gtgttgtaag gatacagcct taaatttcct 5100
   agagcgatgc tcagtaaggc gggttgtcac atgggttcaa atgtaaaacg ggcacgtttg 5160
   ctgctgcctt cccagatcca ggacactaaa ctgcttctgc aactgaggta taaatcgctt 5220
   cagatcccag gaagtgtaga tccacgtgca tattcttaaa gaagaatgaa tactttctaa 5280
   aatatgttgg cataggaagc aagctgcatg gatttatttg ggacttaaat tattttggta 5340
   acggagtgca taggttttaa acacagttgc agcatgctaa cgagtcacag catttatgca 5400
   gaagtgatgc ctgttgcagc tgtttacggc actgccttgc agtgagcatt gcagataggg 5460
   gtggggtgct ttgtgtcgtg ttgggacacg ctgccacaca gccacctccc gaacatatct 5520
   cacctgctgg gtacttttca aaccatctta gcagtagtag atgagttact atgaaacaga 5580
   gaagttcctc agttggatat tctcatggga tgtctttttt cccatgttgg gcaaagtatg 5640
   ataaagcatc tctatttgta aattatgcac ttgttagttc ctgaatcctt tctatagcac 5700
   cacttattgc agcaggtgta ggctctggtg tggcctgtgt ctgtgcttca atcttttaag 5760
   cttacgcgtt gacattgatt attgactagt tattaatagt aatcaattac ggggtcatta 5820
   gttcatagcc catatatgga gttccgcgtt acataactta cggtaaatgg cccgcctggc 5880
   tgaccgccca acgacccccg cccattgacg tcaataatga cgtatgttcc catagtaacg 5940
   ccaataggga ctttccattg acgtcaatgg gtggagtatt tacggtaaac tgcccacttg 6000
   gcagtacatc aagtgtatca tatgccaagt acgcccccta ttgacgtcaa tgacggtaaa 6060
   tggcccgcct ggcattatgc ccagtacatg accttatggg actttcctac ttggcagtac 6120
   atctacgtat tagtcatcgc tattaccatg gtgatgcggt tttggcagta catcaatggg 6180
   cgtggatagc ggtttgactc acggggattt ccaagtctcc accccattga cgtcaatggg 6240
   agtttgtttt ggcaccaaaa tcaacgggac tttccaaaat gtcgtaacaa ctccgcccca 6300
   ttgacgcaaa tgggcggtag gcgtgtacgg tgggaggtct atataagcag agctggttta 6360
   gtgaaccgtc agatcctctt ccgcatcgct gtctgcgagg gccagctgtt ggggtgagta 6420
   ctccctctca aaagcgggca tgacttctgc gctaagaatg tcagtttcca aaaacgagga 6480
   ggatttgata ttcactggcc cgcggtgatg cctttgaggg tggccgcgtc catctggtca 6540
   gaaaagacaa tctttttgtt gtcaagcttc cttgatgatg tcatacttat cctgtccctt 6600
   ttttttccac agctcgcggt tgaggacaaa ctcttcgcgg tctttccagt actcttggat 6660
   cggaaacccg tcggcctccg aacggtactc cgccaccgag ggacctgagc gagtccgcat 6720
   cgaccggatc ggaaaacctc tcgacgtacc aattaattcg ctgtctgcga gggccagctg 6780
   ttggggtgag tactccctct caaaagcggg catgacttct gcgctaagat tgtcagtttc 6840
   caaaaacgag gaggatttga tattcacctg gcccgcggtg atgcctttga gggtggccgc 6900
   gtccatctgg tcagaaaaga caatcttttt gttgtcaagc ttgaggtgtg gcaggcttga 6960
   gatctggcca tacacttgag tgacaatgac atccactttg cctttctctc cacaggtgtc 7020
   cactcccagg tccaactgca ggtcgagcat gcatctaggg cgcccgcact agaggctcga 7080
   gccaccatgg gggtgcacga atgtcctgcc tggctgtggc ttctcctgtc cctgctgtcg 7140
   ctccctctgg gcctcccagt cctgggcgcc ccaccacgcc tcatctgtga cagccgagtc 7200
   ctggagaggt acctcttgga ggccaaggag gccgagaata tcacgacggg ctgtaatgaa 7260
   acctgcagct tgaatgagaa tatcactgtc ccagacacca aagttaattt ctatgcctgg 7320
   aagaggatgg aggtcgggca gcaggccgta gaagtctggc agggcctggc cctgctgtcg 7380
   gaagctgtcc tgcggggcca ggccctgttg gtcaactctt cccaggtgaa cgagaccctg 7440
   cagctgcatg tggataaagc cgtcagtggc cttcgcagcc tcaccactct gcttcgggct 7500
   ctgggagccc agaaggaagc catctcccct ccagatgcgg cctcagctgc tccactccga 7560
   acaatcactg ctgacacttt ccgcaaactc ttccgagtct actccaattt cctccgggga 7620
   aagctgaagc tgtacacagg ggaggcctgc aggacagggg acagatgagc ggccgcccgc 7680
   tgatcagcct cgactgtgcc ttctagttgc cagccatctg ttgtttgccc ctcccccgtg 7740
   ccttccttga ccctggaagg tgccactccc actgtccttt cctaataaaa tgaggaaatt 7800
   gcatcgcatt gtctgagtag gtgtcattct attctggggg gtggggtggg gcaggacagc 7860
   aagggggagg attgggaaga caatagcagg catgctgggg atgcggtggg ctctatggct 7920
   tctgaggcgg aaagaaccag ctggggctct agggggtatc cccacggagc tcgcatccat 7980
   aatataactg taccaggttt tggtttatta catgtgactg acggcttcct atgcgtgctc 8040
   agaaaacggc agttgggcac tgcactgccc ggtgatggtg ccacggtggc tcctgccgcc 8100
   ttctttgata ttcactctgt tgtatttcat ctcttgttgc cgatgaaagg atataacagt 8160
   ctctgaggaa atacttggta tttcttctga tcagcgtttt tataagtaat gttgaatatt 8220
   ggataaggct gtgtgtcctt tgtcttggga gacaaagccc acagcaggtg gtggttgggt 8280
   ggtggcagct cagtgacagg agaggttttt ttgcctgttt tttttgttgt tttttttttt 8340
   taagtaaggt gttctttttt cttagtaaaa tttctactgg actgtatgtt ttgacaggtc 8400
   agaaacattt cttcaaaaga agaacctttt ggaaactgta cagccctttt ctttcattcc 8460
   ctttttgctt tctgtgccaa tgcctttggt tctgattgca ttatggaaaa cgttgatcgg 8520
   aacttgaggt ttttatttat agtgtggctt gaaagcttgg atagctgttg ttacatgaga 8580
   taccttatta agtttaggcc agcttgatgc tttatttttt ttcctttgaa gtagtgagcg 8640
   ttctctggtt tttttccttt gaaactggcg aggcttagat ttttctaatg ggatttttta 8700
   cctgatgatc tagttgcata cccaaatgct tgtaaatgtt ttcctagtta acatgttgat 8760
   aacttcggat ttacatgttg tatatacttg tcatctgtgt ttctagtaaa aatatatggc 8820
   atttatagaa atacgtaatt cctgatttcc tttttttttt atctctatgc tctgtgtgta 8880
   caggtcaaac agacttcact cctattttta tttatagaat tttatatgca gtctgtcgtt 8940
   ggttcttgtg ttgtaaggat acagccttaa atttcctaga gcgatgctca gtaaggcggg 9000
   ttgtcacatg ggttcaaatg taaaacgggc acgtttgctg ctgccttccc agatccagga 9060
   cactaaactg cttctgcaac tgaggtataa atcgcttcag atcccaggaa gtgtagatcc 9120
   acgtgcatat tcttaaagaa gaatgaatac tttctaaaat atgttggcat aggaagcaag 9180
   ctgcatggat ttatttggga cttaaattat tttggtaacg gagtgcatag gttttaaaca 9240
   cagttgcagc atgctaacga gtcacagcat ttatgcagaa gtgatgcctg ttgcagctgt 9300
   ttacggcact gccttgcagt gagcattgca gataggggtg gggtgctttg tgtcgtgttg 9360
   ggacacgctg ccacacagcc acctcccgaa catatctcac ctgctgggta cttttcaaac 9420
   catcttagca gtagtagatg agttactatg aaacagagaa gttcctcagt tggatattct 9480
   catgggatgt cttttttccc atgttgggca aagtatgata aagcatctct atttgtaaat 9540
   tatgcacttg ttagttcctg aatcctttct atagcaccac ttattgcagc aggtgtaggc 9600
   tctggtgtgg cctgtgtctg tgcttcaatc ttttaagctt gagctcatgg cgcgcctagg 9660
   ccttgacggc cttccttcaa ttcgccctat agtgagtcgt attacgtcgc gctcactggc 9720
   cgtcgtttta caacgtcgtg actgggaaaa ccctggcgtt acccaactta atcgccttgc 9780
   agcacatccc cctttcgcca gctggcgtaa tagcgaagag gcccgcaccg aaacgccctt 9840
   cccaacagtt gcgcagcctg aatggcgaat gggagcgccc tgtagcggcc actcaaccct 9900
   atctcggtct attcttttga tttataaggg attttgccga tttcggccta ttggttaaaa 9960
   aatgagctga tttaacaaaa atttaacgcg aattttaaca aaatattaac gcttacaatt 10020
   tag 10023
<210> 11
   <211> 489
   <212> PRT
   <213> Artificial
<220>
   <223> Etanercept
<400> 11
   Met Ala Pro Val Ala Val Trp Ala Ala Leu Ala Val Gly Leu Glu Leu
   1               5                   10                  15
   Trp Ala Ala Ala His Ala Leu Pro Ala Gln Val Ala Phe Thr Pro Tyr
               20                  25                  30
   Ala Pro Glu Pro Gly Ser Thr Cys Arg Leu Arg Glu Tyr Tyr Asp Gln
           35                  40                  45
   Thr Ala Gln Met Cys Cys Ser Lys Cys Ser Pro Gly Gln His Ala Lys
       50                  55                  60
   Val Phe Cys Thr Lys Thr Ser Asp Thr Val Cys Asp Ser Cys Glu Asp
   65                  70                  75                  80
   Ser Thr Tyr Thr Gln Leu Trp Asn Trp Val Pro Glu Cys Leu Ser Cys
                   85                  90                  95
   Gly Ser Arg Cys Ser Ser Asp Gln Val Glu Thr Gln Ala Cys Thr Arg
               100                 105                 110
   Glu Gln Asn Arg Ile Cys Thr Cys Arg Pro Gly Trp Tyr Cys Ala Leu
           115                 120                 125
   Ser Lys Gln Glu Gly Cys Arg Leu Cys Ala Pro Leu Arg Lys Cys Arg
       130                 135                 140
   Pro Gly Phe Gly Val Ala Arg Pro Gly Thr Glu Thr Ser Asp Val Val
   145                 150                 155                 160
   Cys Lys Pro Cys Ala Pro Gly Thr Phe Ser Asn Thr Thr Ser Ser Thr
                   165                 170                 175
   Asp Ile Cys Arg Pro His Gln Ile Cys Asn Val Val Ala Ile Pro Gly
               180                 185                 190
   Asn Ala Ser Met Asp Ala Val Cys Thr Ser Thr Ser Pro Thr Arg Ser
           195                 200                 205
   Met Ala Pro Gly Ala Val His Leu Pro Gln Pro Val Ser Thr Arg Ser
       210                 215                 220
   Gln His Thr Gln Pro Thr Pro Glu Pro Ser Thr Ala Pro Ser Thr Ser
   225 230 235 240
   Phe Leu Leu Pro Met Gly Pro Ser Pro Pro Ala Glu Gly Ser Thr Gly
   245 250 255
   Asp Glu Pro Lys Ser Cys Asp Lys Thr His Thr Cys Pro Pro Cys Pro
   260 265 270
   Ala Pro Glu Leu Leu Gly Gly Pro Ser Val Phe Leu Phe Pro Pro Lys
   275 280 285
   Pro Lys Asp Thr Leu Met Ile Ser Arg Thr Pro Glu Val Thr Cys Val
   290 295 300
   Val Val Asp Val Ser His Glu Asp Pro Glu Val Lys Phe Asn Trp Tyr
   305 310 315 320
   Val Asp Gly Val Glu Val His Asn Ala Lys Thr Lys Pro Arg Glu Glu
   325 330 335
   Gln Tyr Asn Ser Thr Tyr Arg Val Val Ser Val Leu Thr Val Leu His
   340 345 350
   Gln Asp Trp Leu Asn Gly Lys Glu Tyr Lys Cys Lys Val Ser Asn Lys
   355 360 365
   Ala Leu Pro Ala Pro Ile Glu Lys Thr Ile Ser Lys Ala Lys Gly Gln
   370 375 380
   Pro Arg Glu Pro Gln Val Tyr Thr Leu Pro Pro Ser Arg Glu Glu Met
   385 390 395 400
   Thr Lys Asn Gln Val Ser Leu Thr Cys Leu Val Lys Gly Phe Tyr Pro
   405 410 415
   Ser Asp Ile Ala Val Glu Trp Glu Ser Asn Gly Gln Pro Glu Asn Asn
   420 425 430
   Tyr Lys Thr Thr Pro Pro Val Leu Asp Ser Asp Gly Ser Phe Phe Leu
   435 440 445
   Tyr Ser Lys Leu Thr Val Asp Lys Ser Arg Trp Gln Gln Gly Asn Val
   450 455 460
   Phe Ser Cys Ser Val Met His Glu Ala Leu His Asn His Tyr Thr Gln
   465 470 475 480
   Lys Ser Leu Ser Leu Ser Pro Gly Lys
   485
<210> 12
   <211> 10911
   <212> DNA
   <213> Artificial
<220>
   <223> pZRC III - Etanercept - Hyg
<400> 12
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gattgaacag gatggcctgc atgcgggtag cccggcagcg tgggtggaac     180
   gtctgtttgg ctatgattgg gcgcagcaga ccattggctg ctctgatgcg gcggtgtttc     240
   gtctgagcgc gcagggtcgt ccggtgctgt ttgtgaaaac cgatctgagc ggtgcgctga     300
   acgagctgca ggatgaagcg gcgcgtctga gctggctggc caccaccggt gttccgtgtg     360
   cggcggtgct ggatgtggtg accgaagcgg gccgtgattg gctgctgctg ggcgaagtgc     420
   cgggtcagga tctgctgtct agccatctgg cgccggcaga aaaagtgagc attatggcgg     480
   atgccatgcg tcgtctgcat accctggacc cggcgacctg tccgtttgat catcaggcga     540
   aacatcgtat tgaacgtgcg cgtacccgta tggaagcggg cctggtggat caggatgatc     600
   tggatgaaga acatcagggc ctggcaccgg cagagctgtt tgcgcgtctg aaagcgagca     660
   tgccggatgg cgaagatctg gtggtgaccc atggtgatgc gtgcctgccg aacattatgg     720
   tggaaaatgg ccgttttagc ggctttattg attgcggccg tctgggcgtg gcggatcgtt     780
   atcaggatat tgcgctggcc acccgtgata ttgcggaaga actgggcggc gaatgggcgg     840
   atcgttttct ggtgctgtat ggcattgcgg caccggatag ccagcgtatt gcgttttatc     900
   gtctgctgga tgaatttttc taataactgt cagaccaagt ttactcatat atactttaga     960
   ttgatttaaa acttcatttt taatttaaaa ggatctaggt gaagatcctt tttgataatc    1020
   tcatgaccaa aatcccttaa cgtgagtttt cgttccactg agcgtcagac cccgtagaaa    1080
   agatcaaagg atcttcttga gatccttttt ttctgcgcgt aatctgctgc ttgcaaacaa    1140
   aaaaaccacc gctaccagcg gtggtttgtt tgccggatca agagctacca actctttttc    1200
   cgaaggtaac tggcttcagc agagcgcaga taccaaatac tgttcttcta gtgtagccgt    1260
   agttaggcca ccacttcaag aactctgtag caccgcctac atacctcgct ctgctaatcc    1320
   tgttaccagt ggctgctgcc agtggcgata agtcgtgtct taccgggttg gactcaagac    1380
   gatagttacc ggataaggcg cagcggtcgg gctgaacggg gggttcgtgc acacagccca    1440
   gcttggagcg aacgacctac accgaactga gatacctaca gcgtgagcta tgagaaagcg    1500
   ccacgcttcc cgaagggaga aaggcggaca ggtatccggt aagcggcagg gtcggaacag    1560
   gagagcgcac gagggagctt ccagggggaa acgcctggta tctttatagt cctgtcgggt    1620
   ttcgccacct ctgacttgag cgtcgatttt tgtgatgctc gtcagggggg cggagcctat    1680
   ggaaaaacgc cagcaacgcg gcctttttac ggttcctggc cttttgctgg ccttttgctc    1740
   attaggcacc ccaggcttta cccgaacgac cgagcgcagc gagtcagtga gcgaggaagc    1800
   ggagagcgcc caatacgcaa ggaaacagct atgaccatgt taatgcagct ggcacgacag    1860
   gtttcccgac tggaaagcgg gcagtgaaag gaaggcccat gaggccagtt aattaagagg    1920
   atccttatcg attttaccac atttgtagag gttttacttg ctttaaaaaa cctcccacac 1980
   ctccccctga acctgaaaca taaaatgaat gcaattgttg ttgttaactt gtttattgca 2040
   gcttataatg gttacaaata aagcaatagc atcacaaatt tcacaaataa agcatttttt 2100
   tcactgcatt ctagttgtgg tttgtccaaa ctcatcaatg tatcttatca tgtctgctcg 2160
   aagcggccgg ccgccccgac tctagactat tcctttgccc tcggacgagt gctggggcgt 2220
   cggtttccac tatcggcgag tacttctaca cagccatcgg tccagacggc cgcgcttctg 2280
   cgggcgattt gtgtacgccc gacagtcccg gctccggatc ggacgattgc gtcgcatcga 2340
   ccctgcgccc aagctgcatc atcgaaattg ccgtcaacca agctctgata gagttggtca 2400
   agaccaatgc ggagcatata cgcccggagc cgcggcgatc ctgcaagctc cggatgcctc 2460
   cgctcgaagt agcgcgtctg ctgctccata caagccaacc acggcctcca gaagaagatg 2520
   ttggcgacct cgtattggga atccccgaac atcgcctcgc tccagtcaat gaccgctgtt 2580
   atgcggccat tgtccgtcag gacattgttg gagccgaaat ccgcgtgcac gaggtgccgg 2640
   acttcggggc agtcctcggc ccaaagcatc agctcatcga gagcctgcgc gacggacgca 2700
   ctgacggtgt cgtccatcac agtttgccag tgatacacat ggggatcagc aatcgcgcat 2760
   atgaaatcac gccatgtagt gtattgaccg attccttgcg gtccgaatgg gccgaacccg 2820
   ctcgtctggc taagatcggc cgcagcgatc gcatccatgg cctccgcgac cggctgcaga 2880
   acagcgggca gttcggtttc aggcaggtct tgcaacgtga caccctgtgc acggcgggag 2940
   atgcaatagg tcaggctctc gctgaattcc ccaatgtcaa gcacttccgg aatcgggagc 3000
   gcggccgatg caaagtgccg ataaacataa cgatctttgt agaaaccatc ggcgcagcta 3060
   tttacccgca ggacatatcc acgccctcct acatcgaagc tgaaagcacg agattcttcg 3120
   ccctccgaga gctgcatcag gtcggagacg ctgtcgaact tttcgatcag aaacttctcg 3180
   acagacgtcg cggtgagttc aggctttccg gatctatcca tcatggtggc tttaccaaca 3240
   gtaccggaat gccaagcttt ttgcaaaagc ctaggcctcc aaaaaagcct cctcactact 3300
   tctggaatag ctcagaggcc gaggcggcct cggcctctgc ataaataaaa aaaattagtc 3360
   agcgatgggg cggagaatgg gcggaactgg gcggagttag gggcgggatg ggcggagtta 3420
   ggggcgggac tatggttgct gactaattga gatgcagatc gcagatcctc gggacgctct 3480
   ggccggtgag gcgtgcgcag tcgttgacgc tctagaccgt gcaaaaggag agcctgtaag 3540
   cgggcactct tccgtggtct ggtggataaa ttcgcaaggg tatcatggcg gacgaccggg 3600
   gttcgaaccc cggatccggc cgtccgccgt gatccatgcg gttaccgccc gcgtgtcgaa 3660
   cccaggtgtg cgacgtcaga caacggggga gcgctccttt tggcttcctt ccaggcgcgg 3720
   cggctgctgc gctagctttt ttggccactg gccgcgcgcg gcgtaagcgg ttaggctgga 3780
   aagcgaaagc attaagtggc tcgctccctg tagccggagg gttattttcc aagggttgag 3840
   tcgcaggacc cccggttcga gtctcgggcc ggccggactg cggcgaacgg gggtttgcct 3900
   ccccgtcatg caagaccccg cttgcaaatt cctccggaaa cagggacgag cccctttttt 3960
   gcttttccca gatgcatccg gtgctgcggc agatgcgccc ccctcctcag cagcggcaag 4020
   agcaagagca gcggcagaca tgcagggcac cctccccttc tcctaccgcg tcaggagggg    4080
   caacatccga cgcgtgcatc cataatataa ctgtaccagg ttttggttta ttacatgtga 4140
   ctgacggctt cctatgcgtg ctcagaaaac ggcagttggg cactgcactg cccggtgatg 4200
   gtgccacggt ggctcctgcc gccttctttg atattcactc tgttgtattt catctcttgt 4260
   tgccgatgaa aggatataac agtctctgag gaaatacttg gtatttcttc tgatcagcgt 4320
   ttttataagt aatgttgaat attggataag gctgtgtgtc ctttgtcttg ggagacaaag 4380
   cccacagcag gtggtggttg ggtggtggca gctcagtgac aggagaggtt tttttgcctg 4440
   ttttttttgt tgtttttttt ttttaagtaa ggtgttcttt tttcttagta aaatttctac 4500
   tggactgtat gttttgacag gtcagaaaca tttcttcaaa agaagaacct tttggaaact 4560
   gtacagccct tttctttcat tccctttttg ctttctgtgc caatgccttt ggttctgatt 4620
   gcattatgga aaacgttgat cggaacttga ggtttttatt tatagtgtgg cttgaaagct 4680
   tggatagctg ttgttacatg agatacctta ttaagtttag gccagcttga tgctttattt 4740
   tttttccttt gaagtagtga gcgttctctg gtttttttcc tttgaaactg gcgaggctta 4800
   gatttttcta atgggatttt ttacctgatg atctagttgc atacccaaat gcttgtaaat 4860
   gttttcctag ttaacatgtt gataacttcg gatttacatg ttgtatatac ttgtcatctg 4920
   tgtttctagt aaaaatatat ggcatttata gaaatacgta attcctgatt tccttttttt 4980
   tttatctcta tgctctgtgt gtacaggtca aacagacttc actcctattt ttatttatag 5040
   aattttatat gcagtctgtc gttggttctt gtgttgtaag gatacagcct taaatttcct 5100
   agagcgatgc tcagtaaggc gggttgtcac atgggttcaa atgtaaaacg ggcacgtttg 5160
   ctgctgcctt cccagatcca ggacactaaa ctgcttctgc aactgaggta taaatcgctt 5220
   cagatcccag gaagtgtaga tccacgtgca tattcttaaa gaagaatgaa tactttctaa 5280
   aatatgttgg cataggaagc aagctgcatg gatttatttg ggacttaaat tattttggta 5340
   acggagtgca taggttttaa acacagttgc agcatgctaa cgagtcacag catttatgca 5400
   gaagtgatgc ctgttgcagc tgtttacggc actgccttgc agtgagcatt gcagataggg 5460
   gtggggtgct ttgtgtcgtg ttgggacacg ctgccacaca gccacctccc gaacatatct 5520
   cacctgctgg gtacttttca aaccatctta gcagtagtag atgagttact atgaaacaga 5580
   gaagttcctc agttggatat tctcatggga tgtctttttt cccatgttgg gcaaagtatg 5640
   ataaagcatc tctatttgta aattatgcac ttgttagttc ctgaatcctt tctatagcac 5700
   cacttattgc agcaggtgta ggctctggtg tggcctgtgt ctgtgcttca atcttttaag 5760
   cttacgcgtt gacattgatt attgactagt tattaatagt aatcaattac ggggtcatta 5820
   gttcatagcc catatatgga gttccgcgtt acataactta cggtaaatgg cccgcctggc 5880
   tgaccgccca acgacccccg cccattgacg tcaataatga cgtatgttcc catagtaacg 5940
   ccaataggga ctttccattg acgtcaatgg gtggagtatt tacggtaaac tgcccacttg 6000
   gcagtacatc aagtgtatca tatgccaagt acgcccccta ttgacgtcaa tgacggtaaa 6060
   tggcccgcct ggcattatgc ccagtacatg accttatggg actttcctac ttggcagtac 6120
   atctacgtat tagtcatcgc tattaccatg gtgatgcggt tttggcagta catcaatggg 6180
   cgtggatagc ggtttgactc acggggattt ccaagtctcc accccattga cgtcaatggg 6240
   agtttgtttt ggcaccaaaa tcaacgggac tttccaaaat gtcgtaacaa ctccgcccca 6300
   ttgacgcaaa tgggcggtag gcgtgtacgg tgggaggtct atataagcag agctggttta 6360
   gtgaaccgtc agatcctctt ccgcatcgct gtctgcgagg gccagctgtt ggggtgagta 6420
   ctccctctca aaagcgggca tgacttctgc gctaagaatg tcagtttcca aaaacgagga 6480
   ggatttgata ttcactggcc cgcggtgatg cctttgaggg tggccgcgtc catctggtca 6540
   gaaaagacaa tctttttgtt gtcaagcttc cttgatgatg tcatacttat cctgtccctt 6600
   ttttttccac agctcgcggt tgaggacaaa ctcttcgcgg tctttccagt actcttggat 6660
   cggaaacccg tcggcctccg aacggtactc cgccaccgag ggacctgagc gagtccgcat 6720
   cgaccggatc ggaaaacctc tcgacgtacc aattaattcg ctgtctgcga gggccagctg 6780
   ttggggtgag tactccctct caaaagcggg catgacttct gcgctaagat tgtcagtttc 6840
   caaaaacgag gaggatttga tattcacctg gcccgcggtg atgcctttga gggtggccgc 6900
   gtccatctgg tcagaaaaga caatcttttt gttgtcaagc ttgaggtgtg gcaggcttga 6960
   gatctggcca tacacttgag tgacaatgac atccactttg cctttctctc cacaggtgtc 7020
   cactcccagg tccaactgca ggtcgagcat gcatctaggg cgcccgcact agaggctcga 7080
   gccatggctc cagtggctgt gtgggctgct ctggctgtgg gcctggaact gtgggccgct 7140
   gctcacgctc tgcctgctca ggtggccttc accccttatg cccctgagcc tggctccacc 7200
   tgcaggctgc gggagtacta cgaccagacc gcccagatgt gctgctccaa gtgctctcct 7260
   ggccagcacg ccaaggtgtt ctgcaccaag acctccgaca ccgtgtgcga ctcttgcgag 7320
   gactccacct acacccagct ctggaactgg gtgcccgagt gcctgtcctg cggctccaga 7380
   tgctcctccg accaggtgga gacacaggcc tgtacacggg agcagaatcg gatttgcaca 7440
   tgcaggcctg gctggtactg cgccctgtcc aagcaggaag gatgcaggct gtgcgcccct 7500
   ctgaggaagt gcagacctgg cttcggcgtg gctaggcctg gcaccgagac atccgacgtc 7560
   gtgtgcaagc cttgtgcccc tggcaccttc tccaacacca catcctccac cgacatctgc 7620
   cggcctcacc agatctgcaa cgtggtggcc atccctggca acgccagcat ggacgccgtg 7680
   tgcacctcca cctcccccac cagatctatg gcccctggcg ccgtgcatct gcctcagcct 7740
   gtgtccaccc ggtcccagca tacccagcct acacctgagc cctctaccgc cccttctacc 7800
   tccttcctgc tgcctatggg cccttctcct ccagctgagg gctctaccgg cgacgagcct 7860
   aagtcctgcg acaagaccca cacctgtccc ccctgccctg cccctgaact gctgggaggc 7920
   cccagcgtgt tcctgttccc cccaaagccc aaggacaccc tgatgatctc ccggaccccc 7980
   gaagtgacct gcgtggtggt ggacgtgtcc cacgaggacc ctgaagtgaa gttcaattgg 8040
   tacgtggacg gcgtggaagt gcacaacgcc aagaccaagc ccagagagga acagtacaac 8100
   tccacctacc gggtggtgtc cgtgctgacc gtgctgcacc aggactggct gaacggcaaa 8160
   gagtacaagt gcaaggtgtc caacaaggct ctgcctgccc ccatcgaaaa gaccatctcc 8220
   aaggccaagg gccagccccg cgagcctcag gtgtacaccc tgcctccttc ccgggaggag 8280
   atgaccaaga accaggtgtc cctgacctgt ctggtcaagg gcttctaccc ctccgatatc 8340
   gccgtggaat gggagtccaa cggacagccc gagaacaact acaagaccac cccccctgtg 8400
   ctggactccg acggctcatt cttcctgtac tccaagctga ccgtggacaa gtcccggtgg 8460
   cagcagggca acgtgttctc ctgctccgtg atgcacgagg ccctgcacaa ccactacacc 8520
   cagaagagct tatccctgtc tcctggcaag tgataagcgg ccgcccgctg atcagcctcg 8580
   actgtgcctt ctagttgcca gccatctgtt gtttgcccct cccccgtgcc ttccttgacc 8640
   ctggaaggtg ccactcccac tgtcctttcc taataaaatg aggaaattgc atcgcattgt 8700
   ctgagtaggt gtcattctat tctggggggt ggggtggggc aggacagcaa gggggaggat 8760
   tgggaagaca atagcaggca tgctggggat gcggtgggct ctatggcttc tgaggcggaa 8820
   agaaccagct ggggctctag ggggtatccc cacggagctc gcatccataa tataactgta 8880
   ccaggttttg gtttattaca tgtgactgac ggcttcctat gcgtgctcag aaaacggcag 8940
   ttgggcactg cactgcccgg tgatggtgcc acggtggctc ctgccgcctt ctttgatatt 9000
   cactctgttg tatttcatct cttgttgccg atgaaaggat ataacagtct ctgaggaaat 9060
   acttggtatt tcttctgatc agcgttttta taagtaatgt tgaatattgg ataaggctgt 9120
   gtgtcctttg tcttgggaga caaagcccac agcaggtggt ggttgggtgg tggcagctca 9180
   gtgacaggag aggttttttt gcctgttttt tttgttgttt ttttttttta agtaaggtgt 9240
   tcttttttct tagtaaaatt tctactggac tgtatgtttt gacaggtcag aaacatttct 9300
   tcaaaagaag aaccttttgg aaactgtaca gcccttttct ttcattccct ttttgctttc 9360
   tgtgccaatg cctttggttc tgattgcatt atggaaaacg ttgatcggaa cttgaggttt 9420
   ttatttatag tgtggcttga aagcttggat agctgttgtt acatgagata ccttattaag 9480
   tttaggccag cttgatgctt tatttttttt cctttgaagt agtgagcgtt ctctggtttt 9540
   tttcctttga aactggcgag gcttagattt ttctaatggg attttttacc tgatgatcta 9600
   gttgcatacc caaatgcttg taaatgtttt cctagttaac atgttgataa cttcggattt 9660
   acatgttgta tatacttgtc atctgtgttt ctagtaaaaa tatatggcat ttatagaaat 9720
   acgtaattcc tgatttcctt ttttttttat ctctatgctc tgtgtgtaca ggtcaaacag 9780
   acttcactcc tatttttatt tatagaattt tatatgcagt ctgtcgttgg ttcttgtgtt 9840
   gtaaggatac agccttaaat ttcctagagc gatgctcagt aaggcgggtt gtcacatggg 9900
   ttcaaatgta aaacgggcac gtttgctgct gccttcccag atccaggaca ctaaactgct 9960
   tctgcaactg aggtataaat cgcttcagat cccaggaagt gtagatccac gtgcatattc 10020
   ttaaagaaga atgaatactt tctaaaatat gttggcatag gaagcaagct gcatggattt 10080
   atttgggact taaattattt tggtaacgga gtgcataggt tttaaacaca gttgcagcat 10140
   gctaacgagt cacagcattt atgcagaagt gatgcctgtt gcagctgttt acggcactgc 10200
   cttgcagtga gcattgcaga taggggtggg gtgctttgtg tcgtgttggg acacgctgcc 10260
   acacagccac ctcccgaaca tatctcacct gctgggtact tttcaaacca tcttagcagt 10320
   agtagatgag ttactatgaa acagagaagt tcctcagttg gatattctca tgggatgtct 10380
   tttttcccat gttgggcaaa gtatgataaa gcatctctat ttgtaaatta tgcacttgtt 10440
   agttcctgaa tcctttctat agcaccactt attgcagcag gtgtaggctc tggtgtggcc 10500
   tgtgtctgtg cttcaatctt ttaagcttga gctcatggcg cgcctaggcc ttgacggcct 10560
   tccttcaatt cgccctatag tgagtcgtat tacgtcgcgc tcactggccg tcgttttaca 10620
   acgtcgtgac tgggaaaacc ctggcgttac ccaacttaat cgccttgcag cacatccccc 10680
   tttcgccagc tggcgtaata gcgaagaggc ccgcaccgaa acgcccttcc caacagttgc 10740
   gcagcctgaa tggcgaatgg gagcgccctg tagcggccac tcaaccctat ctcggtctat 10800
   tcttttgatt tataagggat tttgccgatt tcggcctatt ggttaaaaaa tgagctgatt 10860
   taacaaaaat ttaacgcgaa ttttaacaaa atattaacgc ttacaattta g 10911
<210> 13
   <211> 116
   <212> PRT
   <213> Artificial
<220>
   <223> FSH alpha
<400> 13
   Met Asp Tyr Tyr Arg Lys Tyr Ala Ala Ile Phe Leu Val Thr Leu Ser
   1 5 10 15
   Val Phe Leu His Val Leu His Ser Ala Pro Asp Val Gln Asp Cys Pro
   20 25 30
   Glu Cys Thr Leu Gln Glu Asn Pro Phe Phe Ser Gln Pro Gly Ala Pro
   35 40 45
   Ile Leu Gln Cys Met Gly Cys Cys Phe Ser Arg Ala Tyr Pro Thr Pro
   50 55 60
   Leu Arg Ser Lys Lys Thr Met Leu Val Gln Lys Asn Val Thr Ser Glu
   65 70 75 80
   Ser Thr Cys Cys Val Ala Lys Ser Tyr Asn Arg Val Thr Val Met Gly
   85 90 95
   Gly Phe Lys Val Glu Asn His Thr Ala Cys His Cys Ser Thr Cys Tyr
   100 105 110
   Tyr His Lys Ser
   115
<210> 14
   <211> 599
   <212> DNA
   <213> Artificial
<220>
   <223> IRES
<400> 14
   gcggccgcgc cgcactagag gaattccgcc cctctccccc cccccctaac gttactggcc      60
   gaagccgctt ggaataaggc cggtgtgcgt ttgtctatat gttattttcc accatattgc     120
   cgtcttttgg caatgtgagg gcccggaaac ctggccctgt cttcttgacg agcattccta     180
   ggggtctttc ccctctcgcc aaaggaatgc aaggtctgtt gaatgtcgtg aaggaagcag     240
   ttcctctgga agcttcttga agacaaacaa cgtctgtagc gaccctttgc aggcagcgga     300
   accccccacc tggcgacagg tgcctctgcg gccaaaagcc acgtgtataa gatacacctg     360
   caaaggcggc acaaccccag tgccacgttg tgagttggat agttgtggaa agagtcaaat     420
   ggctctcctc aagcgtattc aacaaggggc tgaaggatgc ccagaaggta ccccattgta     480
   tgggatctga tctggggcct cggtgcacat gctttacatg tgtttagtcg aggttaaaaa     540
   aacgtctagg ccccccgaac cacggggacg tggttttcct ttgaaaaaca cgatgataa      599
<210> 15
   <211> 129
   <212> PRT
   <213> Artificial
<220>
   <223> FSH beta
<400> 15
   Met Lys Thr Leu Gln Phe Phe Phe Leu Phe Cys Cys Trp Lys Ala Ile
   1               5                   10                  15
   Cys Cys Asn Ser Cys Glu Leu Thr Asn Ile Thr Ile Ala Ile Glu Lys
               20                  25                  30
   Glu Glu Cys Arg Phe Cys Ile Ser Ile Asn Thr Thr Trp Cys Ala Gly
           35                  40                  45
   Tyr Cys Tyr Thr Arg Asp Leu Val Tyr Lys Asp Pro Ala Arg Pro Lys
       50                  55                  60
   Ile Gln Lys Thr Cys Thr Phe Lys Glu Leu Val Tyr Glu Thr Val Arg
   65                  70                  75                  80
   Val Pro Gly Cys Ala His His Ala Asp Ser Leu Tyr Thr Tyr Pro Val
                   85                  90                  95
   Ala Thr Gln Cys His Cys Gly Lys Cys Asp Ser Asp Ser Thr Asp Cys
               100                 105                 110
   Thr Val Arg Gly Leu Gly Pro Ser Tyr Cys Ser Phe Gly Glu Met Lys
           115                 120                 125
   Glu
<210> 16
   <211> 10801
   <212> DNA
   <213> Artificial
<220>
   <223> pZRC III- FSH alpha- IRES- FSH beta- Hyg
<400> 16
   gtggcacttt tcggggaaat gtgcgcggaa cccctatttg tttatttttc taaatacatt      60
   caaatatgta tccgctcatg agacaataac cctgataaat gcttcaataa tattgaaaaa     120
   ggaagagtat gattgaacag gatggcctgc atgcgggtag cccggcagcg tgggtggaac     180
   gtctgtttgg ctatgattgg gcgcagcaga ccattggctg ctctgatgcg gcggtgtttc     240
   gtctgagcgc gcagggtcgt ccggtgctgt ttgtgaaaac cgatctgagc ggtgcgctga     300
   acgagctgca ggatgaagcg gcgcgtctga gctggctggc caccaccggt gttccgtgtg     360
   cggcggtgct ggatgtggtg accgaagcgg gccgtgattg gctgctgctg ggcgaagtgc     420
   cgggtcagga tctgctgtct agccatctgg cgccggcaga aaaagtgagc attatggcgg     480
   atgccatgcg tcgtctgcat accctggacc cggcgacctg tccgtttgat catcaggcga     540
   aacatcgtat tgaacgtgcg cgtacccgta tggaagcggg cctggtggat caggatgatc     600
   tggatgaaga acatcagggc ctggcaccgg cagagctgtt tgcgcgtctg aaagcgagca     660
   tgccggatgg cgaagatctg gtggtgaccc atggtgatgc gtgcctgccg aacattatgg     720
   tggaaaatgg ccgttttagc ggctttattg attgcggccg tctgggcgtg gcggatcgtt     780
   atcaggatat tgcgctggcc acccgtgata ttgcggaaga actgggcggc gaatgggcgg     840
   atcgttttct ggtgctgtat ggcattgcgg caccggatag ccagcgtatt gcgttttatc     900
   gtctgctgga tgaatttttc taataactgt cagaccaagt ttactcatat atactttaga     960
   ttgatttaaa acttcatttt taatttaaaa ggatctaggt gaagatcctt tttgataatc    1020
   tcatgaccaa aatcccttaa cgtgagtttt cgttccactg agcgtcagac cccgtagaaa    1080
   agatcaaagg atcttcttga gatccttttt ttctgcgcgt aatctgctgc ttgcaaacaa    1140
   aaaaaccacc gctaccagcg gtggtttgtt tgccggatca agagctacca actctttttc    1200
   cgaaggtaac tggcttcagc agagcgcaga taccaaatac tgttcttcta gtgtagccgt    1260
   agttaggcca ccacttcaag aactctgtag caccgcctac atacctcgct ctgctaatcc    1320
   tgttaccagt ggctgctgcc agtggcgata agtcgtgtct taccgggttg gactcaagac    1380
   gatagttacc ggataaggcg cagcggtcgg gctgaacggg gggttcgtgc acacagccca    1440
   gcttggagcg aacgacctac accgaactga gatacctaca gcgtgagcta tgagaaagcg    1500
   ccacgcttcc cgaagggaga aaggcggaca ggtatccggt aagcggcagg gtcggaacag    1560
   gagagcgcac gagggagctt ccagggggaa acgcctggta tctttatagt cctgtcgggt    1620
   ttcgccacct ctgacttgag cgtcgatttt tgtgatgctc gtcagggggg cggagcctat    1680
   ggaaaaacgc cagcaacgcg gcctttttac ggttcctggc cttttgctgg ccttttgctc    1740
   attaggcacc ccaggcttta cccgaacgac cgagcgcagc gagtcagtga gcgaggaagc    1800
   ggagagcgcc caatacgcaa ggaaacagct atgaccatgt taatgcagct ggcacgacag    1860
   gtttcccgac tggaaagcgg gcagtgaaag gaaggcccat gaggccagtt aattaagagg 1920
   atccttatcg attttaccac atttgtagag gttttacttg ctttaaaaaa cctcccacac 1980
   ctccccctga acctgaaaca taaaatgaat gcaattgttg ttgttaactt gtttattgca 2040
   gcttataatg gttacaaata aagcaatagc atcacaaatt tcacaaataa agcatttttt 2100
   tcactgcatt ctagttgtgg tttgtccaaa ctcatcaatg tatcttatca tgtctgctcg 2160
   aagcggccgg ccgccccgac tctagactat tcctttgccc tcggacgagt gctggggcgt 2220
   cggtttccac tatcggcgag tacttctaca cagccatcgg tccagacggc cgcgcttctg 2280
   cgggcgattt gtgtacgccc gacagtcccg gctccggatc ggacgattgc gtcgcatcga 2340
   ccctgcgccc aagctgcatc atcgaaattg ccgtcaacca agctctgata gagttggtca 2400
   agaccaatgc ggagcatata cgcccggagc cgcggcgatc ctgcaagctc cggatgcctc 2460
   cgctcgaagt agcgcgtctg ctgctccata caagccaacc acggcctcca gaagaagatg 2520
   ttggcgacct cgtattggga atccccgaac atcgcctcgc tccagtcaat gaccgctgtt 2580
   atgcggccat tgtccgtcag gacattgttg gagccgaaat ccgcgtgcac gaggtgccgg 2640
   acttcggggc agtcctcggc ccaaagcatc agctcatcga gagcctgcgc gacggacgca 2700
   ctgacggtgt cgtccatcac agtttgccag tgatacacat ggggatcagc aatcgcgcat 2760
   atgaaatcac gccatgtagt gtattgaccg attccttgcg gtccgaatgg gccgaacccg 2820
   ctcgtctggc taagatcggc cgcagcgatc gcatccatgg cctccgcgac cggctgcaga 2880
   acagcgggca gttcggtttc aggcaggtct tgcaacgtga caccctgtgc acggcgggag 2940
   atgcaatagg tcaggctctc gctgaattcc ccaatgtcaa gcacttccgg aatcgggagc 3000
   gcggccgatg caaagtgccg ataaacataa cgatctttgt agaaaccatc ggcgcagcta 3060
   tttacccgca ggacatatcc acgccctcct acatcgaagc tgaaagcacg agattcttcg 3120
   ccctccgaga gctgcatcag gtcggagacg ctgtcgaact tttcgatcag aaacttctcg 3180
   acagacgtcg cggtgagttc aggctttccg gatctatcca tcatggtggc tttaccaaca 3240
   gtaccggaat gccaagcttt ttgcaaaagc ctaggcctcc aaaaaagcct cctcactact 3300
   tctggaatag ctcagaggcc gaggcggcct cggcctctgc ataaataaaa aaaattagtc 3360
   agcgatgggg cggagaatgg gcggaactgg gcggagttag gggcgggatg ggcggagtta 3420
   ggggcgggac tatggttgct gactaattga gatgcagatc gcagatcctc gggacgctct 3480
   ggccggtgag gcgtgcgcag tcgttgacgc tctagaccgt gcaaaaggag agcctgtaag 3540
   cgggcactct tccgtggtct ggtggataaa ttcgcaaggg tatcatggcg gacgaccggg 3600
   gttcgaaccc cggatccggc cgtccgccgt gatccatgcg gttaccgccc gcgtgtcgaa 3660
   cccaggtgtg cgacgtcaga caacggggga gcgctccttt tggcttcctt ccaggcgcgg 3720
   cggctgctgc gctagctttt ttggccactg gccgcgcgcg gcgtaagcgg ttaggctgga 3780
   aagcgaaagc attaagtggc tcgctccctg tagccggagg gttattttcc aagggttgag 3840
   tcgcaggacc cccggttcga gtctcgggcc ggccggactg cggcgaacgg gggtttgcct 3900
   ccccgtcatg caagaccccg cttgcaaatt cctccggaaa cagggacgag cccctttttt 3960
   gcttttccca gatgcatccg gtgctgcggc agatgcgccc ccctcctcag cagcggcaag 4020
   agcaagagca gcggcagaca tgcagggcac cctccccttc tcctaccgcg tcaggagggg 4080
   caacatccga cgcgtgcatc cataatataa ctgtaccagg ttttggttta ttacatgtga 4140
   ctgacggctt cctatgcgtg ctcagaaaac ggcagttggg cactgcactg cccggtgatg 4200
   gtgccacggt ggctcctgcc gccttctttg atattcactc tgttgtattt catctcttgt 4260
   tgccgatgaa aggatataac agtctctgag gaaatacttg gtatttcttc tgatcagcgt 4320
   ttttataagt aatgttgaat attggataag gctgtgtgtc ctttgtcttg ggagacaaag 4380
   cccacagcag gtggtggttg ggtggtggca gctcagtgac aggagaggtt tttttgcctg 4440
   ttttttttgt tgtttttttt ttttaagtaa ggtgttcttt tttcttagta aaatttctac 4500
   tggactgtat gttttgacag gtcagaaaca tttcttcaaa agaagaacct tttggaaact 4560
   gtacagccct tttctttcat tccctttttg ctttctgtgc caatgccttt ggttctgatt 4620
   gcattatgga aaacgttgat cggaacttga ggtttttatt tatagtgtgg cttgaaagct 4680
   tggatagctg ttgttacatg agatacctta ttaagtttag gccagcttga tgctttattt 4740
   tttttccttt gaagtagtga gcgttctctg gtttttttcc tttgaaactg gcgaggctta 4800
   gatttttcta atgggatttt ttacctgatg atctagttgc atacccaaat gcttgtaaat 4860
   gttttcctag ttaacatgtt gataacttcg gatttacatg ttgtatatac ttgtcatctg 4920
   tgtttctagt aaaaatatat ggcatttata gaaatacgta attcctgatt tccttttttt 4980
   tttatctcta tgctctgtgt gtacaggtca aacagacttc actcctattt ttatttatag 5040
   aattttatat gcagtctgtc gttggttctt gtgttgtaag gatacagcct taaatttcct 5100
   agagcgatgc tcagtaaggc gggttgtcac atgggttcaa atgtaaaacg ggcacgtttg 5160
   ctgctgcctt cccagatcca ggacactaaa ctgcttctgc aactgaggta taaatcgctt 5220
   cagatcccag gaagtgtaga tccacgtgca tattcttaaa gaagaatgaa tactttctaa 5280
   aatatgttgg cataggaagc aagctgcatg gatttatttg ggacttaaat tattttggta 5340
   acggagtgca taggttttaa acacagttgc agcatgctaa cgagtcacag catttatgca 5400
   gaagtgatgc ctgttgcagc tgtttacggc actgccttgc agtgagcatt gcagataggg 5460
   gtggggtgct ttgtgtcgtg ttgggacacg ctgccacaca gccacctccc gaacatatct 5520
   cacctgctgg gtacttttca aaccatctta gcagtagtag atgagttact atgaaacaga 5580
   gaagttcctc agttggatat tctcatggga tgtctttttt cccatgttgg gcaaagtatg 5640
   ataaagcatc tctatttgta aattatgcac ttgttagttc ctgaatcctt tctatagcac 5700
   cacttattgc agcaggtgta ggctctggtg tggcctgtgt ctgtgcttca atcttttaag 5760
   cttacgcgtt gacattgatt attgactagt tattaatagt aatcaattac ggggtcatta 5820
   gttcatagcc catatatgga gttccgcgtt acataactta cggtaaatgg cccgcctggc 5880
   tgaccgccca acgacccccg cccattgacg tcaataatga cgtatgttcc catagtaacg 5940
   ccaataggga ctttccattg acgtcaatgg gtggagtatt tacggtaaac tgcccacttg 6000
   gcagtacatc aagtgtatca tatgccaagt acgcccccta ttgacgtcaa tgacggtaaa 6060
   tggcccgcct ggcattatgc ccagtacatg accttatggg actttcctac ttggcagtac 6120
   atctacgtat tagtcatcgc tattaccatg gtgatgcggt tttggcagta catcaatggg 6180
   cgtggatagc ggtttgactc acggggattt ccaagtctcc accccattga cgtcaatggg 6240
   agtttgtttt ggcaccaaaa tcaacgggac tttccaaaat gtcgtaacaa ctccgcccca 6300
   ttgacgcaaa tgggcggtag gcgtgtacgg tgggaggtct atataagcag agctggttta 6360
   gtgaaccgtc agatcctctt ccgcatcgct gtctgcgagg gccagctgtt ggggtgagta 6420
   ctccctctca aaagcgggca tgacttctgc gctaagaatg tcagtttcca aaaacgagga 6480
   ggatttgata ttcactggcc cgcggtgatg cctttgaggg tggccgcgtc catctggtca 6540
   gaaaagacaa tctttttgtt gtcaagcttc cttgatgatg tcatacttat cctgtccctt 6600
   ttttttccac agctcgcggt tgaggacaaa ctcttcgcgg tctttccagt actcttggat 6660
   cggaaacccg tcggcctccg aacggtactc cgccaccgag ggacctgagc gagtccgcat 6720
   cgaccggatc ggaaaacctc tcgacgtacc aattaattcg ctgtctgcga gggccagctg 6780
   ttggggtgag tactccctct caaaagcggg catgacttct gcgctaagat tgtcagtttc 6840
   caaaaacgag gaggatttga tattcacctg gcccgcggtg atgcctttga gggtggccgc 6900
   gtccatctgg tcagaaaaga caatcttttt gttgtcaagc ttgaggtgtg gcaggcttga 6960
   gatctggcca tacacttgag tgacaatgac atccactttg cctttctctc cacaggtgtc 7020
   cactcccagg tccaactgca ggtcgagcat gcatctaggg cgcccgcact agaggctcga 7080
   gccaccatgg actactaccg gaagtacgcc gccatcttcc tggtgaccct gtccgtgttc 7140
   ctgcacgtgc tgcactctgc ccctgacgtg caggactgcc ctgagtgcac cctgcaggaa 7200
   aacccattct tcagccagcc tggcgcccct atcctgcagt gcatgggctg ctgcttctcc 7260
   agagcctacc ctacccctct gcggtccaag aaaaccatgc tggtgcagaa aaacgtgacc 7320
   tccgagtcta cctgctgcgt ggccaagtcc tacaacagag tgaccgtgat gggcggcttc 7380
   aaggtggaga accacaccgc ctgccactgc tctacctgct actaccacaa gtcctgatga 7440
   gcggccgcgc cgcactagag gaattccgcc cctctccccc cccccctaac gttactggcc 7500
   gaagccgctt ggaataaggc cggtgtgcgt ttgtctatat gttattttcc accatattgc 7560
   cgtcttttgg caatgtgagg gcccggaaac ctggccctgt cttcttgacg agcattccta 7620
   ggggtctttc ccctctcgcc aaaggaatgc aaggtctgtt gaatgtcgtg aaggaagcag 7680
   ttcctctgga agcttcttga agacaaacaa cgtctgtagc gaccctttgc aggcagcgga 7740
   accccccacc tggcgacagg tgcctctgcg gccaaaagcc acgtgtataa gatacacctg 7800
   caaaggcggc acaaccccag tgccacgttg tgagttggat agttgtggaa agagtcaaat 7860
   ggctctcctc aagcgtattc aacaaggggc tgaaggatgc ccagaaggta ccccattgta 7920
   tgggatctga tctggggcct cggtgcacat gctttacatg tgtttagtcg aggttaaaaa 7980
   aacgtctagg ccccccgaac cacggggacg tggttttcct ttgaaaaaca cgatgataac 8040
   ccgggccacc atgaagaccc tgcagttctt cttcctgttc tgctgctgga aggccatctg 8100
   ctgcaactct tgcgagctga ccaacatcac aatcgccatc gagaaagagg aatgccggtt 8160
   ctgcatctcc atcaacacca cttggtgcgc cggctactgc tacacccgcg acctggtgta 8220
   caaggaccct gcccggccta agatccagaa aacctgcacc ttcaaagaac tggtgtacga 8280
   gacagtgcgg gtgccaggct gcgctcacca cgccgactcc ctgtacacct accctgtggc 8340
   cacccagtgc cactgcggca agtgcgactc cgactccacc gactgtaccg tgcggggcct 8400
   gggcccttct tactgctcct tcggcgagat gaaggaatga tgaccagcgg ccgcccgctg 8460
   atcagcctcg actgtgcctt ctagttgcca gccatctgtt gtttgcccct cccccgtgcc 8520
   ttccttgacc ctggaaggtg ccactcccac tgtcctttcc taataaaatg aggaaattgc 8580
   atcgcattgt ctgagtaggt gtcattctat tctggggggt ggggtggggc aggacagcaa 8640
   gggggaggat tgggaagaca atagcaggca tgctggggat gcggtgggct ctatggcttc 8700
   tgaggcggaa agaaccagct ggggctctag ggggtatccc cacggagctc gcatccataa 8760
   tataactgta ccaggttttg gtttattaca tgtgactgac ggcttcctat gcgtgctcag 8820
   aaaacggcag ttgggcactg cactgcccgg tgatggtgcc acggtggctc ctgccgcctt 8880
   ctttgatatt cactctgttg tatttcatct cttgttgccg atgaaaggat ataacagtct 8940
   ctgaggaaat acttggtatt tcttctgatc agcgttttta taagtaatgt tgaatattgg 9000
   ataaggctgt gtgtcctttg tcttgggaga caaagcccac agcaggtggt ggttgggtgg 9060
   tggcagctca gtgacaggag aggttttttt gcctgttttt tttgttgttt ttttttttta 9120
   agtaaggtgt tcttttttct tagtaaaatt tctactggac tgtatgtttt gacaggtcag 9180
   aaacatttct tcaaaagaag aaccttttgg aaactgtaca gcccttttct ttcattccct 9240
   ttttgctttc tgtgccaatg cctttggttc tgattgcatt atggaaaacg ttgatcggaa 9300
   cttgaggttt ttatttatag tgtggcttga aagcttggat agctgttgtt acatgagata 9360
   ccttattaag tttaggccag cttgatgctt tatttttttt cctttgaagt agtgagcgtt 9420
   ctctggtttt tttcctttga aactggcgag gcttagattt ttctaatggg attttttacc 9480
   tgatgatcta gttgcatacc caaatgcttg taaatgtttt cctagttaac atgttgataa 9540
   cttcggattt acatgttgta tatacttgtc atctgtgttt ctagtaaaaa tatatggcat 9600
   ttatagaaat acgtaattcc tgatttcctt ttttttttat ctctatgctc tgtgtgtaca 9660
   ggtcaaacag acttcactcc tatttttatt tatagaattt tatatgcagt ctgtcgttgg 9720
   ttcttgtgtt gtaaggatac agccttaaat ttcctagagc gatgctcagt aaggcgggtt 9780
   gtcacatggg ttcaaatgta aaacgggcac gtttgctgct gccttcccag atccaggaca 9840
   ctaaactgct tctgcaactg aggtataaat cgcttcagat cccaggaagt gtagatccac 9900
   gtgcatattc ttaaagaaga atgaatactt tctaaaatat gttggcatag gaagcaagct 9960
   gcatggattt atttgggact taaattattt tggtaacgga gtgcataggt tttaaacaca 10020
   gttgcagcat gctaacgagt cacagcattt atgcagaagt gatgcctgtt gcagctgttt 10080
   acggcactgc cttgcagtga gcattgcaga taggggtggg gtgctttgtg tcgtgttggg 10140
   acacgctgcc acacagccac ctcccgaaca tatctcacct gctgggtact tttcaaacca 10200
   tcttagcagt agtagatgag ttactatgaa acagagaagt tcctcagttg gatattctca 10260
   tgggatgtct tttttcccat gttgggcaaa gtatgataaa gcatctctat ttgtaaatta 10320
   tgcacttgtt agttcctgaa tcctttctat agcaccactt attgcagcag gtgtaggctc 10380
   tggtgtggcc tgtgtctgtg cttcaatctt ttaagcttga gctcatggcg cgcctaggcc 10440
   ttgacggcct tccttcaatt cgccctatag tgagtcgtat tacgtcgcgc tcactggccg 10500
   tcgttttaca acgtcgtgac tgggaaaacc ctggcgttac ccaacttaat cgccttgcag 10560
   cacatccccc tttcgccagc tggcgtaata gcgaagaggc ccgcaccgaa acgcccttcc 10620
   caacagttgc gcagcctgaa tggcgaatgg gagcgccctg tagcggccac tcaaccctat 10680
   ctcggtctat tcttttgatt tataagggat tttgccgatt tcggcctatt ggttaaaaaa 10740
   tgagctgatt taacaaaaat ttaacgcgaa ttttaacaaa atattaacgc ttacaattta 10800
   g 10801
<210> 17
   <211> 10710
   <212> DNA
   <213> Artificial
<220>
   <223> pZRC III- TNK- Puro
<400> 17
   gtggcacttt tcggggaaat gtgcgcggaa cccctatttg tttatttttc taaatacatt 60
   caaatatgta tccgctcatg agacaataac cctgataaat gcttcaataa tattgaaaaa 120
   ggaagagtat gattgaacag gatggcctgc atgcgggtag cccggcagcg tgggtggaac 180
   gtctgtttgg ctatgattgg gcgcagcaga ccattggctg ctctgatgcg gcggtgtttc 240
   gtctgagcgc gcagggtcgt ccggtgctgt ttgtgaaaac cgatctgagc ggtgcgctga 300
   acgagctgca ggatgaagcg gcgcgtctga gctggctggc caccaccggt gttccgtgtg 360
   cggcggtgct ggatgtggtg accgaagcgg gccgtgattg gctgctgctg ggcgaagtgc 420
   cgggtcagga tctgctgtct agccatctgg cgccggcaga aaaagtgagc attatggcgg 480
   atgccatgcg tcgtctgcat accctggacc cggcgacctg tccgtttgat catcaggcga 540
   aacatcgtat tgaacgtgcg cgtacccgta tggaagcggg cctggtggat caggatgatc 600
   tggatgaaga acatcagggc ctggcaccgg cagagctgtt tgcgcgtctg aaagcgagca 660
   tgccggatgg cgaagatctg gtggtgaccc atggtgatgc gtgcctgccg aacattatgg 720
   tggaaaatgg ccgttttagc ggctttattg attgcggccg tctgggcgtg gcggatcgtt 780
   atcaggatat tgcgctggcc acccgtgata ttgcggaaga actgggcggc gaatgggcgg 840
   atcgttttct ggtgctgtat ggcattgcgg caccggatag ccagcgtatt gcgttttatc 900
   gtctgctgga tgaatttttc taataactgt cagaccaagt ttactcatat atactttaga 960
   ttgatttaaa acttcatttt taatttaaaa ggatctaggt gaagatcctt tttgataatc 1020
   tcatgaccaa aatcccttaa cgtgagtttt cgttccactg agcgtcagac cccgtagaaa 1080
   agatcaaagg atcttcttga gatccttttt ttctgcgcgt aatctgctgc ttgcaaacaa 1140
   aaaaaccacc gctaccagcg gtggtttgtt tgccggatca agagctacca actctttttc 1200
   cgaaggtaac tggcttcagc agagcgcaga taccaaatac tgttcttcta gtgtagccgt 1260
   agttaggcca ccacttcaag aactctgtag caccgcctac atacctcgct ctgctaatcc 1320
   tgttaccagt ggctgctgcc agtggcgata agtcgtgtct taccgggttg gactcaagac 1380
   gatagttacc ggataaggcg cagcggtcgg gctgaacggg gggttcgtgc acacagccca 1440
   gcttggagcg aacgacctac accgaactga gatacctaca gcgtgagcta tgagaaagcg 1500
   ccacgcttcc cgaagggaga aaggcggaca ggtatccggt aagcggcagg gtcggaacag 1560
   gagagcgcac gagggagctt ccagggggaa acgcctggta tctttatagt cctgtcgggt 1620
   ttcgccacct ctgacttgag cgtcgatttt tgtgatgctc gtcagggggg cggagcctat 1680
   ggaaaaacgc cagcaacgcg gcctttttac ggttcctggc cttttgctgg ccttttgctc 1740
   attaggcacc ccaggcttta cccgaacgac cgagcgcagc gagtcagtga gcgaggaagc 1800
   ggagagcgcc caatacgcaa ggaaacagct atgaccatgt taatgcagct ggcacgacag 1860
   gtttcccgac tggaaagcgg gcagtgaaag gaaggcccat gaggccagtt aattaagagg 1920
   taccggatct gcgatctgca tctcaattag tcagcaacca tagtcccgcc cctaactccg 1980
   cccatcccgc ccctaactcc gcccagttcc gcccattctc cgccccatcg ctgactaatt 2040
   ttttttattt atgcagaggc cgaggccgcc tcggcctctg agctattcca gaagtagtga 2100
   ggaggctttt ttggaggcct aggcttttgc aaaaagcttg gcattccggt actgttggta 2160
   aagccaccat gaccgagtac aagcccacgg tgcgcctcgc cacccgcgac gacgtccccc 2220
   gggccgtacg caccctcgcc gccgcgttcg ccgactaccc cgccacgcgc cacaccgtcg 2280
   acccggaccg ccacatcgag cgggtcaccg agctgcaaga actcttcctc acgcgcgtcg 2340
   ggctcgacat cggcaaggtg tgggtcgcgg acgacggcgc cgcggtggcg gtctggacca 2400
   cgccggagag cgtcgaagcg ggggcggtgt tcgccgagat cggcccgcgc atggccgagt 2460
   tgagcggttc ccggctggcc gcgcagcaac agatggaagg cctcctggcg ccgcaccggc 2520
   ccaaggagcc cgcgtggttc ctggccaccg tcggcgtctc gcccgaccac cagggcaagg 2580
   gtctgggcag cgccgtcgtg ctccccggag tggaggcggc cgagcgcgcc ggggtgcccg 2640
   ccttcctgga gacctccgcg ccccgcaacc tccccttcta cgagcggctc ggcttcaccg 2700
   tcaccgccga cgtcgaggtg cccgaaggac cgcgcacctg gtgcatgacc cgcaagcccg 2760
   gtgcctgatc tagctagagt cggggcggcc ggccgcttcg agcagacatg ataagataca 2820
   ttgatgagtt tggacaaacc acaactagaa tgcagtgaaa aaaatgcttt atttgtgaaa 2880
   tttgtgatgc tattgcttta tttgtaacca ttataagctg caataaacaa gttaacaaca 2940
   acaattgcat tcattttatg tttcaggttc agggggaggt gtgggaggtt ttttaaagca 3000
   agtaaaacct ctacaaatgt ggtaaaatcg ataaggatcc tcgggacgct ctggccggtg 3060
   aggcgtgcgc agtcgttgac gctctagacc gtgcaaaagg agagcctgta agcgggcact 3120
   cttccgtggt ctggtggata aattcgcaag ggtatcatgg cggacgaccg gggttcgaac 3180
   cccagatccg gccgtccgcc gtgatccatg cggttaccgc ccgcgtgtcg aacccaggtg 3240
   tgcgacgtca gacaacgggg gagcgctcct tttggcttcc ttccaggcgc ggcggctgct 3300
   gcgctagctt ttttggccac tggccgcgcg cggcgtaagc ggttaggctg gaaagcgaaa 3360
   gcattaagtg gctcgctccc tgtagccgga gggttatttt ccaagggttg agtcgcagga 3420
   cccccggttc gagtctcggg ccggccggac tgcggcgaac gggggtttgc ctccccgtca 3480
   tgcaagaccc cgcttgcaaa ttcctccgga aacagggacg agcccctttt ttgcttttcc 3540
   cagatgcatc cggtgctgcg gcagatgcgc ccccctcctc agcagcggca agagcaagag 3600
   cagcggcaga catgcagggc accctcccct tctcctaccg cgtcaggagg ggcaacatcc 3660
   gacgcgtgca tccataatat aactgtacca ggttttggtt tattacatgt gactgacggc 3720
   ttcctatgcg tgctcagaaa acggcagttg ggcactgcac tgcccggtga tggtgccacg 3780
   gtggctcctg ccgccttctt tgatattcac tctgttgtat ttcatctctt gttgccgatg 3840
   aaaggatata acagtctctg aggaaatact tggtatttct tctgatcagc gtttttataa 3900
   gtaatgttga atattggata aggctgtgtg tcctttgtct tgggagacaa agcccacagc 3960
   aggtggtggt tgggtggtgg cagctcagtg acaggagagg tttttttgcc tgtttttttt 4020
   gttgtttttt ttttttaagt aaggtgttct tttttcttag taaaatttct actggactgt 4080
   atgttttgac aggtcagaaa catttcttca aaagaagaac cttttggaaa ctgtacagcc 4140
   cttttctttc attccctttt tgctttctgt gccaatgcct ttggttctga ttgcattatg 4200
   gaaaacgttg atcggaactt gaggttttta tttatagtgt ggcttgaaag cttggatagc 4260
   tgttgttaca tgagatacct tattaagttt aggccagctt gatgctttat tttttttcct 4320
   ttgaagtagt gagcgttctc tggttttttt cctttgaaac tggcgaggct tagatttttc 4380
   taatgggatt ttttacctga tgatctagtt gcatacccaa atgcttgtaa atgttttcct 4440
   agttaacatg ttgataactt cggatttaca tgttgtatat acttgtcatc tgtgtttcta 4500
   gtaaaaatat atggcattta tagaaatacg taattcctga tttccttttt tttttatctc 4560
   tatgctctgt gtgtacaggt caaacagact tcactcctat ttttatttat agaattttat 4620
   atgcagtctg tcgttggttc ttgtgttgta aggatacagc cttaaatttc ctagagcgat 4680
   gctcagtaag gcgggttgtc acatgggttc aaatgtaaaa cgggcacgtt tgctgctgcc 4740
   ttcccagatc caggacacta aactgcttct gcaactgagg tataaatcgc ttcagatccc 4800
   aggaagtgta gatccacgtg catattctta aagaagaatg aatactttct aaaatatgtt 4860
   ggcataggaa gcaagctgca tggatttatt tgggacttaa attattttgg taacggagtg 4920
   cataggtttt aaacacagtt gcagcatgct aacgagtcac agcatttatg cagaagtgat 4980
   gcctgttgca gctgtttacg gcactgcctt gcagtgagca ttgcagatag gggtggggtg 5040
   ctttgtgtcg tgttgggaca cgctgccaca cagccacctc ccgaacatat ctcacctgct 5100
   gggtactttt caaaccatct tagcagtagt agatgagtta ctatgaaaca gagaagttcc 5160
   tcagttggat attctcatgg gatgtctttt ttcccatgtt gggcaaagta tgataaagca 5220
   tctctatttg taaattatgc acttgttagt tcctgaatcc tttctatagc accacttatt 5280
   gcagcaggtg taggctctgg tgtggcctgt gtctgtgctt caatctttta agcttacgcg 5340
   ttgacattga ttattgacta gttattaata gtaatcaatt acggggtcat tagttcatag 5400
   cccatatatg gagttccgcg ttacataact tacggtaaat ggcccgcctg gctgaccgcc 5460
   caacgacccc cgcccattga cgtcaataat gacgtatgtt cccatagtaa cgccaatagg 5520
   gactttccat tgacgtcaat gggtggagta tttacggtaa actgcccact tggcagtaca 5580
   tcaagtgtat catatgccaa gtacgccccc tattgacgtc aatgacggta aatggcccgc 5640
   ctggcattat gcccagtaca tgaccttatg ggactttcct acttggcagt acatctacgt 5700
   attagtcatc gctattacca tggtgatgcg gttttggcag tacatcaatg ggcgtggata 5760
   gcggtttgac tcacggggat ttccaagtct ccaccccatt gacgtcaatg ggagtttgtt 5820
   ttggcaccaa aatcaacggg actttccaaa atgtcgtaac aactccgccc cattgacgca 5880
   aatgggcggt aggcgtgtac ggtgggaggt ctatataagc agagctggtt tagtgaaccg 5940
   tcagatcctc ttccgcatcg ctgtctgcga gggccagctg ttggggtgag tactccctct 6000
   caaaagcggg catgacttct gcgctaagaa tgtcagtttc caaaaacgag gaggatttga 6060
   tattcactgg cccgcggtga tgcctttgag ggtggccgcg tccatctggt cagaaaagac 6120
   aatctttttg ttgtcaagct tccttgatga tgtcatactt atcctgtccc ttttttttcc 6180
   acagctcgcg gttgaggaca aactcttcgc ggtctttcca gtactcttgg atcggaaacc 6240
   cgtcggcctc cgaacggtac tccgccaccg agggacctga gcgagtccgc atcgaccgga 6300
   tcggaaaacc tctcgacgta ccaattaatt cgctgtctgc gagggccagc tgttggggtg 6360
   agtactccct ctcaaaagcg ggcatgactt ctgcgctaag attgtcagtt tccaaaaacg 6420
   aggaggattt gatattcacc tggcccgcgg tgatgccttt gagggtggcc gcgtccatct 6480
   ggtcagaaaa gacaatcttt ttgttgtcaa gcttgaggtg tggcaggctt gagatctggc 6540
   catacacttg agtgacaatg acatccactt tgcctttctc tccacaggtg tccactccca 6600
   ggtccaactg caggtcgagc atgcatctag ggcgcccgca ctagaggctc gaggaattcc 6660
   accatggacg ccatgaagcg gggactgtgc tgtgtgctgc tgctgtgtgg cgccgtgttc 6720
   gtgtccccta gccaggaaat ccacgcccgg ttcagaaggg gcgccaggtc ctaccaagtt 6780
   atctgccggg acgaaaagac ccagatgatc taccagcagc accagtcctg gctgcggccc 6840
   gtgctgcggt ccaaccgggt ggagtactgc tggtgcaact ccggcagagc ccagtgccac 6900
   tccgtgcctg tgaagtcctg ctccgagcct agatgcttca acggcggcac ctgtcagcag 6960
   gccctgtact tctccgactt cgtgtgccag tgccctgagg gcttcgccgg caagtgctgc 7020
   gagatcgaca cccgggccac ctgttacgag gaccagggca tctcctaccg gggcaactgg 7080
   tctaccgccg agtctggcgc cgagtgcacc aactggcagt cctccgccct ggcccagaag 7140
   ccttactctg gcagacggcc tgacgccatc agactgggcc tgggcaacca caactactgc 7200
   cggaaccctg accgggactc caagccttgg tgctacgtgt tcaaggccgg caagtactcc 7260
   tccgagttct gctccacccc tgcctgctct gagggcaact ccgactgcta cttcggcaac 7320
   ggctccgcct acagaggcac ccactccctg accgagtccg gcgcctcttg cctgccttgg 7380
   aactccatga tcctgatcgg caaggtgtac accgcccaga acccttccgc tcaggccctg 7440
   ggactcggaa agcacaatta ttgtcgcaat cccgacggcg acgccaaacc ttggtgtcac 7500
   gtgctgaaga accggcggct gacatgggaa tactgcgacg tgccttcctg ctctacctgc 7560
   ggcctgcggc agtactccca gcctcagttc cggatcaagg gcggcctgtt cgccgatatc 7620
   gcctcccacc cttggcaggc cgccatcttc gccgctgctg ccgcttctcc tggcgagaga 7680
   ttcctgtgcg gcggcatcct gatctccagc tgctggattc tgtctgccgc ccactgcttc 7740
   caggaacggt tccctcctca ccacctgacc gtgatcctgg gccggaccta cagagtcgtg 7800
   cccggcgagg aagaacagaa attcgaggtg gagaagtata tcgtgcacaa agagttcgac 7860
   gacgacacct acgacaacga tatcgccctg ctgcagctga agtccgactc ctccagatgc 7920
   gcccaggaat cctccgtcgt tcggaccgtg tgtctgccac ctgccgacct gcagctgcct 7980
   gactggaccg agtgcgagct gtccggctac ggcaagcacg aggccctgtc ccccttctac 8040
   tccgagcggc tgaaagaagc tcatgtacgg ctgtacccct ctagccggtg cacctcccag 8100
   catctgctga accggaccgt gaccgacaac atgctgtgtg ccggcgacac cagatctggc 8160
   ggccctcagg ccaacctgca cgacgcctgc cagggcgata gtggcggacc tctcgtgtgc 8220
   ctcaacgacg gcaggatgac cctcgtgggc atcatctctt ggggcctggg ctgtggccag 8280
   aaagacgtgc ctggcgtgta caccaaagtg accaactacc tggactggat cagggacaac 8340
   atgcggcctt gatgagcggc cgcccgctga tcagcctcga ctgtgccttc tagttgccag 8400
   ccatctgttg tttgcccctc ccccgtgcct tccttgaccc tggaaggtgc cactcccact 8460
   gtcctttcct aataaaatga ggaaattgca tcgcattgtc tgagtaggtg tcattctatt 8520
   ctggggggtg gggtggggca ggacagcaag ggggaggatt gggaagacaa tagcaggcat 8580
   gctggggatg cggtgggctc tatggcttct gaggcggaaa gaaccagctg gggctctagg 8640
   gggtatcccc acggagctcg catccataat ataactgtac caggttttgg tttattacat 8700
   gtgactgacg gcttcctatg cgtgctcaga aaacggcagt tgggcactgc actgcccggt 8760
   gatggtgcca cggtggctcc tgccgccttc tttgatattc actctgttgt atttcatctc 8820
   ttgttgccga tgaaaggata taacagtctc tgaggaaata cttggtattt cttctgatca 8880
   gcgtttttat aagtaatgtt gaatattgga taaggctgtg tgtcctttgt cttgggagac 8940
   aaagcccaca gcaggtggtg gttgggtggt ggcagctcag tgacaggaga ggtttttttg 9000
   cctgtttttt ttgttgtttt ttttttttaa gtaaggtgtt cttttttctt agtaaaattt 9060
   ctactggact gtatgttttg acaggtcaga aacatttctt caaaagaaga accttttgga 9120
   aactgtacag cccttttctt tcattccctt tttgctttct gtgccaatgc ctttggttct 9180
   gattgcatta tggaaaacgt tgatcggaac ttgaggtttt tatttatagt gtggcttgaa 9240
   agcttggata gctgttgtta catgagatac cttattaagt ttaggccagc ttgatgcttt 9300
   attttttttc ctttgaagta gtgagcgttc tctggttttt ttcctttgaa actggcgagg 9360
   cttagatttt tctaatggga ttttttacct gatgatctag ttgcataccc aaatgcttgt 9420
   aaatgttttc ctagttaaca tgttgataac ttcggattta catgttgtat atacttgtca 9480
   tctgtgtttc tagtaaaaat atatggcatt tatagaaata cgtaattcct gatttccttt 9540
   tttttttatc tctatgctct gtgtgtacag gtcaaacaga cttcactcct atttttattt 9600
   atagaatttt atatgcagtc tgtcgttggt tcttgtgttg taaggataca gccttaaatt 9660
   tcctagagcg atgctcagta aggcgggttg tcacatgggt tcaaatgtaa aacgggcacg 9720
   tttgctgctg ccttcccaga tccaggacac taaactgctt ctgcaactga ggtataaatc 9780
   gcttcagatc ccaggaagtg tagatccacg tgcatattct taaagaagaa tgaatacttt 9840
   ctaaaatatg ttggcatagg aagcaagctg catggattta tttgggactt aaattatttt 9900
   ggtaacggag tgcataggtt ttaaacacag ttgcagcatg ctaacgagtc acagcattta 9960
   tgcagaagtg atgcctgttg cagctgttta cggcactgcc ttgcagtgag cattgcagat 10020
   aggggtgggg tgctttgtgt cgtgttggga cacgctgcca cacagccacc tcccgaacat 10080
   atctcacctg ctgggtactt ttcaaaccat cttagcagta gtagatgagt tactatgaaa 10140
   cagagaagtt cctcagttgg atattctcat gggatgtctt ttttcccatg ttgggcaaag 10200
   tatgataaag catctctatt tgtaaattat gcacttgtta gttcctgaat cctttctata 10260
   gcaccactta ttgcagcagg tgtaggctct ggtgtggcct gtgtctgtgc ttcaatcttt 10320
   taagcttgag ctcatggcgc gcctaggcct tgacggcctt ccttcaattc gccctatagt 10380
   gagtcgtatt acgtcgcgct cactggccgt cgttttacaa cgtcgtgact gggaaaaccc 10440
   tggcgttacc caacttaatc gccttgcagc acatccccct ttcgccagct ggcgtaatag 10500
   cgaagaggcc cgcaccgaaa cgcccttccc aacagttgcg cagcctgaat ggcgaatggg 10560
   agcgccctgt agcggccact caaccctatc tcggtctatt cttttgattt ataagggatt 10620
   ttgccgattt cggcctattg gttaaaaaat gagctgattt aacaaaaatt taacgcgaat 10680
   tttaacaaaa tattaacgct tacaatttag 10710
<210> 18
   <211> 9595
   <212> DNA
   <213> Artificial
<220>
   <223> pZRC III- DARBE- Puro
<400> 18
   gtggcacttt tcggggaaat gtgcgcggaa cccctatttg tttatttttc taaatacatt 60
   caaatatgta tccgctcatg agacaataac cctgataaat gcttcaataa tattgaaaaa 120
   ggaagagtat gattgaacag gatggcctgc atgcgggtag cccggcagcg tgggtggaac 180
   gtctgtttgg ctatgattgg gcgcagcaga ccattggctg ctctgatgcg gcggtgtttc 240
   gtctgagcgc gcagggtcgt ccggtgctgt ttgtgaaaac cgatctgagc ggtgcgctga 300
   acgagctgca ggatgaagcg gcgcgtctga gctggctggc caccaccggt gttccgtgtg 360
   cggcggtgct ggatgtggtg accgaagcgg gccgtgattg gctgctgctg ggcgaagtgc 420
   cgggtcagga tctgctgtct agccatctgg cgccggcaga aaaagtgagc attatggcgg 480
   atgccatgcg tcgtctgcat accctggacc cggcgacctg tccgtttgat catcaggcga 540
   aacatcgtat tgaacgtgcg cgtacccgta tggaagcggg cctggtggat caggatgatc 600
   tggatgaaga acatcagggc ctggcaccgg cagagctgtt tgcgcgtctg aaagcgagca 660
   tgccggatgg cgaagatctg gtggtgaccc atggtgatgc gtgcctgccg aacattatgg 720
   tggaaaatgg ccgttttagc ggctttattg attgcggccg tctgggcgtg gcggatcgtt 780
   atcaggatat tgcgctggcc acccgtgata ttgcggaaga actgggcggc gaatgggcgg 840
   atcgttttct ggtgctgtat ggcattgcgg caccggatag ccagcgtatt gcgttttatc 900
   gtctgctgga tgaatttttc taataactgt cagaccaagt ttactcatat atactttaga 960
   ttgatttaaa acttcatttt taatttaaaa ggatctaggt gaagatcctt tttgataatc 1020
   tcatgaccaa aatcccttaa cgtgagtttt cgttccactg agcgtcagac cccgtagaaa 1080
   agatcaaagg atcttcttga gatccttttt ttctgcgcgt aatctgctgc ttgcaaacaa 1140
   aaaaaccacc gctaccagcg gtggtttgtt tgccggatca agagctacca actctttttc 1200
   cgaaggtaac tggcttcagc agagcgcaga taccaaatac tgttcttcta gtgtagccgt 1260
   agttaggcca ccacttcaag aactctgtag caccgcctac atacctcgct ctgctaatcc 1320
   tgttaccagt ggctgctgcc agtggcgata agtcgtgtct taccgggttg gactcaagac 1380
   gatagttacc ggataaggcg cagcggtcgg gctgaacggg gggttcgtgc acacagccca 1440
   gcttggagcg aacgacctac accgaactga gatacctaca gcgtgagcta tgagaaagcg 1500
   ccacgcttcc cgaagggaga aaggcggaca ggtatccggt aagcggcagg gtcggaacag 1560
   gagagcgcac gagggagctt ccagggggaa acgcctggta tctttatagt cctgtcgggt 1620
   ttcgccacct ctgacttgag cgtcgatttt tgtgatgctc gtcagggggg cggagcctat 1680
   ggaaaaacgc cagcaacgcg gcctttttac ggttcctggc cttttgctgg ccttttgctc 1740
   attaggcacc ccaggcttta cccgaacgac cgagcgcagc gagtcagtga gcgaggaagc 1800
   ggagagcgcc caatacgcaa ggaaacagct atgaccatgt taatgcagct ggcacgacag 1860
   gtttcccgac tggaaagcgg gcagtgaaag gaaggcccat gaggccagtt aattaagagg 1920
   taccggatct gcgatctgca tctcaattag tcagcaacca tagtcccgcc cctaactccg 1980
   cccatcccgc ccctaactcc gcccagttcc gcccattctc cgccccatcg ctgactaatt 2040
   ttttttattt atgcagaggc cgaggccgcc tcggcctctg agctattcca gaagtagtga 2100
   ggaggctttt ttggaggcct aggcttttgc aaaaagcttg gcattccggt actgttggta 2160
   aagccaccat gaccgagtac aagcccacgg tgcgcctcgc cacccgcgac gacgtccccc 2220
   gggccgtacg caccctcgcc gccgcgttcg ccgactaccc cgccacgcgc cacaccgtcg 2280
   acccggaccg ccacatcgag cgggtcaccg agctgcaaga actcttcctc acgcgcgtcg 2340
   ggctcgacat cggcaaggtg tgggtcgcgg acgacggcgc cgcggtggcg gtctggacca 2400
   cgccggagag cgtcgaagcg ggggcggtgt tcgccgagat cggcccgcgc atggccgagt 2460
   tgagcggttc ccggctggcc gcgcagcaac agatggaagg cctcctggcg ccgcaccggc 2520
   ccaaggagcc cgcgtggttc ctggccaccg tcggcgtctc gcccgaccac cagggcaagg 2580
   gtctgggcag cgccgtcgtg ctccccggag tggaggcggc cgagcgcgcc ggggtgcccg 2640
   ccttcctgga gacctccgcg ccccgcaacc tccccttcta cgagcggctc ggcttcaccg 2700
   tcaccgccga cgtcgaggtg cccgaaggac cgcgcacctg gtgcatgacc cgcaagcccg 2760
   gtgcctgatc tagctagagt cggggcggcc ggccgcttcg agcagacatg ataagataca 2820
   ttgatgagtt tggacaaacc acaactagaa tgcagtgaaa aaaatgcttt atttgtgaaa 2880
   tttgtgatgc tattgcttta tttgtaacca ttataagctg caataaacaa gttaacaaca 2940
   acaattgcat tcattttatg tttcaggttc agggggaggt gtgggaggtt ttttaaagca 3000
   agtaaaacct ctacaaatgt ggtaaaatcg ataaggatcc tcgggacgct ctggccggtg 3060
   aggcgtgcgc agtcgttgac gctctagacc gtgcaaaagg agagcctgta agcgggcact 3120
   cttccgtggt ctggtggata aattcgcaag ggtatcatgg cggacgaccg gggttcgaac 3180
   cccagatccg gccgtccgcc gtgatccatg cggttaccgc ccgcgtgtcg aacccaggtg 3240
   tgcgacgtca gacaacgggg gagcgctcct tttggcttcc ttccaggcgc ggcggctgct 3300
   gcgctagctt ttttggccac tggccgcgcg cggcgtaagc ggttaggctg gaaagcgaaa 3360
   gcattaagtg gctcgctccc tgtagccgga gggttatttt ccaagggttg agtcgcagga 3420
   cccccggttc gagtctcggg ccggccggac tgcggcgaac gggggtttgc ctccccgtca 3480
   tgcaagaccc cgcttgcaaa ttcctccgga aacagggacg agcccctttt ttgcttttcc 3540
   cagatgcatc cggtgctgcg gcagatgcgc ccccctcctc agcagcggca agagcaagag 3600
   cagcggcaga catgcagggc accctcccct tctcctaccg cgtcaggagg ggcaacatcc 3660
   gacgcgtgca tccataatat aactgtacca ggttttggtt tattacatgt gactgacggc 3720
   ttcctatgcg tgctcagaaa acggcagttg ggcactgcac tgcccggtga tggtgccacg 3780
   gtggctcctg ccgccttctt tgatattcac tctgttgtat ttcatctctt gttgccgatg 3840
   aaaggatata acagtctctg aggaaatact tggtatttct tctgatcagc gtttttataa 3900
   gtaatgttga atattggata aggctgtgtg tcctttgtct tgggagacaa agcccacagc 3960
   aggtggtggt tgggtggtgg cagctcagtg acaggagagg tttttttgcc tgtttttttt 4020
   gttgtttttt ttttttaagt aaggtgttct tttttcttag taaaatttct actggactgt 4080
   atgttttgac aggtcagaaa catttcttca aaagaagaac cttttggaaa ctgtacagcc 4140
   cttttctttc attccctttt tgctttctgt gccaatgcct ttggttctga ttgcattatg 4200
   gaaaacgttg atcggaactt gaggttttta tttatagtgt ggcttgaaag cttggatagc 4260
   tgttgttaca tgagatacct tattaagttt aggccagctt gatgctttat tttttttcct 4320
   ttgaagtagt gagcgttctc tggttttttt cctttgaaac tggcgaggct tagatttttc 4380
   taatgggatt ttttacctga tgatctagtt gcatacccaa atgcttgtaa atgttttcct 4440
   agttaacatg ttgataactt cggatttaca tgttgtatat acttgtcatc tgtgtttcta 4500
   gtaaaaatat atggcattta tagaaatacg taattcctga tttccttttt tttttatctc 4560
   tatgctctgt gtgtacaggt caaacagact tcactcctat ttttatttat agaattttat 4620
   atgcagtctg tcgttggttc ttgtgttgta aggatacagc cttaaatttc ctagagcgat 4680
   gctcagtaag gcgggttgtc acatgggttc aaatgtaaaa cgggcacgtt tgctgctgcc 4740
   ttcccagatc caggacacta aactgcttct gcaactgagg tataaatcgc ttcagatccc 4800
   aggaagtgta gatccacgtg catattctta aagaagaatg aatactttct aaaatatgtt 4860
   ggcataggaa gcaagctgca tggatttatt tgggacttaa attattttgg taacggagtg 4920
   cataggtttt aaacacagtt gcagcatgct aacgagtcac agcatttatg cagaagtgat 4980
   gcctgttgca gctgtttacg gcactgcctt gcagtgagca ttgcagatag gggtggggtg 5040
   ctttgtgtcg tgttgggaca cgctgccaca cagccacctc ccgaacatat ctcacctgct 5100
   gggtactttt caaaccatct tagcagtagt agatgagtta ctatgaaaca gagaagttcc 5160
   tcagttggat attctcatgg gatgtctttt ttcccatgtt gggcaaagta tgataaagca 5220
   tctctatttg taaattatgc acttgttagt tcctgaatcc tttctatagc accacttatt 5280
   gcagcaggtg taggctctgg tgtggcctgt gtctgtgctt caatctttta agcttacgcg 5340
   ttgacattga ttattgacta gttattaata gtaatcaatt acggggtcat tagttcatag 5400
   cccatatatg gagttccgcg ttacataact tacggtaaat ggcccgcctg gctgaccgcc 5460
   caacgacccc cgcccattga cgtcaataat gacgtatgtt cccatagtaa cgccaatagg 5520
   gactttccat tgacgtcaat gggtggagta tttacggtaa actgcccact tggcagtaca 5580
   tcaagtgtat catatgccaa gtacgccccc tattgacgtc aatgacggta aatggcccgc 5640
   ctggcattat gcccagtaca tgaccttatg ggactttcct acttggcagt acatctacgt 5700
   attagtcatc gctattacca tggtgatgcg gttttggcag tacatcaatg ggcgtggata 5760
   gcggtttgac tcacggggat ttccaagtct ccaccccatt gacgtcaatg ggagtttgtt 5820
   ttggcaccaa aatcaacggg actttccaaa atgtcgtaac aactccgccc cattgacgca 5880
   aatgggcggt aggcgtgtac ggtgggaggt ctatataagc agagctggtt tagtgaaccg 5940
   tcagatcctc ttccgcatcg ctgtctgcga gggccagctg ttggggtgag tactccctct 6000
   caaaagcggg catgacttct gcgctaagaa tgtcagtttc caaaaacgag gaggatttga 6060
   tattcactgg cccgcggtga tgcctttgag ggtggccgcg tccatctggt cagaaaagac 6120
   aatctttttg ttgtcaagct tccttgatga tgtcatactt atcctgtccc ttttttttcc 6180
   acagctcgcg gttgaggaca aactcttcgc ggtctttcca gtactcttgg atcggaaacc 6240
   cgtcggcctc cgaacggtac tccgccaccg agggacctga gcgagtccgc atcgaccgga 6300
   tcggaaaacc tctcgacgta ccaattaatt cgctgtctgc gagggccagc tgttggggtg 6360
   agtactccct ctcaaaagcg ggcatgactt ctgcgctaag attgtcagtt tccaaaaacg 6420
   aggaggattt gatattcacc tggcccgcgg tgatgccttt gagggtggcc gcgtccatct 6480
   ggtcagaaaa gacaatcttt ttgttgtcaa gcttgaggtg tggcaggctt gagatctggc 6540
   catacacttg agtgacaatg acatccactt tgcctttctc tccacaggtg tccactccca 6600
   ggtccaactg caggtcgagc atgcatctag ggcgcccgca ctagaggctc gagccaccat 6660
   gggggtgcac gaatgtcctg cctggctgtg gcttctcctg tccctgctgt cgctccctct 6720
   gggcctccca gtcctgggcg ccccaccacg cctcatctgt gacagccgag tcctggagag 6780
   gtacctcttg gaggccaagg aggccgagaa tatcacgacg ggctgtaatg aaacctgcag 6840
   cttgaatgag aatatcactg tcccagacac caaagttaat ttctatgcct ggaagaggat 6900
   ggaggtcggg cagcaggccg tagaagtctg gcagggcctg gccctgctgt cggaagctgt 6960
   cctgcggggc caggccctgt tggtcaactc ttcccaggtg aacgagaccc tgcagctgca 7020
   tgtggataaa gccgtcagtg gccttcgcag cctcaccact ctgcttcggg ctctgggagc 7080
   ccagaaggaa gccatctccc ctccagatgc ggcctcagct gctccactcc gaacaatcac 7140
   tgctgacact ttccgcaaac tcttccgagt ctactccaat ttcctccggg gaaagctgaa 7200
   gctgtacaca ggggaggcct gcaggacagg ggacagatga gcggccgccc gctgatcagc 7260
   ctcgactgtg ccttctagtt gccagccatc tgttgtttgc ccctcccccg tgccttcctt 7320
   gaccctggaa ggtgccactc ccactgtcct ttcctaataa aatgaggaaa ttgcatcgca 7380
   ttgtctgagt aggtgtcatt ctattctggg gggtggggtg gggcaggaca gcaaggggga 7440
   ggattgggaa gacaatagca ggcatgctgg ggatgcggtg ggctctatgg cttctgaggc 7500
   ggaaagaacc agctggggct ctagggggta tccccacgga gctcgcatcc ataatataac 7560
   tgtaccaggt tttggtttat tacatgtgac tgacggcttc ctatgcgtgc tcagaaaacg 7620
   gcagttgggc actgcactgc ccggtgatgg tgccacggtg gctcctgccg ccttctttga 7680
   tattcactct gttgtatttc atctcttgtt gccgatgaaa ggatataaca gtctctgagg 7740
   aaatacttgg tatttcttct gatcagcgtt tttataagta atgttgaata ttggataagg 7800
   ctgtgtgtcc tttgtcttgg gagacaaagc ccacagcagg tggtggttgg gtggtggcag 7860
   ctcagtgaca ggagaggttt ttttgcctgt tttttttgtt gttttttttt tttaagtaag 7920
   gtgttctttt ttcttagtaa aatttctact ggactgtatg ttttgacagg tcagaaacat 7980
   ttcttcaaaa gaagaacctt ttggaaactg tacagccctt ttctttcatt ccctttttgc 8040
   tttctgtgcc aatgcctttg gttctgattg cattatggaa aacgttgatc ggaacttgag 8100
   gtttttattt atagtgtggc ttgaaagctt ggatagctgt tgttacatga gataccttat 8160
   taagtttagg ccagcttgat gctttatttt ttttcctttg aagtagtgag cgttctctgg 8220
   tttttttcct ttgaaactgg cgaggcttag atttttctaa tgggattttt tacctgatga 8280
   tctagttgca tacccaaatg cttgtaaatg ttttcctagt taacatgttg ataacttcgg 8340
   atttacatgt tgtatatact tgtcatctgt gtttctagta aaaatatatg gcatttatag 8400
   aaatacgtaa ttcctgattt cctttttttt ttatctctat gctctgtgtg tacaggtcaa 8460
   acagacttca ctcctatttt tatttataga attttatatg cagtctgtcg ttggttcttg 8520
   tgttgtaagg atacagcctt aaatttccta gagcgatgct cagtaaggcg ggttgtcaca 8580
   tgggttcaaa tgtaaaacgg gcacgtttgc tgctgccttc ccagatccag gacactaaac 8640
   tgcttctgca actgaggtat aaatcgcttc agatcccagg aagtgtagat ccacgtgcat 8700
   attcttaaag aagaatgaat actttctaaa atatgttggc ataggaagca agctgcatgg 8760
   atttatttgg gacttaaatt attttggtaa cggagtgcat aggttttaaa cacagttgca 8820
   gcatgctaac gagtcacagc atttatgcag aagtgatgcc tgttgcagct gtttacggca 8880
   ctgccttgca gtgagcattg cagatagggg tggggtgctt tgtgtcgtgt tgggacacgc 8940
   tgccacacag ccacctcccg aacatatctc acctgctggg tacttttcaa accatcttag 9000
   cagtagtaga tgagttacta tgaaacagag aagttcctca gttggatatt ctcatgggat 9060
   gtcttttttc ccatgttggg caaagtatga taaagcatct ctatttgtaa attatgcact 9120
   tgttagttcc tgaatccttt ctatagcacc acttattgca gcaggtgtag gctctggtgt 9180
   ggcctgtgtc tgtgcttcaa tcttttaagc ttgagctcat ggcgcgccta ggccttgacg 9240
   gccttccttc aattcgccct atagtgagtc gtattacgtc gcgctcactg gccgtcgttt 9300
   tacaacgtcg tgactgggaa aaccctggcg ttacccaact taatcgcctt gcagcacatc 9360
   cccctttcgc cagctggcgt aatagcgaag aggcccgcac cgaaacgccc ttcccaacag 9420
   ttgcgcagcc tgaatggcga atgggagcgc cctgtagcgg ccactcaacc ctatctcggt 9480
   ctattctttt gatttataag ggattttgcc gatttcggcc tattggttaa aaaatgagct 9540
   gatttaacaa aaatttaacg cgaattttaa caaaatatta acgcttacaa tttag 9595
<210> 19
   <211> 10869
   <212> DNA
   <213> Artificial
<220>
   <223> pZRC III- Etanercept- Neo
<400> 19
   gtggcacttt tcggggaaat gtgcgcggaa cccctatttg tttatttttc taaatacatt 60
   caaatatgta tccgctcatg agacaataac cctgataaat gcttcaataa tattgaaaaa 120
   ggaagagtat gattgaacag gatggcctgc atgcgggtag cccggcagcg tgggtggaac 180
   gtctgtttgg ctatgattgg gcgcagcaga ccattggctg ctctgatgcg gcggtgtttc 240
   gtctgagcgc gcagggtcgt ccggtgctgt ttgtgaaaac cgatctgagc ggtgcgctga 300
   acgagctgca ggatgaagcg gcgcgtctga gctggctggc caccaccggt gttccgtgtg 360
   cggcggtgct ggatgtggtg accgaagcgg gccgtgattg gctgctgctg ggcgaagtgc 420
   cgggtcagga tctgctgtct agccatctgg cgccggcaga aaaagtgagc attatggcgg 480
   atgccatgcg tcgtctgcat accctggacc cggcgacctg tccgtttgat catcaggcga 540
   aacatcgtat tgaacgtgcg cgtacccgta tggaagcggg cctggtggat caggatgatc 600
   tggatgaaga acatcagggc ctggcaccgg cagagctgtt tgcgcgtctg aaagcgagca 660
   tgccggatgg cgaagatctg gtggtgaccc atggtgatgc gtgcctgccg aacattatgg 720
   tggaaaatgg ccgttttagc ggctttattg attgcggccg tctgggcgtg gcggatcgtt 780
   atcaggatat tgcgctggcc acccgtgata ttgcggaaga actgggcggc gaatgggcgg 840
   atcgttttct ggtgctgtat ggcattgcgg caccggatag ccagcgtatt gcgttttatc 900
   gtctgctgga tgaatttttc taataactgt cagaccaagt ttactcatat atactttaga 960
   ttgatttaaa acttcatttt taatttaaaa ggatctaggt gaagatcctt tttgataatc 1020
   tcatgaccaa aatcccttaa cgtgagtttt cgttccactg agcgtcagac cccgtagaaa 1080
   agatcaaagg atcttcttga gatccttttt ttctgcgcgt aatctgctgc ttgcaaacaa 1140
   aaaaaccacc gctaccagcg gtggtttgtt tgccggatca agagctacca actctttttc 1200
   cgaaggtaac tggcttcagc agagcgcaga taccaaatac tgttcttcta gtgtagccgt 1260
   agttaggcca ccacttcaag aactctgtag caccgcctac atacctcgct ctgctaatcc 1320
   tgttaccagt ggctgctgcc agtggcgata agtcgtgtct taccgggttg gactcaagac 1380
   gatagttacc ggataaggcg cagcggtcgg gctgaacggg gggttcgtgc acacagccca 1440
   gcttggagcg aacgacctac accgaactga gatacctaca gcgtgagcta tgagaaagcg 1500
   ccacgcttcc cgaagggaga aaggcggaca ggtatccggt aagcggcagg gtcggaacag 1560
   gagagcgcac gagggagctt ccagggggaa acgcctggta tctttatagt cctgtcgggt 1620
   ttcgccacct ctgacttgag cgtcgatttt tgtgatgctc gtcagggggg cggagcctat 1680
   ggaaaaacgc cagcaacgcg gcctttttac ggttcctggc cttttgctgg ccttttgctc 1740
   attaggcacc ccaggcttta cccgaacgac cgagcgcagc gagtcagtga gcgaggaagc 1800
   ggagagcgcc caatacgcaa ggaaacagct atgaccatgt taatgcagct ggcacgacag 1860
   gtttcccgac tggaaagcgg gcagtgaaag gaaggcccat gaggccagtt aattaactgt 1920
   ggaatgtgtg tcagttaggg tgtggaaagt ccccaggctc cccagcaggc agaagtatgc 1980
   aaagcatgca tctcaattag tcagcaacca ggtgtggaaa gtccccaggc tccccagcag 2040
   gcagaagtat gcaaagcatg catctcaatt agtcagcaac catagtcccg cccctaactc 2100
   cgcccatccc gcccctaact ccgcccagtt ccgcccattc tccgccccat ggctgactaa 2160
   ttttttttat ttatgcagag gccgaggccg cctctgcctc tgagctattc cagaagtagt 2220
   gaggaggctt ttttggaggc ctaggctttt gcaaaaagct cccgggagct tgtatatcca 2280
   ttttcggatc tgatcaagag acaggatgag gatcgtttcg catgattgaa caagatggat 2340
   tgcacgcagg ttctccggcc gcttgggtgg agaggctatt cggctatgac tgggcacaac 2400
   agacaatcgg ctgctctgat gccgccgtgt tccggctgtc agcgcagggg cgcccggttc 2460
   tttttgtcaa gaccgacctg tccggtgccc tgaatgaact gcaggacgag gcagcgcggc 2520
   tatcgtggct ggccacgacg ggcgttcctt gcgcagctgt gctcgacgtt gtcactgaag 2580
   cgggaaggga ctggctgcta ttgggcgaag tgccggggca ggatctcctg tcatctcacc 2640
   ttgctcctgc cgagaaagta tccatcatgg ctgatgcaat gcggcggctg catacgcttg 2700
   atccggctac ctgcccattc gaccaccaag cgaaacatcg catcgagcga gcacgtactc 2760
   ggatggaagc cggtcttgtc gatcaggatg atctggacga agagcatcag gggctcgcgc 2820
   cagccgaact gttcgccagg ctcaaggcgc gcatgcccga cggcgaggat ctcgtcgtga 2880
   cccatggcga tgcctgcttg ccgaatatca tggtggaaaa tggccgcttt tctggattca 2940
   tcgactgtgg ccggctgggt gtggcggacc gctatcagga catagcgttg gctacccgtg 3000
   atattgctga agagcttggc ggcgaatggg ctgaccgctt cctcgtgctt tacggtatcg 3060
   ccgctcccga ttcgcagcgc atcgccttct atcgccttct tgacgagttc ttctgagcgg 3120
   gactctgggg ttcgaaatga ccgaccaagc gacgcccaac ctgccatcac gagatttcga 3180
   ttccaccgcc gccttctatg aaaggttggg cttcggaatc gttttccggg acgccggctg 3240
   gatgatcctc cagcgcgggg atctcatgct ggagttcttc gcccacccca acttgtttat 3300
   tgcagcttat aatggttaca aataaagcaa tagcatcaca aatttcacaa ataaagcatt 3360
   tttttcactg cattctagtt gtggtttgtc caaactcatc aatgtatctt atcatgtctg 3420
   ggatcctcgg gacgctctgg ccggtgaggc gtgcgcagtc gttgacgctc tagaccgtgc 3480
   aaaaggagag cctgtaagcg ggcactcttc cgtggtctgg tggataaatt cgcaagggta 3540
   tcatggcgga cgaccggggt tcgaacccca gatccggccg tccgccgtga tccatgcggt 3600
   taccgcccgc gtgtcgaacc caggtgtgcg acgtcagaca acgggggagc gctccttttg 3660
   gcttccttcc aggcgcggcg gctgctgcgc tagctttttt ggccactggc cgcgcgcggc 3720
   gtaagcggtt aggctggaaa gcgaaagcat taagtggctc gctccctgta gccggagggt 3780
   tattttccaa gggttgagtc gcaggacccc cggttcgagt ctcgggccgg ccggactgcg 3840
   gcgaacgggg gtttgcctcc ccgtcatgca agaccccgct tgcaaattcc tccggaaaca 3900
   gggacgagcc ccttttttgc ttttcccaga tgcatccggt gctgcggcag atgcgccccc 3960
   ctcctcagca gcggcaagag caagagcagc ggcagacatg cagggcaccc tccccttctc 4020
   ctaccgcgtc aggaggggca acatccgacg cgtgcatcca taatataact gtaccaggtt 4080
   ttggtttatt acatgtgact gacggcttcc tatgcgtgct cagaaaacgg cagttgggca 4140
   ctgcactgcc cggtgatggt gccacggtgg ctcctgccgc cttctttgat attcactctg 4200
   ttgtatttca tctcttgttg ccgatgaaag gatataacag tctctgagga aatacttggt 4260
   atttcttctg atcagcgttt ttataagtaa tgttgaatat tggataaggc tgtgtgtcct 4320
   ttgtcttggg agacaaagcc cacagcaggt ggtggttggg tggtggcagc tcagtgacag 4380
   gagaggtttt tttgcctgtt ttttttgttg tttttttttt ttaagtaagg tgttcttttt 4440
   tcttagtaaa atttctactg gactgtatgt tttgacaggt cagaaacatt tcttcaaaag 4500
   aagaaccttt tggaaactgt acagcccttt tctttcattc cctttttgct ttctgtgcca 4560
   atgcctttgg ttctgattgc attatggaaa acgttgatcg gaacttgagg tttttattta 4620
   tagtgtggct tgaaagcttg gatagctgtt gttacatgag ataccttatt aagtttaggc 4680
   cagcttgatg ctttattttt tttcctttga agtagtgagc gttctctggt ttttttcctt 4740
   tgaaactggc gaggcttaga tttttctaat gggatttttt acctgatgat ctagttgcat 4800
   acccaaatgc ttgtaaatgt tttcctagtt aacatgttga taacttcgga tttacatgtt 4860
   gtatatactt gtcatctgtg tttctagtaa aaatatatgg catttataga aatacgtaat 4920
   tcctgatttc cttttttttt tatctctatg ctctgtgtgt acaggtcaaa cagacttcac 4980
   tcctattttt atttatagaa ttttatatgc agtctgtcgt tggttcttgt gttgtaagga 5040
   tacagcctta aatttcctag agcgatgctc agtaaggcgg gttgtcacat gggttcaaat 5100
   gtaaaacggg cacgtttgct gctgccttcc cagatccagg acactaaact gcttctgcaa 5160
   ctgaggtata aatcgcttca gatcccagga agtgtagatc cacgtgcata ttcttaaaga 5220
   agaatgaata ctttctaaaa tatgttggca taggaagcaa gctgcatgga tttatttggg 5280
   acttaaatta ttttggtaac ggagtgcata ggttttaaac acagttgcag catgctaacg 5340
   agtcacagca tttatgcaga agtgatgcct gttgcagctg tttacggcac tgccttgcag 5400
   tgagcattgc agataggggt ggggtgcttt gtgtcgtgtt gggacacgct gccacacagc 5460
   cacctcccga acatatctca cctgctgggt acttttcaaa ccatcttagc agtagtagat 5520
   gagttactat gaaacagaga agttcctcag ttggatattc tcatgggatg tcttttttcc 5580
   catgttgggc aaagtatgat aaagcatctc tatttgtaaa ttatgcactt gttagttcct 5640
   gaatcctttc tatagcacca cttattgcag caggtgtagg ctctggtgtg gcctgtgtct 5700
   gtgcttcaat cttttaagct tacgcgttga cattgattat tgactagtta ttaatagtaa 5760
   tcaattacgg ggtcattagt tcatagccca tatatggagt tccgcgttac ataacttacg 5820
   gtaaatggcc cgcctggctg accgcccaac gacccccgcc cattgacgtc aataatgacg 5880
   tatgttccca tagtaacgcc aatagggact ttccattgac gtcaatgggt ggagtattta 5940
   cggtaaactg cccacttggc agtacatcaa gtgtatcata tgccaagtac gccccctatt 6000
   gacgtcaatg acggtaaatg gcccgcctgg cattatgccc agtacatgac cttatgggac 6060
   tttcctactt ggcagtacat ctacgtatta gtcatcgcta ttaccatggt gatgcggttt 6120
   tggcagtaca tcaatgggcg tggatagcgg tttgactcac ggggatttcc aagtctccac 6180
   cccattgacg tcaatgggag tttgttttgg caccaaaatc aacgggactt tccaaaatgt 6240
   cgtaacaact ccgccccatt gacgcaaatg ggcggtaggc gtgtacggtg ggaggtctat 6300
   ataagcagag ctggtttagt gaaccgtcag atcctcttcc gcatcgctgt ctgcgagggc 6360
   cagctgttgg ggtgagtact ccctctcaaa agcgggcatg acttctgcgc taagaatgtc 6420
   agtttccaaa aacgaggagg atttgatatt cactggcccg cggtgatgcc tttgagggtg 6480
   gccgcgtcca tctggtcaga aaagacaatc tttttgttgt caagcttcct tgatgatgtc 6540
   atacttatcc tgtccctttt ttttccacag ctcgcggttg aggacaaact cttcgcggtc 6600
   tttccagtac tcttggatcg gaaacccgtc ggcctccgaa cggtactccg ccaccgaggg 6660
   acctgagcga gtccgcatcg accggatcgg aaaacctctc gacgtaccaa ttaattcgct 6720
   gtctgcgagg gccagctgtt ggggtgagta ctccctctca aaagcgggca tgacttctgc 6780
   gctaagattg tcagtttcca aaaacgagga ggatttgata ttcacctggc ccgcggtgat 6840
   gcctttgagg gtggccgcgt ccatctggtc agaaaagaca atctttttgt tgtcaagctt 6900
   gaggtgtggc aggcttgaga tctggccata cacttgagtg acaatgacat ccactttgcc 6960
   tttctctcca caggtgtcca ctcccaggtc caactgcagg tcgagcatgc atctagggcg 7020
   cccgcactag aggctcgagc catggctcca gtggctgtgt gggctgctct ggctgtgggc 7080
   ctggaactgt gggccgctgc tcacgctctg cctgctcagg tggccttcac cccttatgcc 7140
   cctgagcctg gctccacctg caggctgcgg gagtactacg accagaccgc ccagatgtgc 7200
   tgctccaagt gctctcctgg ccagcacgcc aaggtgttct gcaccaagac ctccgacacc 7260
   gtgtgcgact cttgcgagga ctccacctac acccagctct ggaactgggt gcccgagtgc 7320
   ctgtcctgcg gctccagatg ctcctccgac caggtggaga cacaggcctg tacacgggag 7380
   cagaatcgga tttgcacatg caggcctggc tggtactgcg ccctgtccaa gcaggaagga 7440
   tgcaggctgt gcgcccctct gaggaagtgc agacctggct tcggcgtggc taggcctggc 7500
   accgagacat ccgacgtcgt gtgcaagcct tgtgcccctg gcaccttctc caacaccaca 7560
   tcctccaccg acatctgccg gcctcaccag atctgcaacg tggtggccat ccctggcaac 7620
   gccagcatgg acgccgtgtg cacctccacc tcccccacca gatctatggc ccctggcgcc 7680
   gtgcatctgc ctcagcctgt gtccacccgg tcccagcata cccagcctac acctgagccc 7740
   tctaccgccc cttctacctc cttcctgctg cctatgggcc cttctcctcc agctgagggc 7800
   tctaccggcg acgagcctaa gtcctgcgac aagacccaca cctgtccccc ctgccctgcc 7860
   cctgaactgc tgggaggccc cagcgtgttc ctgttccccc caaagcccaa ggacaccctg 7920
   atgatctccc ggacccccga agtgacctgc gtggtggtgg acgtgtccca cgaggaccct 7980
   gaagtgaagt tcaattggta cgtggacggc gtggaagtgc acaacgccaa gaccaagccc 8040
   agagaggaac agtacaactc cacctaccgg gtggtgtccg tgctgaccgt gctgcaccag 8100
   gactggctga acggcaaaga gtacaagtgc aaggtgtcca acaaggctct gcctgccccc 8160
   atcgaaaaga ccatctccaa ggccaagggc cagccccgcg agcctcaggt gtacaccctg 8220
   cctccttccc gggaggagat gaccaagaac caggtgtccc tgacctgtct ggtcaagggc 8280
   ttctacccct ccgatatcgc cgtggaatgg gagtccaacg gacagcccga gaacaactac 8340
   aagaccaccc cccctgtgct ggactccgac ggctcattct tcctgtactc caagctgacc 8400
   gtggacaagt cccggtggca gcagggcaac gtgttctcct gctccgtgat gcacgaggcc 8460
   ctgcacaacc actacaccca gaagagctta tccctgtctc ctggcaagtg ataagcggcc 8520
   gcccgctgat cagcctcgac tgtgccttct agttgccagc catctgttgt ttgcccctcc 8580
   cccgtgcctt ccttgaccct ggaaggtgcc actcccactg tcctttccta ataaaatgag 8640
   gaaattgcat cgcattgtct gagtaggtgt cattctattc tggggggtgg ggtggggcag 8700
   gacagcaagg gggaggattg ggaagacaat agcaggcatg ctggggatgc ggtgggctct 8760
   atggcttctg aggcggaaag aaccagctgg ggctctaggg ggtatcccca cggagctcgc 8820
   atccataata taactgtacc aggttttggt ttattacatg tgactgacgg cttcctatgc 8880
   gtgctcagaa aacggcagtt gggcactgca ctgcccggtg atggtgccac ggtggctcct 8940
   gccgccttct ttgatattca ctctgttgta tttcatctct tgttgccgat gaaaggatat 9000
   aacagtctct gaggaaatac ttggtatttc ttctgatcag cgtttttata agtaatgttg 9060
   aatattggat aaggctgtgt gtcctttgtc ttgggagaca aagcccacag caggtggtgg 9120
   ttgggtggtg gcagctcagt gacaggagag gtttttttgc ctgttttttt tgttgttttt 9180
   tttttttaag taaggtgttc ttttttctta gtaaaatttc tactggactg tatgttttga 9240
   caggtcagaa acatttcttc aaaagaagaa ccttttggaa actgtacagc ccttttcttt 9300
   cattcccttt ttgctttctg tgccaatgcc tttggttctg attgcattat ggaaaacgtt 9360
   gatcggaact tgaggttttt atttatagtg tggcttgaaa gcttggatag ctgttgttac 9420
   atgagatacc ttattaagtt taggccagct tgatgcttta ttttttttcc tttgaagtag 9480
   tgagcgttct ctggtttttt tcctttgaaa ctggcgaggc ttagattttt ctaatgggat 9540
   tttttacctg atgatctagt tgcataccca aatgcttgta aatgttttcc tagttaacat 9600
   gttgataact tcggatttac atgttgtata tacttgtcat ctgtgtttct agtaaaaata 9660
   tatggcattt atagaaatac gtaattcctg atttcctttt ttttttatct ctatgctctg 9720
   tgtgtacagg tcaaacagac ttcactccta ttttatttta tagaatttta tatgcagtct 9780
   gtcgttggtt cttgtgttgt aaggatacag ccttaaattt cctagagcga tgctcagtaa 9840
   ggcgggttgt cacatgggtt caaatgtaaa acgggcacgt ttgctgctgc cttcccagat 9900
   ccaggacact aaactgcttc tgcaactgag gtataaatcg cttcagatcc caggaagtgt 9960
   agatccacgt gcatattctt aaagaagaat gaatactttc taaaatatgt tggcatagga 10020
   agcaagctgc atggatttat ttgggactta aattattttg gtaacggagt gcataggttt 10080
   taaacacagt tgcagcatgc taacgagtca cagcatttat gcagaagtga tgcctgttgc 10140
   agctgtttac ggcactgcct tgcagtgagc attgcagata ggggtggggt gctttgtgtc 10200
   gtgttgggac acgctgccac acagccacct cccgaacata tctcacctgc tgggtacttt 10260
   tcaaaccatc ttagcagtag tagatgagtt actatgaaac agagaagttc ctcagttgga 10320
   tattctcatg ggatgtcttt tttcccatgt tgggcaaagt atgataaagc atctctattt 10380
   gtaaattatg cacttgttag ttcctgaatc ctttctatag caccacttat tgcagcaggt 10440
   gtaggctctg gtgtggcctg tgtctgtgct tcaatctttt aagcttgagc tcatggcgcg 10500
   cctaggcctt gacggccttc cttcaattcg ccctatagtg agtcgtatta cgtcgcgctc 10560
   actggccgtc gttttacaac gtcgtgactg ggaaaaccct ggcgttaccc aacttaatcg 10620
   ccttgcagca catccccctt tcgccagctg gcgtaatagc gaagaggccc gcaccgaaac 10680
   gcccttccca acagttgcgc agcctgaatg gcgaatggga gcgccctgta gcggccactc 10740
   aaccctatct cggtctattc ttttgattta taagggattt tgccgatttc ggcctattgg 10800
   ttaaaaaatg agctgattta acaaaaattt aacgcgaatt ttaacaaaat attaacgctt 10860
   acaatttag 10869
<210> 20
   <211> 1710
   <212> DNA
   <213> Artificial
<220>
   <223> TNK (DNA sequence corresponding to Seq ID No. 7)
<400> 20
   gaattccacc atggacgcca tgaagcgggg actgtgctgt gtgctgctgc tgtgtggcgc 60
   cgtgttcgtg tcccctagcc aggaaatcca cgcccggttc agaaggggcg ccaggtccta 120
   ccaagttatc tgccgggacg aaaagaccca gatgatctac cagcagcacc agtcctggct 180
   gcggcccgtg ctgcggtcca accgggtgga gtactgctgg tgcaactccg gcagagccca 240
   gtgccactcc gtgcctgtga agtcctgctc cgagcctaga tgcttcaacg gcggcacctg 300
   tcagcaggcc ctgtacttct ccgacttcgt gtgccagtgc cctgagggct tcgccggcaa 360
   gtgctgcgag atcgacaccc gggccacctg ttacgaggac cagggcatct cctaccgggg 420
   caactggtct accgccgagt ctggcgccga gtgcaccaac tggcagtcct ccgccctggc 480
   ccagaagcct tactctggca gacggcctga cgccatcaga ctgggcctgg gcaaccacaa 540
   ctactgccgg aaccctgacc gggactccaa gccttggtgc tacgtgttca aggccggcaa   600
   gtactcctcc gagttctgct ccacccctgc ctgctctgag ggcaactccg actgctactt   660
   cggcaacggc tccgcctaca gaggcaccca ctccctgacc gagtccggcg cctcttgcct   720
   gccttggaac tccatgatcc tgatcggcaa ggtgtacacc gcccagaacc cttccgctca   780
   ggccctggga ctcggaaagc acaattattg tcgcaatccc gacggcgacg ccaaaccttg   840
   gtgtcacgtg ctgaagaacc ggcggctgac atgggaatac tgcgacgtgc cttcctgctc   900
   tacctgcggc ctgcggcagt actcccagcc tcagttccgg atcaagggcg gcctgttcgc   960
   cgatatcgcc tcccaccctt ggcaggccgc catcttcgcc gctgctgccg cttctcctgg  1020
   cgagagattc ctgtgcggcg gcatcctgat ctccagctgc tggattctgt ctgccgccca  1080
   ctgcttccag gaacggttcc ctcctcacca cctgaccgtg atcctgggcc ggacctacag  1140
   agtcgtgccc ggcgaggaag aacagaaatt cgaggtggag aagtatatcg tgcacaaaga  1200
   gttcgacgac gacacctacg acaacgatat cgccctgctg cagctgaagt ccgactcctc  1260
   cagatgcgcc caggaatcct ccgtcgttcg gaccgtgtgt ctgccacctg ccgacctgca  1320
   gctgcctgac tggaccgagt gcgagctgtc cggctacggc aagcacgagg ccctgtcccc  1380
   cttctactcc gagcggctga aagaagctca tgtacggctg tacccctcta gccggtgcac  1440
   ctcccagcat ctgctgaacc ggaccgtgac cgacaacatg ctgtgtgccg gcgacaccag  1500
   atctggcggc cctcaggcca acctgcacga cgcctgccag ggcgatagtg gcggacctct  1560
   cgtgtgcctc aacgacggca ggatgaccct cgtgggcatc atctcttggg gcctgggctg  1620
   tggccagaaa gacgtgcctg gcgtgtacac caaagtgacc aactacctgg actggatcag  1680
   ggacaacatg cggccttgat gagcggccgc                                   1710
<210> 21
   <211> 601
   <212> DNA
   <213> Artificial
<220>
   <223> Darbepoetin (DNA sequence corresponding to Seq ID No. 9)
<400> 21
   ctcgagccac catgggggtg cacgaatgtc ctgcctggct gtggcttctc ctgtccctgc    60
   tgtcgctccc tctgggcctc ccagtcctgg gcgccccacc acgcctcatc tgtgacagcc   120
   gagtcctgga gaggtacctc ttggaggcca aggaggccga gaatatcacg acgggctgta   180
   atgaaacctg cagcttgaat gagaatatca ctgtcccaga caccaaagtt aatttctatg   240
   cctggaagag gatggaggtc gggcagcagg ccgtagaagt ctggcagggc ctggccctgc   300
   tgtcggaagc tgtcctgcgg ggccaggccc tgttggtcaa ctcttcccag gtgaacgaga   360
   ccctgcagct gcatgtggat aaagccgtca gtggccttcg cagcctcacc actctgcttc   420
   gggctctggg agcccagaag gaagccatct cccctccaga tgcggcctca gctgctccac   480
   tccgaacaat cactgctgac actttccgca aactcttccg agtctactcc aatttcctcc   540
   ggggaaagct gaagctgtac acaggggagg cctgcaggac aggggacaga tgagcggccg   600
   c 601
<210> 22
   <211> 1489
   <212> DNA
   <213> Artificial
<220>
   <223> Etanercept (DNA sequence corresponding to Seq ID No. 11)
<400> 22
   ctcgagccat ggctccagtg gctgtgtggg ctgctctggc tgtgggcctg gaactgtggg 60
   ccgctgctca cgctctgcct gctcaggtgg ccttcacccc ttatgcccct gagcctggct 120
   ccacctgcag gctgcgggag tactacgacc agaccgccca gatgtgctgc tccaagtgct 180
   ctcctggcca gcacgccaag gtgttctgca ccaagacctc cgacaccgtg tgcgactctt 240
   gcgaggactc cacctacacc cagctctgga actgggtgcc cgagtgcctg tcctgcggct 300
   ccagatgctc ctccgaccag gtggagacac aggcctgtac acgggagcag aatcggattt 360
   gcacatgcag gcctggctgg tactgcgccc tgtccaagca ggaaggatgc aggctgtgcg 420
   cccctctgag gaagtgcaga cctggcttcg gcgtggctag gcctggcacc gagacatccg 480
   acgtcgtgtg caagccttgt gcccctggca ccttctccaa caccacatcc tccaccgaca 540
   tctgccggcc tcaccagatc tgcaacgtgg tggccatccc tggcaacgcc agcatggacg 600
   ccgtgtgcac ctccacctcc cccaccagat ctatggcccc tggcgccgtg catctgcctc 660
   agcctgtgtc cacccggtcc cagcataccc agcctacacc tgagccctct accgcccctt 720
   ctacctcctt cctgctgcct atgggccctt ctcctccagc tgagggctct accggcgacg 780
   agcctaagtc ctgcgacaag acccacacct gtcccccctg ccctgcccct gaactgctgg 840
   gaggccccag cgtgttcctg ttccccccaa agcccaagga caccctgatg atctcccgga 900
   cccccgaagt gacctgcgtg gtggtggacg tgtcccacga ggaccctgaa gtgaagttca 960
   attggtacgt ggacggcgtg gaagtgcaca acgccaagac caagcccaga gaggaacagt 1020
   acaactccac ctaccgggtg gtgtccgtgc tgaccgtgct gcaccaggac tggctgaacg 1080
   gcaaagagta caagtgcaag gtgtccaaca aggctctgcc tgcccccatc gaaaagacca 1140
   tctccaaggc caagggccag ccccgcgagc ctcaggtgta caccctgcct ccttcccggg 1200
   aggagatgac caagaaccag gtgtccctga cctgtctggt caagggcttc tacccctccg 1260
   atatcgccgt ggaatgggag tccaacggac agcccgagaa caactacaag accacccccc 1320
   ctgtgctgga ctccgacggc tcattcttcc tgtactccaa gctgaccgtg gacaagtccc 1380
   ggtggcagca gggcaacgtg ttctcctgct ccgtgatgca cgaggccctg cacaaccact 1440
   acacccagaa gagcttatcc ctgtctcctg gcaagtgata agcggccgc 1489
<210> 23
   <211> 365
   <212> DNA
   <213> Artificial
<220>
   <223> FSH alpha (DNA sequence corresponding to Seq ID No. 13)
<400> 23
   ctcgagccac catggactac taccggaagt acgccgccat cttcctggtg accctgtccg     60
   tgttcctgca cgtgctgcac tctgcccctg acgtgcagga ctgccctgag tgcaccctgc    120
   aggaaaaccc attcttcagc cagcctggcg cccctatcct gcagtgcatg ggctgctgct    180
   tctccagagc ctaccctacc cctctgcggt ccaagaaaac catgctggtg cagaaaaacg    240
   tgacctccga gtctacctgc tgcgtggcca agtcctacaa cagagtgacc gtgatgggcg    300
   gcttcaaggt ggagaaccac accgcctgcc actgctctac ctgctactac cacaagtcct    360
   gatga                                                                365
<210> 24
   <211> 415
   <212> DNA
   <213> Artificial
<220>
   <223> FSH beta (DNA sequence corresponding to Seq ID No. 15)
<400> 24
   cccgggccac catgaagacc ctgcagttct tcttcctgtt ctgctgctgg aaggccatct     60
   gctgcaactc ttgcgagctg accaacatca caatcgccat cgagaaagag gaatgccggt    120
   tctgcatctc catcaacacc acttggtgcg ccggctactg ctacacccgc gacctggtgt    180
   acaaggaccc tgcccggcct aagatccaga aaacctgcac cttcaaagaa ctggtgtacg    240
   agacagtgcg ggtgccaggc tgcgctcacc acgccgactc cctgtacacc taccctgtgg    300
   ccacccagtg ccactgcggc aagtgcgact ccgactccac cgactgtacc gtgcggggcc    360
   tgggcccttc ttactgctcc ttcggcgaga tgaaggaatg atgaccagcg gccgc         415